(19) Europäisches Patentamt
European Patent Office
Office européen des brevets

(11) Veröffentlichungsnummer: **0 300 969 B1**

# (12) EUROPÄISCHE PATENTSCHRIFT

(45) Veröffentlichungstag der Patentschrift: **18.01.95**

(51) Int. Cl.[6]: **C08G 65/32**, A61K 31/00

(21) Anmeldenummer: **88810484.1**

(22) Anmeldetag: **15.07.88**

## (54) Polyethylenglykolcarbaminate.

(30) Priorität: **23.07.87 CH 2794/87**

(43) Veröffentlichungstag der Anmeldung:
**25.01.89 Patentblatt 89/04**

(45) Bekanntmachung des Hinweises auf die Patenterteilung:
**18.01.95 Patentblatt 95/03**

(84) Benannte Vertragsstaaten:
**AT BE CH DE ES FR GB GR IT LI LU NL SE**

(56) Entgegenhaltungen:
**EP-A- 0 271 443**
**WO-A-85/03290**
**WO-A-86/03747**

(73) Patentinhaber: **CIBA-GEIGY AG**
**Klybeckstrasse 141**
**CH-4002 Basel (CH)**

(72) Erfinder: **Peter, Heinrich, Dr.**
**Bündtenweg 69**
**CH-4102 Binningen (CH)**
Erfinder: **Moerker, Theophile**
**Ergolzstrasse 72**
**CH-4414 Füllinsdorf (CH)**

**EP 0 300 969 B1**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

## Beschreibung

Die Erfindung betrifft Polyethylenglycolcarbaminate, insbesondere entsprechende Derivate von Desferrioxaminverbindungen und deren Metallkomplexe, in erster Linie Verbindungen der Formel

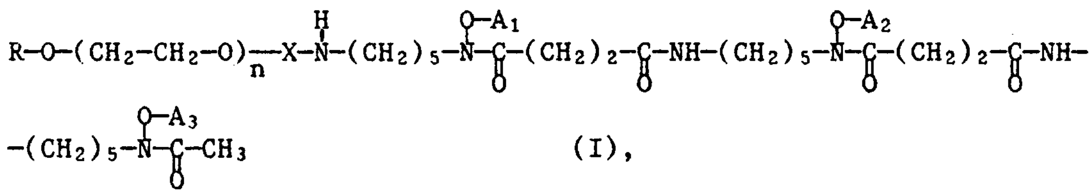

(I),

worin R für Alkyl mit bis zu 4 Kohlenstoffatomen steht, n einen Durchschnittswert von mindestens 9 darstellt, X einen Rest der Formel $-C(=O)-(NH-SO_2)_m-$ bedeutet, worin m für 0 oder 1 steht und, falls m für 1 steht, die Carbonylgruppe mit dem Sauerstoffatom oder dem Stickstoffatom verbunden sein kann, und jeder der Reste $A_1$, $A_2$ und $A_3$, unabhängig von den anderen, Wasserstoff oder einen Acylrest darstellt, und Salze von salzbildenden Verbindungen der Formel I, sowie Metallkomplexe von Verbindungen der Formel I, worin $A_1$, $A_2$ und $A_3$ für Wasserstoff stehen, ferner Verfahren zur Herstellung von solchen Verbindungen, diese enthaltende Darreichungsformen, wie pharmazeutische und diagnostische Darreichungsformen, und ihre Verwendung für therapeutische bzw. diagnostische Zwecke.

Desferrioxamin B (H. Bickel et al., Helv. Chim. Acta, Bd. 46, S. 1385 [1963]) mit der Formel

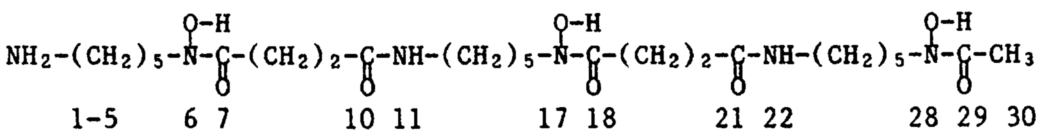

(II)

wird aufgrund der Regel C-O6 (Austausch-Nomenklatur) der offiziellen IUPAC-Nomenklatur mit dem systematischen Namen 6,17,28-Trihydroxy-7,10,18,21,29-pentaoxo-6,11,17,22,28-pentaazatriacontylamin bezeichnet. Der Einfachheit halber werden jedoch nachfolgend die Namen der Verbindungen der vorliegenden Erfindung vom Trivialnamen abgeleitet, wobei die Position einzelner Acylreste jeweils auf das mit N bezeichnete Stickstoffatom der Aminogruppe in Stellung 1 bzw. auf die mit O, O' und O" bezeichneten Sauerstoffatome der Hydroxylgruppen in Stellungen 6, 17 bzw. 28 bezogen wird.

Zu den Eigenschaften von Desferrioxamin B und seinen Additionssalzen gehört die Fähigkeit, stabile Chelat-artige Metallkomplexe, insbesondere mit trivalenten Metallionen, wie Chrom-(III)-, Aluminium- und in erster Linie Eisen-(III)-ionen, zu bilden. Entsprechend angewandt kann Desferrioxamin B die Ablagerung eisenhaltiger Pigmente im Gewebe verhindern und bei bestehenden Eisenablagerungen im Organismus, z.B. bei Hämochromatose, Hämosiderose, Lebercirrhose und Vergiftungen mit Verbindungen des dreiwertigen Eisens, eine Ausscheidung des Eisens bewirken. Die therapeutische Anwendung von Desferrioxamin B und seiner Salze, z.B. dem Methansulfonat, erstreckt sich allgemein auf krankhafte Zustände, welche mit übermässiger Belastung des Organismus mit Eisen-(III)-ionen einhergehen, wie Thalassämie major, Sichelzell-Anämie, sideroachrestische Anämie, aplastische Anämie und weitere anämische Formen, in welchen Hämosiderose, d.h. eine lokale oder allgemeine Erhöhung der Eisenvorräte in sonst unbeschädigten Körpergeweben, eine Rolle spielt. Dazu gehören auch krankhafte Zustände, die sich bei Patienten nach mehrmaligen Bluttransfusionen oder mehrfach wiederholter Dialysebehandlung bei fehlender oder geschädigter Nierenfunktion entwickeln. Dank den komplexbildenden Eigenschaften zeigt Desferrioxamin B ebenfalls eine Wirkung bei Erkrankungen, die durch Eisen-(III)-abhängige Mikroorganismen und Parasiten bewirkt werden, wie insbesondere bei Malaria. Auch die Komplex-Bildung mit anderen dreiwertigen Metallen kann zu deren Ausscheidung aus dem Organismus verwendet werden, z.B. zur Entfernung von Aluminium bei der Dialyse-Encephalopathie und Osteomalacia, sowie bei der Alzheimer Krankheit.

Als nachteilig erweist sich jedoch, dass Desferrioxamin B und seine Salze einerseits bei oraler Verabreichung nur eine unzureichende Wirksamkeit aufweisen, und andererseits bei parenteraler Verabreichung rasch ausgeschieden werden. Deshalb wird die Wirksubstanz üblicherweise mittels einer langsamen subkutanen Infusion verabreicht, was jedoch entweder eine Hospitalisierung des Patienten oder, bei ambulanter Behandlung, die Anwendung einer tragbaren mechanischen Vorrichtung, wie einer durch elektrischen Antrieb betätigten Infusionsspritze, bedingt. Abgesehen von ihrer Umständlichkeit sind solche

Behandlungsmethoden mit relativ hohen - Kosten verbunden, wodurch ihre Anwendung stark eingeschränkt ist; insbesondere ist unter solchen Bedingungen eine umfassende Behandlung in Ländern der dritten Welt praktisch ausgeschlossen. Die kurze Verweildauer von Desferrioxamin B im Organismus hat zur Folge, dass bei gewöhnlichen Verabreichungsformen ein Grossteil des Wirkstoffes ungenutzt wieder ausgeschieden wird, ehe er zur gewünschten Wirkung kommt.

Die neuen Verbindungen der Formel I mit den längeren Polyethylenglykolketten im N-Acylrest zeigen eine unerwartet langsame Ausscheidungsrate und damit eine verlängerte Verweildauer im Organismus. Dadurch wird es möglich, diese Desferrioxamin B-Derivate in den für eine parenterale Verabreichung üblichen Intervallen, z.B. 1-3 mal täglich, in Form von Bolusinjektionen einzusetzen. Als besonders wichtigen Vorteil weisen die erfindungsgemässen Verbindungen eine überraschend gute Löslichkeit sowohl in organischen Lösungsmitteln (wie in halogenierten Niederalkanen, z.B. Chloroform und Dichlormethan), wie insbesondere auch in Wasser (bis etwa 30 Gew.-%) auf. Die ausserordentlich gute Wasserlöslichkeit ist insbesondere für parenterale Arzneimittelformen wichtig, umso mehr als die neutralen Verbindungen der Formel I in freier Form eingesetzt werden können und auf Säureadditionssalze, wie sie beim Desferrioxamin B verwendet werden, verzichtet werden kann. Durch geeignete Wahl der Polyethylenglykol-sequenz können zudem die gewünschten physikalischen und physiologischen Eigenschaften für spezifische Zwecke noch fein abgestuft und optimiert werden.

Die neuen Verbindungen der Formel I, in welchen $A_1$, $A_2$ und/oder $A_3$ für Acylreste stehen, erweisen sich bei oraler Verabreichung wesentlich wirksamer als Desferrioxamin B und dessen Salze und können entsprechend verwendet werden.

Die erfindungsgemässen Verbindungen der Formel I sind deshalb in denselben Indikationen anwendbar, in welchen Desferrioxamin B bzw. dessen Salze, wie das Methansulfonat, anwendbar ist, d.h. zur Behandlung der oben angegebenen krankhaften Zustände.

Die Komplexe von Verbindungen der Formel I, worin $A_1$, $A_2$ und $A_3$ für Wasserstoff stehen, insbesondere mit geeigneten paramagnetischen und/oder radioaktiven Metallen, können wegen ihrer hohen Löslichkeit und guten Verträglichkeit in der diagnostischen Medizin, z.B. der Röntgen-, Radionuklid-, Ultraschall- und/oder magnetischen Kernresonanzdiagnostik, als Kontrastmittel verwendet werden.

Im Hinblick darauf, dass Polyethylenglycolmonoalkylether, die in der Herstellung der Verbindungen der Formel I bzw. von Ausgangs- und Zwischenprodukten zu deren Herstellung verwendet werden, sofern sie eine gewisse Anzahl, z.B. mehr als 3 bis 4 Einheiten der Formel $-(CH_2-CH_2-O)-$ (Ia) aufweisen, in Form von einheitlichen Verbindungen kaum zur Verfügung stehen, sondern normalerweise als Gemische von mehreren Polyethylenglycolmonoalkylethern vorliegen und deshalb zu Verbindungen der Formel I führen, die üblicherweise in Form von entsprechenden Gemischen vorliegen, in denen sich die individuellen Verbindungen durch eine verschiedene Anzahl von Einheiten der Formel Ia unterscheiden, wird im vorliegenden Fall die Anzahl dieser Einheiten als Durchschnittswert angegeben. D.h. in einer Verbindung der Formel I mit einem Durchschnittswert für n von mindestens 9 können die individuellen Verbindungen der Formel I von etwa 5 bis etwa 13 Einheiten der Formel Ia aufweisen. Vorzugsweise zeigen sie Durchschnittswerte für n von etwa 9 bis etwa 115, in erster Linie von etwa 10 bis 17. D.h. das Durchschnittsmolekulargewicht der sich wiederholenden Einheiten der Formel Ia beträgt mindestens etwa 396, vorzugsweise von etwa 440 bis etwa 5060 und in erster Linie von etwa 440 bis etwa 748.

Ein Alkylrest R ist in erster Linie Methyl, kann jedoch auch Aethyl, n-Propyl, Isopropyl, n-Butyl oder tert.Butyl darstellen, während m insbesondere für 0 steht.

Die Reste $A_1$, $A_2$ und $A_3$ können untereinander verschieden sein, haben jedoch vorzugsweise die gleiche Bedeutung. Acylreste $A_1$, $A_2$ und $A_3$ sind z.B. die entsprechenden Reste von Carbonsäuren oder von Kohlensäuremonoestern oder -monoamiden.

Ein Acylrest $A_1$, $A_2$ und/oder $A_3$ entspricht z.B. der Formel Z-C(=O)- (Ib), worin Z Wasserstoff oder einen Hydrocarbylrest $R^o$ darstellt, der zusammen mit der Carbonylgruppe den Acylrest einer gegebenenfalls substituierten acyclischen, carbocyclischen, carbocyclisch-acyclischen, heterocyclischen oder heterocyclisch-acyclischen Carbonsäure bildet, oder einen Hydrocarbyloxyrest der Formel $R^o$-O- darstellt, der zusammen mit der Carbonylgruppe den Acylrest einer monoveresterten Kohlensäure bildet, oder einen Hydrocarbylaminorest der Formel $R^o$-N($R^1$)- bedeutet, worin $R^1$ für Wasserstoff steht oder die Bedeutung von $R^o$, insbesondere die unten gegebene Bedeutung hat, und der zusammen mit der Carbonylgruppe-den Acylrest einer mono- oder di-substituierten Carbaminsäure bildet.

Der Hydrocarbylrest $R^o$ ist ein acyclischer, carbocyclischer oder carbocyclisch-acyclischer Kohlenwasserstoffrest, der vorzugsweise höchstens 40, insbesondere höchstens 20 Kohlenstoffatome aufweist und gesättigt oder ungesättigt, unsubstituiert oder substituiert sein kann. Er kann auch anstelle von einem, zwei oder mehreren Kohlenstoffatomen gleiche oder verschiedene Heteroatome, wie insbesondere Sauerstoff, Schwefel und Stickstoff, im acyclischen und/oder cyclischen Teil enthalten; im letzteren Fall kann er als

heterocyclischer oder heterocyclischacyclischer Rest bezeichnet werden.

Ungesättigte Reste sind solche, die eine oder mehrere Doppel- und/oder Dreifachbindungen enthalten. Cyclische Reste, worin mindestens ein 6-gliedriger carbocyclischer oder ein 5- bis 8-gliedriger heterocyclischer Ring die maximale Anzahl nichtkumulierter Doppelbindungen enthält, werden als aromatisch bezeichnet. Carbocyclische Reste, worin mindestens ein Ring als ein 6-gliedriger aromatischer Ring vorliegt, werden als Arylreste bezeichnet.

Wenn nicht anders angegeben, enthalten mit "nieder" bezeichnete organische Reste bis und mit 7, vorzugsweise bis und mit 4 Kohlenstoffatome.

Ein acyclischer Kohlenwasserstoffrest ist insbesondere Alkyl, Alkenyl, Alkadienyl oder Alkynyl, welches verzweigt oder vorzugsweise linear ist, wie Niederalkyl, z.B. Methyl, Ethyl, n-Propyl, Isopropyl, n-Butyl oder tert.-Butyl, ferner n-Pentyl, n-Hexyl oder n-Heptyl, oder höheres Alkyl, z.B. n-Octyl, n-Dodecyl oder n-Hexadecyl, Niederalkenyl, z.B. Allyl oder Methallyl, oder Niederalkinyl, z.B. Propargyl.

Ein carbocyclischer Kohlenwasserstoffrest ist insbesondere ein mono-, bi- oder polycyclischer Cycloalkyl-, Cycloalkenyl- oder Cycloalkadienylrest, oder ein entsprechender, aromatische Ringe enthaltender Arylrest, vorzugsweise ein solcher mit höchstens 12 Ringkohlenstoffatomen und 5-bis 7, vor allem 6-gliedrigen Ringen. Carbocyclisch-acyclische Reste sind solche, in welchen ein acyclischer Rest, insbesondere einer mit bis und mit 7, vorzugsweise mit bis und mit 4 Kohlenstoffatomen, wie Niederalkyl oder -alkenyl, z.B. Methyl, Ethyl oder Vinyl, einen oder mehrere carbocyclische, gegebenenfalls aromatische Reste trägt.

Ein Arylrest ist in erster Linie Phenyl, ferner Naphthyl, wie 1- oder 2-Naphthyl, Biphenylyl, wie insbesondere 4-Biphenylyl, ferner Anthryl, Fluorenyl und Azulenyl, sowie ihre Analogen mit einem oder mehreren gesättigten Ringen. Bevorzugte Aryl-niederalkyl- und -niederalkenylreste sind z.B. Phenyiniederalkyl oder Phenyl-niederalkenyl mit endständigem Phenylrest, wie z.B. Benzyl, Phenethyl, bzw. Styryl und Cinnamyl, ferner auch o-, m- und p-Tolyl.

Heterocyclische Reste, einschliesslich heterocyclisch-acyclische Reste, sind insbesondere monocyclische, aber auch bi- oder polycyclische, aza-, thia-, oxa-, thiaza-, oxaza-, diaza-, triaza- oder tetraza-Reste aromatischen Charakters, sowie entsprechende partiell oder in erster Linie ganz gesättigte heterocyclische Reste dieser Art, wobei solche Reste gegebenenfalls, z.B. wie die obgenannten carbocyclischen Reste oder Arylreste, weitere acyclische, carbocyclische oder heterocyclische Reste tragen und/oder durch funktionelle Gruppen mono-, di- oder polysubstituiert sein können. Der acyclische Teil in heterocyclisch-acyclischen Resten hat z.B. die für die entsprechenden carbocyclisch-acyclischen Reste gegebene Bedeutung. Sofern sich ein Heterocyclyl als direkter Substituent $R^o$ im Rest Z am Sauerstoff oder Stickstoff befindet, geht seine freie Valenz von einem seiner C-Atome aus.

Wie bereits erwähnt, kann ein Hydrocarbylrest (einschliesslich eines Heterocyclylrests) $R^o$ durch einen, zwei oder mehrere gleichartige oder verschiedenartige Substituenten substituiert sein, wobei folgende Substituenten insbesondere in Betracht kommen: freie, veretherte und veresterte Hydroxylgruppen, Mercapto-, sowie Niederalkylthio- und gegebenenfalls substituierte Phenylthiogruppen, Halogenatome, wie Chlor und Fluor, aber auch Brom und Jod, Oxogruppen, die in der Form von Formyl- und Ketogruppen, ferner als entsprechende Acetale bzw. Ketale vorliegen können, und Nitrogruppen, primäre, sekundäre und vorzugsweise tertiäre Aminogruppen, durch übliche Schutzgruppen geschützte primäre oder sekundäre Aminogruppen, Acylaminogruppen und Diacylaminogruppen, sowie gegebenenfalls funktionell abgewandelte Sulfogruppen, wie Sulfamoyl- oder in Salzform vorliegende Sulfogruppen. Diese Gruppen substituieren nicht das Kohlenstoffatom, von dem die freie Valenz zum Sauerstoff ausgeht; vorzugsweise sind sie von dieser, und somit vom Heteroatom, durch mindestens zwei C-Atome getrennt. Der Hydrocarbylrest kann auch freie und funktionell abgewandelte Carboxylgruppen, wie in Salzform vorliegende oder veresterte Carboxylgruppen, gegebenenfalls einen oder mehrere Substituenten aufweisenden Carbamoyl-, Ureido-carbonyl- oder Guanidinocarbonylgruppen, ferner Cyangruppen enthalten.

Eine als Substituent im Hydrocarbyl vorliegende veretherte Hydroxylgruppe ist z.B. Niederalkoxy, wie Methoxy, Ethoxy oder tert-Butyloxy, die ferner z.B. durch Halogenatome, insbesondere in 2-Stellung, oder durch Niederalkoxy, insbesondere in 2-Stellung, wie im 2-Methoxyethoxyrest, substituiert sein kann. Eine besonders bevorzugte Ausgestaltung der veretherten Hydroxylgruppen liegt in Oxaalkylresten vor, in welchen, vorzugsweise lineares, Alkyl anstelle mehrerer C-Atome Sauerstoffatome enthält, die durch mehrere, vor allem zwei, C-Atome voneinander getrennt sind, so dass sie eine, gegebenenfalls mehrfach sich wiederholende Gruppe der Formel $-(CH_2-CH_2-O)_x-$ (Ic), worin x für einen Durchschnittswert von 1 bis 17, z.B. von 1 bis etwa 8, vorzugsweise für 1 bis 4, steht, bilden.

Eine als Substituent im Hydrocarbylrest vorliegende veresterte Hydroxylgruppe kann einen Acylrest mit bis und mit 12 C-Atomen enthalten, der innerhalb dieser Gesamtzahl von C-Atomen analog wie der Rest der Formel Ib substituiert sein kann, kann aber auch durch eine im Hydrocarbylrest ebenfalls vorhandene

Carboxylgruppe lactonisiert sein.

Eine als Substituent im Hydrocarbylrest vorliegende veresterte Carboxylgruppe ist eine durch einen der oben beschriebenen Kohlenwasserstoffreste, vorzugsweise durch einen Niederalkyl- oder Phenylniederalkylrest verestert; Beispiele von veresterten Carboxylgruppen sind insbesondere Methoxy-, Ethoxy-, tert-Butoxy- und Benzyloxycarbonyl, ferner eine lactonisierte Carboxylgruppe.

Eine bevorzugte Aminogruppe ist z.B. eine der Formel

$$R^1-\overset{|}{N}-R^2\ ,$$

worin $R^1$ und $R^2$ unabhängig je Wasserstoff, unsubstituiertes acyclisches $C_1$-$C_7$-Hydrocarbyl, wie insbesondere $C_1$-$C_4$-Alkyl oder $C_1$-$C_4$-Alkenyl, oder monocyclisches, gegebenenfalls durch $C_1$-$C_4$-Alkyl, $C_1$-$C_4$-Alkoxy, Halogen und/oder Nitro substituiertes Aryl, Aralkyl oder Aralkenyl mit höchstens 10 C-Atomen darstellen, wobei zwei kohlenstoffhaltige Reste $R_1$ und $R_2$ über eine Kohlenstoff-Kohlenstoff-Bindung oder über ein Sauerstoff-, Schwefel- oder gegebenenfalls durch Hydrocarbyl, z.B. Niederalkyl, substituiertes Stickstoffatom miteinander verbunden sein können. In einem solchen Fall bilden sie zusammen mit dem Stickstoffatom, an das sie gebunden sind, einen stickstoffhaltigen heterocyclischen Ring.

In einem bevorzugten Acylrest der Formel Ib ist ein Hydrocarbylrest $R^o$ z.B. $C_1$-$C_{19}$-Alkyl oder $C_2$-$C_{19}$-Alkenyl, insbesondere ein solches, das bei mehr als 5 C-Atomen eine lineare Kette aufweist, und das z.B. folgende Substituenten aufweisen kann: Carboxyl, das gegebenenfalls in Salzform oder, funktionell abgewandelt, als Cyan, eine Carbamoylgruppe oder $C_1$-$C_4$-Alkoxycarbonyl vorliegen kann, und das sich vorzugsweise in $\omega$-Stellung befindet, eine Aminogruppe der oben definierten Formel

$$R^1-\overset{|}{N}-R^2\ ,$$

oder ein oder mehrere Halogenatome, insbesondere Fluor oder Chlor, die sich vorzugsweise in $\alpha$-Stellung zur Carbonylgruppe befinden. Ein anderer bevorzugter Acylrest der Formel Ib ist bicyclisches oder insbesondere monocyclisches Aroyl, vor allem Benzoyl, welches gegebenenfalls einen oder mehrere Substituenten enthält, wie Halogen, insbesondere Chlor oder Fluor, Nitro, $C_1$-$C_4$-Alkyl, insbesondere Methyl, Hydroxy und verethertes Hydroxy, insbesondere $C_1$-$C_4$-Alkoxy, wie Methoxy, Phenoxy und Methylendioxy, sowie Carboxyl, das auch in Salzform oder in Form von Cyan oder von $C_1$-$C_4$-Alkoxycarbonyl vorliegen kann. Vorzugsweise weisen Aroylreste nicht mehr als zwei, vor allem nur einen solchen Substituenten auf. Bevorzugt sind auch analoge Heteroaroylreste, insbesondere solche, die Pyridyl, Furyl, Thienyl oder Imidazolyl, oder ihre Analogen mit ankondensiertem Benzoring, wie Chinolinyl, Isochinolinyl, Benzofuryl oder Benzimidazolyl, enthalten, wobei diese gegebenenfalls, wie z.B. oben angegeben, substituiert sein können. Bevorzugte Acylreste dieser Art sind z.B. auch Phenylacetyl oder Cinnamoyl, die z.B. in der oben angegebenen Weise substituiert sein können.

Carbonsäuren, die dem besonders bevorzugten Acylrest der Formel Ib zugrundeliegen, sind beispielsweise aliphatische Monocarbonsäuren mit höchstens 20 Kohlenstoffatomen, wie Niederalkancarbonsäuren, z.B. Essig-, Propion-, Butter-, Isobutter-. Valerian-, Isovalerian, Capron-, Trimethylessig-, Oenanth- oder Diethylessigsäure, sowie Laurin-, Myristin-, Palmitin- und Stearinsäure, sowie Olesäure, Elaidinsäure, Linolsäure und Linolensäure, aber auch entsprechende halogenierte Niederalkancarbonsäuren, wie Trifluoressig-, Chloressig-, Bromessig- oder $\alpha$-Bromisovaleriansäure, carbocyclische oder carbocyclisch-acyclische Monocarbonsäuren, z.B. Cyclopropan-, Cyclopentan- und Cyclohexan-carbonsäure bzw. Cyclopentan- oder Cyclohexan-essigsäure oder -propionsäure, aromatische carbocyclische Carbonsäuren, z.B. Benzoesäure, die einfach oder mehrfach, z.B. wie oben angegeben, substituiert sein kann, Aryl-oder Aryloxyniederalkancarbonsäuren und deren in der Kette ungesättigten Analogen, wie gegebenenfalls, z.B. wie oben für die Benzoesäure angegeben, substituierte Phenylessig-, Phenoxyessig-, Phenylpropion- oder Zimtsäure, sowie heterocyclische Säuren, z B. Furan-2-carbonsäure, 5-tert-Butyl-furan-2-carbonsäure, Thiophen-2-carbonsäure, Nicotin- oder Isonicotinsäure, 4-Pyridinpropionsäure, und gegebenenfalls durch Niederalkylreste substituierte Pyrrol-2- oder Pyrrol-3-carbonsäuren, ferner auch entsprechende $\alpha$-Aminosäuren, insbesondere in der Natur vorkommende $\alpha$-Aminosäuren der L-Reihe, z.B. Glycin, Phenylglycin, Prolin, Leucin, Valin, Tyrosin, Histidin und Asparagin, gegebenenfalls in N-geschützter Form, in welcher die Aminogruppe durch eine konventionelle Aminoschutzgruppe substituiert ist, ferner Dicarbonsäuren, wie Oxal-, Malon-, Mono- oder Di-niederalkylmalon-, Bernstein-, Glutar-, Adipin-, Malein-, Fumar- oder Erucasäure, eine durch Halogen, wie Fluor, Chlor oder Brom, Niederalkyl, Hydroxy, Niederalkoxy und/oder Nitro gegebenenfalls

substituierte Phthal-, Chinolin-, Isochinolin- oder Phenylbernsteinsäure, sowie Glutaminsäure und Asparginsäure, wobei die beiden letztgenannten vorzugsweise mit geschützten Aminogruppen vorliegen. Dabei braucht in Dicarbonsäuren die zweite Carboxylgruppe nicht in freier Form vorzuliegen, sondern kann funktionell abgewandelt sein, z.B in Form eines $C_1$-$C_4$-Alkylesters, eines Amids oder eines Salzes, vorzugsweise in Form eines physiologisch verträglichen Salzes, mit einer salzbildenden basischen Komponente. Als Salze kommen in erster Linie Metall- oder Ammoniumsalze, wie Alkalimetall-, oder Erdalkalimetall-, z.B. Natrium-, Kalium-, Magnesium- oder Calciumsalze, bzw. Ammoniumsalze mit Ammoniak oder geeigneten organischen Aminen, in Betracht.

Ein von einem Monoester der Kohlensäure abgeleiteter Acylrest kann z.B. durch die Formel $R^o$-O-C(=O)- (Id) wiedergegeben werden. Acylreste dieser Art sind z.B. diejenigen, worin $R^o$ die Bedeutung eines acyclischen Hydrocarbylrestes hat, wie $C_1$-$C_{20}$-Alkyl, $C_2$-$C_{20}$-Hydroxyalkyl, dessen Hydroxygruppe sich in beliebiger Stellung ausser in 1-Stellung, vorzugsweise in 2-Stellung, befindet, Cyan-[$C_1$-$C_{20}$]-alkyl, worin Cyan sich vorzugsweise in 1- oder $\omega$-Stellung befindet, oder Carboxy-[$C_1$-$C_{20}$]-alkyl, worin Carboxy sich vorzugsweise in 1- oder $\omega$-Stellung befindet und gegebenenfalls in Salzform oder in Form einer Carbamoylgruppe oder von $C_1$-$C_4$-Alkoxycarbonyl oder Benzyloxycarbonyl vorliegen kann, sowie auch ein lineares (Mono-, Di- bis Hexa)-oxaalkyl mit 4-20 Kettengliedern, worin eines oder mehrere C-Atome, von C-3 an, von linearem $C_4$-$C_{20}$-Alkyl durch Sauerstoffatome, welche voneinander durch mindestens 2 C-Atome getrennt sind und sich vorzugsweise in Stellungen 3-, 6-, 9-, 12-, 15- und 18- befinden, ersetzt ist.

Ein von einer Carbaminsäure abgeleiteter Acylrest kann z.B. durch die Formel $R^o$-N($R^1$)-C(=O)- (Ie) wiedergegeben werden. Als Beispiele solcher Acylreste sind insbesondere diejenigen zu nennen, worin $R^1$ Wasserstoff bedeutet und $R^o$ unsubstituiertes $C_1$-$C_{20}$-Alkyl oder -Alkenyl ist, eine bevorzugte Gruppe von Carbaminsäure-acylresten ist diejenige der Formel $R_a^{'}$ -O-CO-Alk-NH-C-(=O)- (If), worin $R_a^{'}$ -$C_{1-4}$-Alkyl und Alk ein gegebenenfalls durch Hydroxy, $C_{1-4}$-Alkanoyloxy, Amino, Carboxy, $C_{1-4}$-Alkoxycarbonyl, Carbamoyl, Phenyl, Hydroxyphenyl, Methoxyphenyl oder Indolyl substituiertes $C_{1-7}$-Alkylen bedeutet. Dieses kann beliebig verzweigt sein, wobei die beiden freien Valenzen vom gleichen oder von zwei verschiedenen C-Atomen ausgehen können; der Rest kann auch einen der obgenannten Substituenten an einem beliebigen C-Atom tragen. Bevorzugt sind lineare Alkylenreste mit den freien Valenzen an den endständigen C-Atomen, wie Tri- bis Heptamethylen und Ethylen, die vorzugsweise an ihren endständigen C-Atomen Substituenten, wie insbesondere Carbamoyl oder $C_{1-4}$-Alkoxycarbonyl, vor allem Methoxy- und Ethoxycarbonyl, oder primäres Amino, enthalten können; die beiden ersten Substituententypen sind vorzugsweise an dasjenige Ende des Alkylenrestes gebunden, das mit der Aminogruppe verbunden ist, letztere befindet sich vorzugsweise an demjenigen Ende, das mit der Carbonylgruppe verbunden ist. Bevorzugt sind auch lineare oder höchstens einmal verzweigte Alkylenreste, deren zwei freie Valenzen von demselben, vorzugsweise von einem endständigen, C-Atom ausgehen, d.h. 1,1-Alkylidenreste, z.B. insbesondere Methylen, ferner Ethyliden oder 1,1-Propyliden. Diese können z.B. einen der obgenannten Substituenten enthalten, vorzugsweise am endständigen C-Atom, z.B. freies Amino, wie in 4-Amino-1,1-butyliden oder 5-Amino-1,1-pentyliden, Carbamoyl oder $C_{1-4}$-Alkoxycarbonyl, wie z.B. in 2-Carbamoyl-1,1-ethyliden, 2-(Methoxy- oder Ethoxy)-carbonyl-1,1-ethyliden oder entsprechendem, 3-substituiertem 1,1-Propyliden, ferner Hydroxy oder $C_{1-4}$-Alkanoyloxy, z.B. Acetyloxy, welches sich vorzugsweise in 2-Stellung befindet, wie in im 2-Hydroxy-1,1-ethyliden oder 2-Hydroxy-1,1-propyliden, sowie entsprechenden O-acylierten, vor allem O-acetylierten Resten. Cyclische Substituenten befinden sich vorzugsweise am Methylen oder auch in 2-Stellung eines Ethylidenrests.

Besonders bevorzugtes Alkylen ist ein entsprechender Rest, der zusammen mit den Amino- und Carbonylgruppen einen Rest der Teilformel -NH-Alk-C(=O)- (Ig) bildet, welcher der Struktur von natürlichen $\alpha$-Aminosäuren, in Form ihrer individuellen optischen Isomeren oder razemischen Gemischen, entspricht. Entsprechende Acylreste sind diejenigen der Formel If, worin $R_a^{'}$ die obgenannten Bedeutungen hat, und die Teilformel Ig dem Rest einer natürlichen $\alpha$-Aminosäure in Form eines optischen Isomeren oder eines Racemates entspricht. Als optisch individuelle Form ist das in der Natur vorkommende Isomere der L-Reihe bevorzugt, als Isomerengemische die Racemate. In erster Linie steht der Rest der Teilformel Ig für den Glycinrest (-Gly-) und in der Formel If bedeutet $R_a^{'}$ vor allem Methyl oder Ethyl.

Salze von Verbindungen der obigen Formel I mit salzbildenden Eigenschaften leiten sich von denjenigen ab, die in einem Acylrest $A_1$, $A_2$ und/oder $A_3$ eine salzbildende Gruppe, z.B. eine Aminogruppe oder eine Carboxylgruppe als Substituenten aufweisen. Basische Verbindungen der Formel I können Säureadditionssalze, insbesondere pharmazeutisch verwendbare, nicht-toxische Säureadditionssalze mit anorganischen Säuren, z.B. Chlorwasserstoffsäure, Bromwasserstoffsäure, Schwefelsäure oder Phosphorsäuren, oder mit organischen Säuren, wie Sulfonsäuren, z.B. Benzolsulfonsäure, p-Toluolsulfonsäure, Naphthalin-2-sulfonsäure, Methansulfonsäure, Ethansulfonsäure, 2-Hydroxyethansulfonsäure oder Ethan-1,2-disulfonsäure, sowie Carbonsäuren, z.B. Essigsäure, Propionsäure, Glykolsäure, Bernsteinsäure, Maleinsäure, Hydroxymaleinsäure, Benzoesäure, Zimtsäure, Mandelsäure, Salicylsäure, 4-Aminosalicylsäure, 2-Phenoxybenzoesäure, 2-Acetoxy-benzoesäure, Embonsäure, Nicotinsäure oder Isonicotinsäure, saure Verbindungen der Formel I Salze der oben beschriebenen Art bilden.

Metallkomplexe von Verbindungen der Formel I, worin $A_1$, $A_2$ und $A_3$ für Wasserstoff stehen, sind in erster Linie solche mit geeigneten paramagnetischen Uebergangsmetallen, einschliesslich Lanthaniden, sowie mit Metallen der dritten Hauptgruppe des Periodensystems, gegebenenfalls in Form von Radionukliden, in erster Linie mit entsprechenden dreiwertigen Metallen. Zu erwähnen sind insbesondere dreiwertiges Eisen, Mangan und Chrom, als entsprechende Lanthaniden insbesondere das Gadolinium, ferner Dysprosium, dreiwertiges Europium, Holmium, Lanthan und dreiwertiges Ytterbium. Geeignete Metalle der dritten Hauptgruppe des Periodensystems sind vorzugsweise Gallium und Indium, wobei insbesondere die radioaktiven Isotopen, z.B. $^{67}Ga$ und $^{115}In$, in Frage kommen; zusätzliche Radionuklide sind z.B. die radioaktiven Isotopen der obengenannten Metalle, z.B. des dreiwertigen $^{140}La$ oder $^{169}Yb$.

Die Verbindungen der vorliegenden Erfindung weisen wertvolle Eigenschaften auf, diejenigen der Formel I physiologische Wirkungen, die denjenigen von Desferrioxamin B analog sind, weshalb sie für den gleichen Zweck wie dieses verwendbar sind, und die beschriebenen Metallkomplexe von Verbindungen der Formel I, worin $A_1$, $A_2$ und $A_3$ für Wasserstoff stehen, mit den für eine Verwendung als Kontrastmittel geeigneten Eigenschaften, weshalb sie als solche in der Diagnostik verwendet werden können. Der Vorteil von Verbindungen der Formel I, deren Wirkung z.B. an der sogenannten Gallenfistelratte anhand der erhöhten Eisenausscheidung in der Galle und im Urin nachgewiesen werden kann, gegenüber Desferrioxamin B liegt in der wesentlich besseren Löslichkeit, einer Eigenschaft, die sie einer normalen parenteralen Verabreichung zugänglich macht. Zusätzliche Vorteile bilden die Tatsachen, dass es sich um neutrale Verbindungen handelt, die lokal gut verträglich sind, und dass sie im Organismus eine längere Verweildauer aufweisen. Ferner zeigen Verbindungen der Formel I, worin $A_1$, $A_2$ und/oder $A_3$ für einen Acylrest stehen, bei oraler Verabreichung die gewünschte Aktivität. Die neuen Verbindungen der Formel I können deshalb in den für Desferrioxamin B vorgesehenen Indikationen, z.B. zur Behandlung von Funktionsstörungen, bei denen die Konzentration des dreiwertigen Eisens in Körperzellen übernormal hoch ist, z.B. zur Behandlung von Hämochromatose oder Hämosiderose und, weil sie überdies auch Aluminiumionen binden, z.B. bei der Behandlung von Dialyse-Encephalopathie, Osteomalacia und der Alzheimer-Krankheit eingesetzt werden. Die oben erwähnten Metallkomplexe, die in Wasser ausgezeichnet löslich sind und zudem gut vertragen werden, können als Kontrastmittel (sogenannte "image enhancers") in der diagnostischen Medizin, z.B. der Röntgen-, Radionuclid-, Ultraschall- und/oder insbesondere in der magnetischen Resonanzdiagnostik (MRI: magnetic resonance imageing) verwendet werden.

Die Erfindung betrifft in erster Linie Verbindungen der Formel I, worin R, X und m die oben gegebenen Bedeutungen haben, n einen Durchschnittswert zwischen etwa 9 und etwa 115 darstellt, und jeder der Reste $A_1$, $A_2$ und $A_3$, unabhängig von den anderen, Wasserstoff oder den Rest der Formel Z-C(=O)- (Ib) darstellt, worin Z für Wasserstoff steht oder einen Hydrocarbylrest $R^o$ darstellt, der zusammen mit der Carbonylgruppe den Acylrest einer gegebenenfalls substituierten acyclischen, carbocyclischen, carbocyclisch-acyclischen, heterocyclischen oder heterocyclisch-acyclischen Carbonsäure bildet, oder einen Hydrocarbyloxyrest der Formel $R^o$-O- darstellt, der zusammen mit der Carbonylgruppe den Acylrest einer monoveresterten Kohlensäure bildet, oder einen Hydrocarbylaminorest der Formel $R^o$-N($R^1$)- bedeutet, worin $R^1$ für Wasserstoff steht oder die Bedeutung von $R^o$ hat, und der zusammen mit der Carbonylgruppe den Acylrest einer mono- oder di-substituierten Carbaminsäure bildet, oder Salze von solchen Verbindungen mit salzbildenden Eigenschaften, oder Komplexe von solchen Verbindungen, worin $A_1$, $A_2$ und $A_3$ für Wasserstoff stehen, mit dreiwertigen paramagnetischen Uebergangsmetallen, einschliesslich entsprechenden Lanthaniden, Metallen der dritten Hauptgruppe des Periodensystems und Radionucliden.

Die Erfindung betrifft insbesondere Verbindungen der Formel I, worin R, X und m die oben gegebenen Bedeutungen haben, n einen Durchschnittswert von etwa 10 bis etwa 17 darstellt, und jeder der Reste $A_1$, $A_2$ und $A_3$, unabhängig von den anderen, Wasserstoff, Alk(en)oyl mit bis und mit 20 Kohlenstoffatomen, Alkyloxycarbonyl mit bis und mit 20 Kohlenstoffatomen im Alkylteil, worin bis und mit 5 Methylengruppen durch Sauerstoffatome ersetzt sein können, wobei jeweils zwei Kohlenstoffatome die Sauerstoffatome voneinander trennen, oder Alkylaminocarbonyl mit bis und mit 20, vorzugsweise mit bis und mit 7 Kohlenstoffatomen, im Alkylteil, der gegebenenfalls durch Carboxy, Niederalkyloxycarbonyl mit bis und mit 4 Kohlenstoffatomen im Niederalkylteil, Carbamoyl und/oder durch Amino, Hydroxy, Mercapto, Niederalkylthio mit bis und mit 4 Kohlenstoffatomen, Phenyl oder Hydroxyphenyl substituiert sein kann, oder Salze von solchen Verbindungen mit salzbildenden Eigenschaften, und Komplexe von solchen Verbindungen, worin $A_1$, $A_2$ und $A_3$ für Wasserstoff stehen, mit dreiwertigen paramagnetischen Uebergangsmetallen, inklusive entsprechenden Lanthaniden, und geeigneten Metallen der dritten Hauptgruppe des Periodensystems, sowie mit geeigneten Radionucliden.

Die Erfindung betrifft vor allem Verbindungen der Formel I, worin R die oben gegebene Bedeutung hat und in erster Linie für Methyl steht, X die oben gegebene Bedeutung hat und m für 0 steht, n einen Durchschnittswert von etwa 10 bis etwa 17, insbesondere von etwa 11 bis etwa 12 darstellt, und jeder der Reste $A_1$, $A_2$ und $A_3$, unabhängig von den anderen, in erster Linie Wasserstoff bedeutet, ferner für Alkanoyl mit bis und mit 12, vorzugsweise mit 6 bis und mit 12 Kohlenstoffatomen, z.B. Octanoyl, für Alkyloxycarbonyl, worin Alkyl bis und mit 7, vorzugsweise bis und mit 4 Kohlenstoffatome enthält, wobei eine oder zwei Methylengruppen durch Sauerstoff ersetzt sein können, und Sauerstoffatome jeweils durch zwei Kohlenstoffatome voneinander getrennt sind, und z.B. Methyl oder Ethyl bedeutet, oder für Alkylaminocarbonyl stehen kann, worin Alkyl bis und mit 7, vorzugsweise bis und mit 4 Kohlenstoffatome enthält, das als Substituenten in 2-, insbesondere in 1-Stellung, gegebenenfalls Alkyloxycarbonyl aufweist, worin Alkyl bis und mit 4 Kohlenstoffatome enthält und z.B. Methyl oder Ethyl darstellt, insbesondere Alkyloxycarbonylmethylaminocarbonyl, worin Alkyl bis und mit 4 Kohlenstoffatome aufweist und z.B. für Methyl oder Ethyl steht, und Komplexe von solchen Verbindungen, worin $A_1$, $A_2$ und $A_3$ für Wasserstoff stehen, mit dreiwertigen paramagnetischen Uebergangsmetallen, einschliesslich entsprechenden Lanthaniden, und mit Metallen der dritten Hauptgruppe des Periodensystems, sowie mit geeigneten Radionucliden.

Vor allem betrifft die Erfindung Verbindungen der Formel I, worin R für Methyl steht, X die oben gegebene Bedeutung hat und m für 0 steht, n einen Durchschnittswert von etwa 10 bis etwa 17, insbesondere von etwa 11 bis etwa 12 hat, und jeder der Reste $A_1$, $A_2$ und $A_3$ für Wasserstoff steht, und Komplexe von solchen Verbindungen mit dreiwertigen paramagnetischen Uebergangsmetallen, einschliesslich Lanthaniden, insbesondere mit Eisen-III und Mangan-III, sowie Gadolinium-III, oder mit Metallen der dritten Hauptgruppe des Periodensystems, insbesondere mit Gallium-III und Indium-III, sowie mit geeigneten Radionucliden.

Die Erfindung betrifft in erster Linie die in den Beispielen beschriebenen Verbindungen.

Die Verbindungen der Formel I können in an sich bekannter Weise, u.a. nach aus der Peptidchemie allgemein bekannten Analogieverfahren, hergestellt werden, z.B. indem man eine Verbindung der Formel

$R—O—(CH_2\text{-}CH_2\text{-}O)_n\text{-}Y_1$ (III)

mit einer Verbindung der Formel

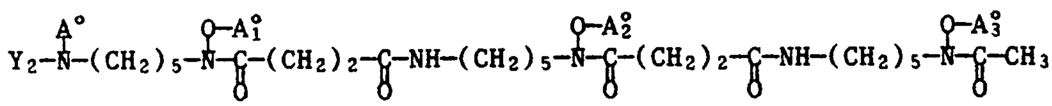 (IV)

oder ein Salz davon umsetzt, wobei (a) $Y_2$ für Wasserstoff steht, und $Y_1$ eine Gruppe der Formel $-X-Z_1$ (IIIa) bedeutet, in welcher $Z_1$ eine, zusammen mit dem Wasserstoff $Y_2$ unter Bildung der Bindung zwischen den Reaktionsteilnehmern abspaltbare Gruppe Z darstellt, oder (b) $Y_1$ für Wasserstoff steht, und $Y_2$ eine Gruppe der Formel $Z_2$-X- (IVa) bedeutet, worin $Z_2$ eine Gruppe Z darstellt, oder, falls m in einem Rest X für 0 steht, zusammen mit $A°$ eine Bindung bildet, und worin $A°$ Wasserstoff oder, falls $Y_2$ Wasserstoff bedeutet, eine Aminoschutzgruppe darstellt, und jeder der Reste $A_1°$, $A_2°$ und $A_3°$, unabhängig voneinander, je für Wasserstoff, eine geeignete Schutzgruppe oder einen Acylrest steht, wobei in Acylresten $A_1°$, $A_2°$ und $A_3°$ funktionelle Gruppen gegebenenfalls in geschützter Form vorliegen, und, wenn erwünscht oder notwendig, in einer erfindungsgemäss erhältlichen Verbindung vorhandene Schutzgruppen abspaltet, und, wenn erwünscht, in einer erfindungsgemäss erhältlichen Verbindung der Formel I, in welcher mindestens eine der Gruppen $A_1$, $A_2$ und $A_3$ Wasserstoff bedeutet, diesen durch einen Acylrest ersetzt, und/oder, wenn erwünscht, eine erfindungsgemäss erhältliche Verbindung der Formel I, worin $A_1$, $A_2$ und $A_3$ für Wasserstoff stehen, in einen Metallkomplex umwandelt, und/oder, wenn erwünscht, ein erfindungsgemäss erhältliches Salz einer salzbildenden Verbindung der Formel I in die freie Verbindung bzw. eine erfindungsgemäss erhältliche Verbindung mit salzbildenden Eigenschaften in ein Salz überführt.

Eine zusammen mit Wasserstoff unter Bildung der gewünschten Bindung abspaltbare Gruppe Z ist z.B. reaktionsfähiges verestertes Hydroxy, insbesondere mit einer starken, vorzugsweise anorganischen, Säure verestertes Hydroxy, wie Halogen (Ester mit Halogenwasserstoffsäure) mit einer Atomzahl von mindestens 19, in erster Linie Chlor, ferner Brom oder Jod, oder Azido (Ester mit Stickstoffwasserstoffsäure). Weitere abspaltbare Gruppen Z sind geeignete, über ein Ringstickstoffatom gebundene, vorzugsweise monocyclische, in erster Linie pentacyclische, azacyclische, z.B. diazacyclische, Gruppen, wie 1-Imidazolyl. Letztere werden üblicherweise in Ausgangsstoffen der Formeln III bzw. IV verwendet, in denen m im Rest X der

Teilformeln IIIa bzw. IVa für O steht.

Schutzgruppen $A_1^\circ$ , $A_2^\circ$ und/oder $A_3^\circ$ sind in erster Linie geeignete organische Silylgruppen, wie z.B. Gruppen der Formel $(R_a)(R_b)(R_c)Si-$ (IVb), worin $R_a$ und $R_b$ unabhängig voneinander einen Kohlenwasserstoffrest, z.B. Niederalkyl, wie Ethyl, tert.-Butyl, n-Pentyl, Esopentyl oder n-Hexyl, insbesondere Methyl, oder gegebenenfalls Niederalkyl-substituiertes Phenyl oder Phenylniederalkyl, z.B. Phenyl, p-Tolyl, Benzyl oder Phenylethyl, bedeuten, vorzugsweise jedoch für gleiche Substituenten stehen, und $R_c$ für Halogen, insbesondere Chlor, oder einen der für $R_a$ bzw. $R_b$ genannten Kohlenwasserstoffreste, insbesondere Methyl steht. Geeignete Silylgruppen sind z.B. Tribenzylsilyl, Phenyl-dimethylsilyl, Benzyl-dimethylsilyl, Hexyl-dimethylsilyl, tert-Butyl-dimethylsilyl, Triethylsilyl, Diethyl-chlorsilyl, insbesondere Dimethyl-chlorsilyl und vor allem Trimethylsilyl.

Eine Aminoschutzgruppe $A^\circ$, welche die Reaktionsfähigkeit der Aminogruppe nicht beeinträchtigt, ist vorzugsweise ebenfalls eine organische Silylgruppe, wie eine der obgenannten, insbesondere Dimethylchlorsilyl und vor allem Trimethylsilyl. Eine solche hat überdies eine aktivierende Wirkung auf die Reaktionsfähigkeit der Aminogruppe und kann deshalb auch als Aminoaktivierungsgruppe dienen.

Die Reaktion wird in an sich bekannter Weise durchgeführt. Die Variante (a), laut welcher ein Ausgangsmaterial der Formel III, worin $Y_1$ für den Rest der Formel IIIa steht, wobei $Z_1$ eine reaktionsfähige veresterte Hydroxygruppe, insbesondere Halogen und in erster Linie Chlor darstellt, mit einem Ausgangsmaterial der Formel IV umgesetzt wird, worin $Y_2$ für Wasserstoff steht, und $A_1^\circ$ , $A_2^\circ$ und $A_3^\circ$ von Wasserstoff verschieden sind, wobei entsprechende Schutzgruppen üblicherweise für organische Silylgruppen, insbesondere für Dimethylchlorsilyl und in erster Linie für Trimethylsilyl stehen, und in Acylresten funktionelle Gruppen, die mit einer Verbindung der Formel III in Reaktion treten könnten, vorzugsweise in geschützter Form vorliegen, und $A^\circ$ vorzugsweise eine Aminoschutzgruppe, üblicherweise eine organische Silylgruppe, insbesondere Dimethylchlorsilyl und in erster Linie Trimethylsilyl darstellt, wird vorzugsweise unter basischen Bedingungen in Gegenwart einer geeigneten säurebindenden, aprotischen Base durchgeführt. Solche sind z.B. entsprechende organische Basen, z.B. tertiäre Amine, wie Triniederalkylamine, z.B. Triethylamin, Ethyl-diisopropyl-amin oder Tributylamin, Diniederalkylanilin, z.B. N,N-Dimethylanilin oder N,N-Diethylanilin, N-Niederalkyl(oxa- oder aza)niederalkylenamine, z.B. N-Methyl-piperidin, N-Ethyl-piperidin, N-Methyl-morpholin, N-Ethyl-morpholin oder 1,4-Dimethyl-piperazin, oder stickstoffhaltige heteroaromatische Basen, z.B. Pyridin, Collidin oder Chinolin. Die Reaktion wird vorzugsweise unter Ausschluss von Wasser, üblicherweise in Gegenwart eines geeigneten aprotischen Lösungsmittels oder Lösungsmittelgemisches, wenn notwendig, unter Kühlen oder Erwärmen und/oder unter einer Inertgasatmosphäre durchgeführt.

Falls ein Ausgangsmaterial der Formel III, worin $Y_1$ den Rest der Formel IIIa bedeutet, wobei m in der Teilformel für X den Wert 0 hat, und $Z_1$ für eine über ein Ringstickstoffatom gebundene azacyclische Gruppe, wie 1-Imidazolyl steht, kann die Reaktion mit einer Verbindung der Formel IV durchgeführt werden, worin $A_1^\circ$ , $A_2^\circ$ und/oder $A_3^\circ$ ausser für einen Acylrest oder eine Schutzgruppe auch für Wasserstoff stehen können, und worin $A^o$ ausser einer Aminoschutzgruppe ebenfalls Wasserstoff bedeuten kann. Die Reaktion wird in Gegenwart eines Lösungsmittels oder Lösungsmittelgemisches durchgeführt, wobei auch protische Lösungsmittel, inkl. Wasser verwendet werden können, ferner, wenn notwendig, unter Kühlen oder Erwärmen und/oder in einer Inertgasatmosphäre durchgeführt.

Entsprechend wird die Reaktion eines Ausgangsmaterials der Formel III, worin $Y_1$ für Wasserstoff steht, mit einem solchen der Formel IV, worin $Y_2$ für den Rest der Formel IVa steht, durchgeführt, wobei $Z_2$ vorzugsweise für eine veresterte Hydroxygruppe, insbesondere für Halogen, z.B. Chlor, steht. Wie erwähnt kann, falls m in der Teilformel für X den Wert 0 hat, $Z_2$ zusammen mit $A^o$ eine Bindung bilden; in diesem Fall bildet in dem an der Reaktion teilnehmenden Ausgangsmaterial der Formel IV die Gruppe $Z_2$ im Rest der Formel IVa zusammen mit dem, den Rest $Y_2$ enthaltenden Stickstoffatom, eine Isocyanatogruppe. Sinngemäss werden die Reaktionsbedingungen für die Variante (a) des allgemeinen Verfahrens auch für dessen Variante (b) angewandt.

Die in den verfahrensgemäss erhältlichen Verbindungen vorhandenen geschützten funktionellen Gruppen können in an sich bekannter Weise freigesetzt werden. Insbesondere können die durch organische Silylgruppen geschützten Gruppen, wie z.B. die Hydroxygruppen im Desferrioxaminteil von Verbindungen der Formel I, ferner auch die eine Silylgruppe enthaltende Aminogruppe durch Solvolyse im Verlauf des Aufarbeitungsverfahrens freigesetzt werden, z.B. indem man das Reaktionsprodukt mit einem protischen Reagens, wie einem Niederalkanol, z.B. Methanol oder Ethanol, und/oder Wasser behandelt, wobei die Solvolyse gegebenenfalls unter Zugabe einer geeigneten Säure, z.B. Chlorwasserstoff, auch säurekatalytisch erfolgen kann.

Ueblicherweise werden die Ausgangsstoffe der Formeln III und IV, sofern $Y_1$ bzw. $Y_2$ von Wasserstoff verschieden sind, und die Ausgangsstoffe der Formel IV, worin $A_1^\circ$ , $A_2^\circ$ und $A_3^\circ$ Silylschutzgruppen, und/oder $A^o$ eine Silylgruppe darstellen, <u>in situ</u> oder unmittelbar vor der eigentlichen Reaktion und ohne zusätzliche

Aufarbeitung hergestellt.

So kann man z.B. eine Verbindung der Formel III, worin $Y_1$ für Wasserstoff steht, mit einem geeigneten Kohlensäurederivat, wie einem Kohlensäuredihalogenid, z.B. Phosgen, oder mit einem geeigneten Kohlensäurediamid, worin der Amidteil einen über ein Ringstickstoffatom gebundenen aza-cyclischen Rest, z.B. 1,1'-Carbonyl-bis-1H-imidazol darstellt, vorzugsweise in einem geeigneten Verdünnungsmittel oder einem Gemisch davon umsetzen. Man erhält so ein Ausgangsmaterial der Formel III, worin $Y_1$ den Rest der Formel IIIa bedeutet, m in der Teilformel für X den Wert 0 hat, und $Z_1$ die oben gegebene Bedeutung hat; ein solches Ausgangsmaterial wird üblicherweise ohne besondere Aufarbeitung und Reinigung mit dem Reaktionspartner der Formel IV umgesetzt. Ein Ausgangsmaterial der Formel III, worin $Y_1$ den Rest der Formel IIIa darstellt, in welcher m in der Teilformel für X den Wert 1 hat, und $Z_1$ z.B. für Halogen, insbesondere Chlor steht, kann man in situ herstellen, indem man eine Verbindung der Formel III, worin $Y_1$ für Wasserstoff steht, mit einem Halogen-, z.B. Chlorsulfonylisocyanat, umsetzt. Wird die so erhältliche Verbindung als Ausgangsmaterial verwendet, so kann man zu Verbindungen der Formel I gelangen, worin m in der Teilformel für X den Wert 1 hat, und die Gruppe der Teilformel -X-NH- für den bivalenten Rest der Formel $-C(=O)-NH-S(O)_2-NH-$ steht.

In analoger Weise können die Ausgangsstoffe der Formel IV, worin $Z_2$ in der Teilformel IVa von Wasserstoff verschieden ist, hergestellt werden; dabei werden üblicherweise Desferrioxamin B bzw. Derivate davon eingesetzt, worin vorhandene funktionelle Gruppen, die an der Reaktion teilnehmen könnten, in geschützter Form vorliegen. Ausgehend von einem geeigneten Zwischenprodukt kann man z.B. zu Verbindungen der Formel IV gelangen, worin m in der Teilformel für X den Wert 1 hat und die Gruppe der Teilformel -X-NH- für den bivalenten Rest der Formel $-S(O)_2-NH-C(=O)-NH-$ steht, indem man das Zwischenprodukt mit der reaktionsfähigen Aminogruppe z.B. mit einem Halogensulfonylisocyanat, wie Chlorsulfonylisocyanat, umsetzt.

In einem Ausgangsmaterial der Formel IV, worin mindestens einer der Reste $A_1$, $A_2$ und $A_3$ für Wasserstoff steht, werden vorhandene Hydroxygruppen vorzugsweise mittels organischen Silylgruppen der Formel IVb geschützt, wobei gleichzeitig auch die Aminogruppe in einem solchen Ausgangsmaterial, worin $Y_2$ und $A^o$ für Wasserstoff stehen, silyliert und dadurch aktiviert werden kann. Dabei wird eine solche Verbindung der Formel IV oder ein Säureadditionssalz davon in Gegenwart einer aprotischen organischen Base, wie einer der obgenannten, vor allem Pyridin, mit einem geeigneten Silylierungsreagens, insbesondere einem Silylhalogenid der Formel $(R_a)(R_b)(R_c)Si-Hal$ (V), worin $R_a$, $R_b$ und $R_c$ die oben genannten gegebenen Bedeutungen haben und Hal für Brom oder insbesondere Chlor steht, umsetzt. Besonders bevorzugte Silylierungsreagentien sind z.B. Triniederalkylsilylchloride, wie Trimethylsilylchlorid, oder auch ein Diniederalkyldichlorsilan, wie Dimethyldichlorsilan. Das Silylierungsmittel wird üblicherweise in überschüssiger Menge zugesetzt; seine Gegenwart beeinträchtigt die Hauptreaktion, d.h. das Umsetzen mit der Komponente der Formel III nicht; im Gegenteil, damit können z.B. Spuren von störender Feuchtigkeit entfernt werden. Deshalb kann die Hauptreaktion im Anschluss an die Silylierung im gleichen Reaktionsmedium vorgenommen und zusätzlich mit der nachfolgenden solvolytischen Abspaltung der Silylgruppen zusammengelegt werden, so dass alle 3 Stufen (Herstellung des Ausgangsstoffes der Formel IV, Behandeln mit Komponente der Formel III und Abspalten der Silylgruppen) im gleichen Reaktionsmedium duchgeführt werden können.

Zum vorübergehenden Schutz von in Acylresten $A_1$, $A_2$ und/oder $A_3$ in einem Ausgangsmaterial der Formel IV gegebenenfalls vorhandenen funktionellen Gruppen, wie Aminogruppen, eignen sich die üblichen Schutzgruppen, wie Aminoschutzgruppen, die z.B. bei der Synthese von Peptiden verwendet werden, und die, einschliesslich der entsprechenden Abspaltungsmethoden, in Uebersichtsreferaten und Nachschlagewerken, wie Houben-Weyl, Methoden der organischen Chemie (4. Auflage), Band 15/I und II, E. Wünsch (Herausgeber), Synthese von Peptiden (Georg Thieme Verlag, Stuttgart; 1974), ausführlich beschrieben sind. Vorzugsweise werden acidolytisch oder neutral abspaltbare Schutzgruppen verwendet.

Geeignete Aminoschutzgruppen sind, abgesehen von den unter Umständen auch in Frage kommenden, obgenannten organischen Silylgruppen, z.B. gegebenenfalls substituiertes Trityl, das z.B. durch Behandeln mit 50 %-iger Essigsäure abspaltbar ist, 2-Nitrophenylsulfenyl, das z.B. durch eine säurekatalysierte Solvolyse oder Acidolyse, z.B. durch Behandeln mit Pyridinhydrochlorid, abspaltbar ist, gegebenenfalls substituiertes Benzyloxycarbonyl, das z.B. unter neutralen Bedingungen hydrogenolytisch oder acidolytisch abgespalten werden kann, tert-Butyloxycarbonyl, das acidolytisch abspaltbar ist, oder Allyloxycarbonyl, das sich acidolytisch oder unter milden neutralen Bedingungen durch Behandeln mit Dimedon, oder durch die reduktive Einwirkung von Tributylzinnhydrid unter Katalyse mit dem Palladium-(O)-tetrakis-(triphenylphosphin)-Komplex abspalten lässt.

Im Ausgangsmaterial der Formel IV enthaltene freie Carboxygruppen werden üblicherweise in Form von veresterten Carboxygruppen geschützt, die üblicherweise mittels konventioneller Hydrolyse, vor allem unter

Einwirkung von Basen, wie Alkalimetallhydroxiden, -carbonaten der -hydrocarbonaten, geeignete Ester auch mittels anderer Methoden, wie Ester mit tertiären Alkoholen, z.B. tert-Butanol, durch Acidolyse, z.B. mittels Fluorwasserstoff oder Trifluoressigsäure, oder Ester mit Benzylalkoholen mittels konventioneller Hydrogenolyse, gespalten werden können. Carboxygruppen können z.B. auch in der Form von Silylestern geschützt werden, die als veresternde Gruppierung z.B. die obgenannten organischen Silylgruppen enthalten und in an sich bekannter Weise, d.h. solvolytisch, gespalten werden.

Vorhandene Hydroxylgruppen können z.B. in Form von Estern mit Carbonsäuren, wie mit Niederalkansäuren oder mit Monoestern der Kohlensäure, z.B. Formiate oder Acetate bzw. tert-Butyloxy- oder Benzyloxy-carbonate, oder in Form von Ethern, wie solchen mit tertiären Alkoholen, z.B. tert-Butanol, oder in Form von Acetalen, z.B. als 2-Tetrahydropyranylether, geschützt sein. Erstere werden üblicherweise analog wie die veresterten Carboxylgruppen, die beiden letzteren Typen werden z.B. mittels Acidolyse gespalten.

Nach stattgefundener Reaktion können in geschützter Form vorliegende funktionelle Gruppen in an sich bekannter Weise, z.B. wie beschrieben, freigesetzt werden.

Gewünschtenfalls kann man in erfindungsgemäss erhältlichen Verbindungen der Formel I, worin mindestens ein Rest $A_1$, $A_2$ und $A_3$ Wasserstoff bedeutet, diesen durch einen Acylrest ersetzen, indem man solche Verbindungen mit einen Acylrest einführenden Mitteln behandelt. Solche sind z.B. Anhydride von entsprechenden Säuren, worunter symmetrische, gemischte und innere Anhydride zu verstehen sind. Gemischte Anhydride sind z.B. solche von Carbonsäuren mit starken anorganischen Säuren, wie Halogen-, z.B. insbesondere Chlor-, ferner Brom- oder Iodwasserstoffsäure (d.h. Säurehalogenide), ferner Phosphor- oder Schwefelsäure, sowie Stickstoffwasserstoffsäure, oder mit geeigneten organischen Säuren, wie Kohlensäure-niederalkyl-, wie -ethylhalbestern, oder Trifluoressigsäure. Innere Anhydride sind z.B. Ketene (innere Anhydride von Carbonsäuren) oder Isocyanatoverbindungen (innere Anhydride von Carbaminsäureverbindungen). Weitere Acylierungsmittel sind z.B. geeignete aktivierte Ester und Amide von Carbonsäuren, z.B. entsprechende Cyanmethyl- oder Pentachlorphenylester, sowie Ester mit heterocyclischen N-Hydroxy-Verbindungen, z.B. N-Hydroxy-succinimid oder N-Hydroxy-benztriazol, ferner Amide von Carbonsäuren, z.B. 1-Imidazolide. Ferner kann auch eine freie Säure in Gegenwart eines geeigneten Kondensationsmittels, z.B. Dicyclohexylcarbodiimid, als acylierendes Mittel verwendet werden.

Die Acylierung wird in an sich bekannter Weise durchgeführt, wenn notwendig, unter Kühlen oder Erwärmen, z.B. im Temperaturbereich von etwa -10 bis etwa +100°C, und/oder unter erhöhtem Druck, und/oder in einer Inertgasatmosphäre, in heterogener Phase, wie in Suspension, oder in homogener flüssiger Phase unter Verwendung von geeigneten Lösungsmitteln, und gegebenenfalls in Gegenwart von säurebindenden Mitteln, wie organischen stickstoffhaltigen Basen, z.B. tertiären Aminen, wie Triethylamin, Ethyldiisopropylamin, N,N-Dimethylanilin, N-Ethylpiperidin oder N,N'-Dimethylpiperazin, oder aromatischen heterocyclischen Basen, z.B. Pyridin, Collidin, Chinolin oder 4-Dimethylaminopyridin, ferner basischen anorganischen Verbindungen, wie Alkalimetallhydroxiden, -carbonaten und -hydrocarbonaten, sowie Salzen von Carbonsäuren, wie Natrium- oder Kaliumacetat. Ferner können neutral reagierende, stickstoffhaltige Verbindungen, die unter Umständen auch als Lösungsmittel eingesetzt werden können, z.B. Carbonsäureamide, wie Dimethylformamid oder N-Methylpyrrolidon, sowie Urethane oder Harnstoff verwendet werden.

Falls notwendig, sind im Acylierungsreagens vorhandene freie funktionelle Gruppen in geschützter Form vorhanden und können nach der Acylierungsreaktion freigesetzt werden; Schutzgruppen sind z.B. die oben erwähnten und ihre Abspaltung erfolgt z.B. nach den angegebenen Verfahren.

Die Komplexe von Verbindungen der Formel I, worin $A_1$, $A_2$ und $A_3$ für Wasserstoff stehen, mit Metallen werden in an sich bekannter Weise hergestellt, indem man solche Verbindungen mit einer geeigneten Metallverbindung, wie einem anorganischen oder organischen Salz oder Derivat davon umsetzt, wobei Ausgangsmaterial und Metallreagens üblicherweise in Form von entsprechenden Lösungen verwendet werden. Salze sind z.B. anorganische oder organische Metallsalze, wie entsprechende Metallhalogenide, z.B. Chloride, u.a. Eisen-III-chlorid oder Mangan-III-chlorid, oder -sulfate, z.B. mit Ammoniumsulfat komplexiertes Eisen-III-sulfat. Derivate sind u.a. Komplexe mit gewissen organischen Verbindungen, bevorzugt insbesondere Komplexe mit geeigneten $\beta$-Dicarbonylverbindungen, deren Bindungsaffinität zu den Metallionen niedriger ist, als diejenige der Verbindungen der Formel I, worin $A_1$, $A_2$ und $A_3$ für Wasserstoff stehen (d.h. der negative dekadische Logarithmus der Dissoziationskonstanten (pK) für die Komplexe von letzteren mit den Metallionen muss grösser sein als derjenige für die Komplexe der $\beta$-Dicarbonylverbindungen und den Metallionen). Solche $\beta$-Dicarbonylverbindungen sind üblicherweise aliphatischen und cycloaliphatischen Charakters, wobei mindestens eine der beiden Carbonylgruppen, die sich in 1- und 3-Stellung zueinander befinden, in der Enolform vorliegen kann, und die zwei Carbonylgruppen für die Komplexierung eines Metallions verfügbar und nicht sterisch gehindert sind. Eine besonders bevorzugte 1,3-Dicarbonylverbindung ist z.B. Acetylaceton; Acetylacetonate zahlreicher Metalle sind im Handel erhältlich.

Diese Derivate werden vorzugsweise in Form von Lösungen in Lösungsmitteln oder Lösungsmittelgemischen eingesetzt, die mit Wasser nicht beliebig mischbar sind. So sind z.B. die oben erwähnten Metallacetylacetonate in einem mit Wasser nicht beliebig, praktisch nicht mischbaren Niederalkancarbonsäureniederalkylester, wie Essigsäureethylester, in einem entsprechenden, vorzugsweise acyclischen Ether, wie Diethylether, oder in einem unsubstituierten oder halogenierten Kohlenwasserstoff, z.B. einem aromatischen Kohlenwasserstoff, wie Benzol oder Toluol, einem aliphatischen Kohlenwasserstoff, wie Pentan oder Heptan, oder einem halogenierten Kohlenwasserstoff, wie Chloroform oder Methylenchlorid, löslich.

Da die verfahrensgemäss erhältlichen Komplexe von Verbindungen der Formel I, worin $A_1$, $A_2$ und $A_3$ für Wasserstoff stehen, in solchen Lösungsmitteln nicht, in Wasser jedoch gut löslich sind, wird die Reaktion üblicherweise so durchgeführt, dass man eine Lösung des Komplexes aus der $\beta$-Dicarbonylverbindung und dem Metallion in einem mit Wasser höchstens teilweise mischbaren Lösungsmittel mit einer Lösung oder Suspension der Verbindung der Formel I, worin $A_1$, $A_2$ und $A_3$ für Wasserstoff stehen, in Wasser versetzt, und das Gemisch gerührt. Die Reaktanden können in äquivalenten Mengen eingesetzt werden; man kann jedoch auch einen leichten Ueberschuss, z.B. 10-20 %, des Komplexes mit der $\beta$-Dicarbonylverbindung verwenden. Die Reaktion wird vorzugsweise bei einer Temperatur zwischen etwa -20°C und etwa +150°C, vornehmlich zwischen etwa +10°C und etwa +70°C, hauptsächlich bei Raumtemperatur durchgeführt. Die im Einzelfall anzuwendende Reaktionstemperatur hängt unter anderem vom Schmelz- bzw. Siedepunkt des Lösungsmittel(gemische)s, von der Stabilität der Reaktanden und des gebildeten Komplexes und der gewünschten Reaktionsgeschwindigkeit ab. Wenn erwünscht oder erforderlich, kann die Reaktion unter Druck und/oder in einer Inertgasatmosphäre durchgeführt werden.

Je nach Arbeitsweise erhält man erfindungsgemässe Verbindungen der Formel I mit salzbildenden Eigenschaften in freier Form oder in Form von Salzen. Aus diesen können jene in an sich bekannter Weise freigesetzt werden, Säureadditionssalze z.B. durch Behandeln mit geeigneten Basen, und Salze mit Basen z.B. durch Behandeln mit geeigneten Säuren. Saure Verbindungen der Formel I können z.B. durch Behandeln mit einer geeigneten Base, wie einem Alkalimetall- oder Erdalkalimetallhydroxid oder -carbonat, oder mit Ammoniak oder einer organischen Base, z.B. einem Amin, basische Verbindungen der Formel I z.B. durch Behandeln mit einer geeigneten Säure, wie einer anorganischen oder organischen Säure, in die entsprechenden Salze umgewandelt werden.

Infolge der engen Beziehung zwischen den neuen Verbindungen in freier Form und in Form ihrer Salze, inkl. auch solchen, die als Zwischenprodukte, z.B. bei zur Reinigung oder zur Identifikation verwendet werden können, sind vorausgehend und nachfolgend unter den freien Verbindungen sinn- und zweckgemäss gegebenenfalls auch die entsprechenden Salze zu verstehen.

Beim Verfahren der vorliegenden Erfindung werden vorzugsweise solche Ausgangsstoffe verwendet, welche zu den eingangs als besonders wertvoll geschilderten Verbindungen führen.

Die Erfindung betrifft auch diejenigen Ausführungsformen des Verfahrens, bei denen ein Ausgangsstoff unter den Reaktionsbedingungen gebildet oder in Form eines Derivats davon, zum Beispiel eines Salzes, verwendet wird.

Die vorliegende Erfindung betrifft ebenfalls Zusammensetzungen, wie pharmazeutische Präparate, die als Wirkstoff eine der erfindungsgemässen Verbindungen der Formel I enthalten. Besonders bevorzugt sind pharmazeutische Präparate und Zusammensetzungen zur parenteralen, wie insbesondere intravenösen, subkutanen und intramuskularen, ferner zur enteralen, wie oralen oder rektalen Verabreichung. Die Präparate enthalten den Wirkstoff allein oder vorzugsweise zusammen mit pharmakologisch verträglichen Hilfsstoffen. Die Dosierung des Wirkstoffs hängt von der zu behandelnden Krankheit, sowie vom Individuum, dessen Alter, Gewicht und/oder Zustand, sowie von der Anwendungsweise ab; im allgemeinen entspricht sie jedoch mengenmässig etwa derjenigen, die für eine Dauerinfusion mit Desferrioxamin B oder einem Salz davon verwendet wird.

Die pharmazeutischen Zusammensetzungen enthalten von etwa 5 % bis etwa 95 % des Wirkstoffs, wobei einzeldosierte Applikationsformen vorzugsweise von etwa 20 % bis etwa 90 % und nicht-einzeldosierte Applikationsformen, wie Injektonslösungen, vorzugsweise von etwa 5 % bis etwa 30 % an Wirkstoff aufweisen; pharmazeutische Präparate in Dosiseinheitsform, wie Dragées, Tabletten oder Kapseln, bzw. Suppositorien, enthalten von etwa 0,1 g bis etwa 3,0 g, vorzugsweise von etwa 0,3 g bis etwa 1,0 g des Wirkstoffs.

Die pharmazeutischen Zusammensetzungen der vorliegenden Erfindung werden in an sich bekannter Weise, z.B. mittels konventioneller Misch-, Granulier-, Dragier-, Lösungs-, oder Lyophilisierungsverfahren hergestellt. So kann man pharmazeutische Zusammensetzungen zur oralen Anwendung erhalten, indem man den Wirkstoff mit einem oder mehreren festen Trägerstoffen kombiniert, ein erhaltenes Gemisch gegebenenfalls granuliert, und das Gemisch bzw. Granulat, wenn erwünscht, gegebenenfalls durch Zugabe von zusätzlichen Hilfsstoffen, zu Tabletten oder Dragées-Kernen verarbeitet. Injektionslösungen werden

vorzugsweise durch Lösen des Wirkstoffes in deionisiertem pyrogenfreiem Wasser, gegebenenfalls unter Zusatz von Puffermitteln und Konservierungsmitteln hergestellt, sterilfiltriert und je nach Bedarf abgefüllt und lyophilisiert.

Geeignete Trägerstoffe sind insbesondere Füllstoffe, wie Zucker, z.B. Lactose, Saccharose, Mannit oder Sorbit, Cellulosepräparate und/oder Calciumphosphate, z.B. Tricalciumphosphat oder Calciumhydrogenphosphat, ferner Bindemittel, wie Stärken, z.B. Mais-, Weizen-, Reis- oder Kartoffelstärke, Methylcellulose, Hydroxypropylmethylcellulose, Natriumcarboxymethylcellulose und/oder Polyvinylpyrrolidon, und/oder, wenn erwünscht, Sprengmittel, wie die obgenannten Stärken, ferner Carboxymethylstärke, quervernetztes Polyvinylpyrrolidon, Alginsäure oder ein Salz davon, wie Natriumalginat. Zusätzliche Hilfsmittel sind in erster Linie Fliessregulier- und Schmiermittel, z.B. Kieselsäure, Talk, Stearinsäure oder Salze davon, wie Magnesium- oder Calciumstearat, und/oder Polyethylenglykol.

Dragées-Kerne werden mit geeigneten, gegebenenfalls Magensaft-resistenten Ueberzügen versehen, wobei man u.a. konzentrierte Zuckerlösungen, welche gegebenenfalls arabischen Gummi, Talk, Polyvinylpyrrolidon, Polyethylenglykol und/oder Titandioxid enthalten, Lacklösungen in geeigneten organischen Lösungsmitteln oder Lösungsmittelgemischen oder, zur Herstellung von Magensaft-resistenten Ueberzügen, Lösungen von geeigneten Cellulosepräparaten, wie Acetylcellulosephthalat oder Hydroxypropylmethylcellulosephthalat, verwendet. Den Tabletten oder Dragée-Ueberzügen können Farbstoffe oder Pigmente, z.B. zur Identifizierung oder zur Kennzeichnung verschiedener Wirkstoffdosen, beigefügt werden.

Weitere, oral anwendbare pharmazeutische Zusammensetzungen sind Steckkapseln aus Gelatine, sowie weiche, geschlossene Kapseln aus Gelatine und einem Weichmacher, wie Glycerin oder Sorbit. Die Steckkapseln können den Wirkstoff in Form eines Granulats, z.B. im Gemisch mit Füllstoffen, wie Maisstärke, Bindemitteln und/oder Gleitmitteln, wie Talk oder Magnesiumstearat, und gegebenenfalls von Stabilisatoren, enthalten. In weichen Kapseln ist der Wirkstoff vorzugsweise in geeigneten Flüssigkeiten, wie fetten Oelen, Paraffinöl oder flüssigen Polyethylenglykolen, gelöst oder suspendiert, wobei ebenfalls Stabilisatoren zugefügt sein können.

Weitere orale Applikationsformen sind z.B. in üblicher Weise bereitete Sirups, die den Wirkstoff z.B. in suspendierter Form und in einer Konzentration von ca. 5 % bis 20 %, vorzugsweise ca. 10 % oder in einer ähnlichen Konzentration, die z.B. beim Abmessen von 5 oder 10 ml eine geeignete Einzeldosis ergibt, enthalten. Ferner kommen z.B. auch pulverförmige oder flüssige Konzentrate zur Bereitung von Shakes, z.B. in Milch, in Betracht. Solche Konzentrate können auch in Einzeldosismengen abgepackt sein.

Als rektal anwendbare pharmazeutische Präparate kommen z.B. Suppositorien in Betracht, welche aus einer Kombination des Wirkstoffs mit einer Suppositoriengrundmasse bestehen. Als Suppositoriengrundmasse eignen sich z.B. natürliche oder synthetische Triglyceride, Paraffinkohlenwasserstoffe, Polyäthylenglykole oder höhere Alkanole. Ferner können auch Gelatine-Rektalkapseln verwendet werden, die eine Kombination des Wirkstoffs mit einer Grundmasse enthalten; als Grundmassenstoffe kommen z.B. flüssige Triglyceride, Polyäthylenglykole oder Paraffinkohlenwasserstoffe in Frage.

Zur parenteralen Verabreichung eignen sich in erster Linie wässrige Lösungen eines Wirkstoffs in wasserlöslicher Form, ferner Suspensionen des Wirkstoffs, wie entsprechende ölige Injektionssuspensionen, wobei man geeignete lipophile Lösungsmittel oder Vehikel, wie fette Oele, z.B. Sesamöl, oder synthetische Fettsäureester, z.B. Aethyloleat oder Triglyceride, verwendet, oder wässrige Injektionssuspensionen, welche viskositätserhöhende Stoffe, z.B. Natrium-carboxymethylcellulose, Sorbit und/oder Dextran und gegebenenfalls auch Stabilisatoren, enthalten.

Die Erfindung betrifft ebenfalls Zusammensetzungen für diagnostische Zwecke, die einen geeigneten Metallkomplex einer Verbindung der Formel I, worin $A_1$, $A_2$ und $A_3$ für Wasserstoff stehen, vorzugsweise in Form einer wässrigen Lösung oder als Trockenpräparat enthalten.

Die Erfindung betrifft ebenfalls ein Verfahren zur Behandlung von Krankheiten, bei denen z.B., wie oben beschrieben, ein Ueberschuss von Eisen-(III) oder Aluminium im Körper vorhanden ist, indem man eine prophylaktisch oder therapeutisch wirksame Menge einer Verbindung der Formel I verabreicht. Dabei verwendet man in erster Linie die obengenannten pharmazeutischen Zusammensetzungen, wobei man einem Warmblüter von etwa 70 kg Gewicht eine tägliche Dosis von etwa 0,2 g bis etwa 10 g, vorzugsweise von etwa 0,5 g bis etwa 5 g einer erfindungsgemässen Verbindung verabreicht.

Die Erfindung betrifft ebenfalls die Verwendung von geeigneten Metallkomplexen von Verbindungen der Formel I, worin $A_1$, $A_2$ und $A_3$ für Wasserstoff stehen, für diagnostische Zwecke, insbesondere in der magnetischen Resonanz-diagnostik. Ueblicherweise verwendet man wässrige Lösungen von solchen Metallkomplexen, die vorzugsweise vor der Verabreichung hergestellt werden und deshalb keine weiteren Hilfsmittel enthalten. Die Lösungen mit Konzentrationen von bis zu etwa 25 %, üblicherweise von etwa 10 % bis etwa 25 % des Metallkomplexes, werden üblicherweise als Bolusinjektionen verwendet, wobei Dosen von etwa 0,01 bis etwa 1 mMol/kg, insbesondere von etwa 0,2 bis etwa 0,4 mMol/kg des Metallkomplexes

parenteral, insbesondere intravenös, verabreicht werden.

Die Erfindung wird anhand der nachfolgenden Beispiele illustriert; Temperaturen werden in Celsiusgraden angegeben.

Beispiel 1: N-[ω-Methoxy-(dodecakis-ethylenoxy)-carbonyl]-desferrioxamin B, worin Dodecakis-ethylenoxy einen Rest der Formel $-(CH_2-CH_2-O)_n-$ bedeutet, wobei n für einen Durchschnittswert von 12 steht.

Eine Suspension von 86,5 g (132 mMol) Desferrioxamin B-methansulfonat in 2'000 ml Pyridin versetzt man bei Raumtemperatur mit 194,0 ml (1'500 mMol) Trimethylchiorsilan; die entstandene Lösung wird anschliessend während 3 Stunden bei Raumtemperatur gerührt. Das Reaktionsgemisch wird innerhalb von 15 Minuten bei Raumtemperatur tropfenweise mit dem Acylierungsmittel versetzt, das durch Mischen einer Lösung von 72,6 g (132 mMol) eines Polyethylenglycolmonomethylethers mit einem durchschnittlichen Molekulargewicht von etwa 560 (Carbowax MPEG 550 der Firma Union Carbide, das im Durchschnitt 12 Einheiten der Formel $-CH_2-CH_2-O-$ aufweist) in 1'000 ml Toluol mit 66,0 ml (132 mMol) einer 20%igen Lösung von Phosgen in Toluol bei 70°C, Rühren während 3 Stunden bei dieser Temperatur und Abkühlen erhalten wird. Das Gemisch wird während 16 Stunden bei Raumtemperatur gerührt; dann werden durch Zugabe von 2'000 ml Methanol überschüssige Reagentien zerstört und Silylgruppen abgespalten, wonach die Lösungsmittel möglichst vollständig abdestilliert werden. Der Rückstand, noch stark Pyridin-haltig, wird aus etwa 500 ml Methylenchlorid und 1'000 ml Diethylether kristallisiert und während 16 Stunden unter Hochvakuum getrocknet. Durch Chromatographie an einem hydroxypropylierten Dextrangelmaterial in Kügelchenform (Sephadex® LH-20; Säulegrösse: 6'000 ml; das Rohprodukt wird in Methanol aufgetragen) wird aus dem rohen Kristallisat die Titelverbindung erhalten, wobei man diese nach Elution mit einer ersten Fraktion von 1'360 ml Methanol und weiteren Fraktionen zu je 100 ml Methanol aus den Fraktionen 6-10 erhält, Smp. 131-132° nach Kristallisation aus Essigsäureethylester und wenig Methylenchlorid.

Die Elementaranalyse des Produkts, das ein halbes Mol Wasser enthält, entspricht der Durchschnittssummenformel von $C_{51}H_{98}N_6O_{22}$ x 0,5 Mol $H_2O$:

| | | | | |
|---|---|---|---|---|
| Gefunden: | C 52.80 %; | H 8.63 %; | N 7.44 %; | O 31.23 %. |
| Berechnet: | C 52.97 %; | H 8.63 %; | N 7.27 %; | O 31.13 %. |

Das Produkt ist bis zu 25 % in Wasser, bis zu 40 % in Dimethylsulfoxid, bis zu 10 % in Methanol und bis zu 5 % in Methylenchlorid löslich.

Beispiel 2: N-[ω-Methoxy-(heptadecakis-ethylenoxy)-carbonyl]-desferrioxamin B, worin Heptadecakis-ethylenoxy einen Rest der Formel $-(CH_2-CH_2-O)_n-$ bedeutet, wobei n für einen Durchschnittswert von 17 steht.

Ein Gemisch von 86,5 g Desferrioxamin B-methansulfonat in 2'000 ml Pyridin wird bei Raumtemperatur mit 194,0 ml Trimethylchlorsilan versetzt, die Lösung während 3 Stunden gerührt und anschliessend mit einem Acylierungsreagens tropfenweise innerhalb von 15 Minuten behandelt. Dieses wird aus 99,0 g (132 mMol) eines Polyethylenglycolmonomethylethers mit einem durchschnittlichen Molekulargewicht von etwa 780 (Carbowax MPEG 750 der Firma Union Carbide, das im Durchschnitt 17 Einheiten der Formel $-CH_2-CH_2-O-$ enthält) in 1'000 ml Toluol durch Behandeln mit 66,0 ml (132 mMol) einer 20%igen Lösung von Phosgen in Toluol zubereitet, wobei die Lösung während 3 Stunden bei 70° gehalten wird. Das Reaktionsgemisch wird während 16 Stunden bei Raumtemperatur gerührt, dann mit 2'000 ml Methanol versetzt und eingedampft. Der noch Pyridin enthaltende Rückstand wird aus etwa 500 ml Methylenchlorid und 1'000 ml Diethylether kristallisiert und das Produkt während 16 Stunden unter Hochvakuum getrocknet. Das Rohprodukt wird mittels Chromatographie an einem hydroxypropylierten Dextrangelmaterial in Kügelchenform (Sephadex® LH-20) gereinigt, wobei man das Rohprodukt in Methanol aufträgt und mit Methanol eluiert, und die gewünschte Verbindung nach der ersten Fraktion von 1'590 ml und nachfolgenden 100 ml-Fraktionen mit den Fraktionen 14 bis 20 erhalten wird. Der Rückstand wird aus Essigsäureethylester und Diethylether kristalliert, Smp. 125-126°C.

Die Elementaranalyse des Produktes, das ein Mol Wasser enthält, entspricht der Durchschnittssummenformel von $C_{61}H_{118}N_6O_{27}$ x 1 Mol $H_2O$:

| | | | | |
|---|---|---|---|---|
| Gefunden: | C 53.17 %; | H 8.72 %; | N 5.80 %; | O 32.48 %. |
| Berechnet: | C 52.87 %; | H 8.72 %; | N 6.06 %; | O 32.33 %. |

Das Produkt ist bis zu 40 % in Wasser (viskos; bis zu 30 % eine klare Lösung), bis zu 45 % in Dimethylsulfoxid, bis zu 40 % in Methanol und bis zu 20 % in Methylenchlorid löslich.

Beispiel 3: N-[ω-Methoxy-(dodecakis-ethylenoxy)-carbonyl]-desferrioxamin B, worin Dodecakis-ethylenoxy einen Rest der Formel $-(CH_2-CH_2-O)_n-$ bedeutet, wobei n für einen Durchschnittswert von 12 steht.

In analoger Weise wie im Beispiel 1 werden 6,56 g (10 mMol) Desferrioxamin B-methansulfonat in 150 ml Pyridin mit 15,5 ml (120 mMol) Trimethylchlorsilan silyliert und mit einem folgendermassen zubereiteten Acylierungsreagens umgesetzt: Eine Lösung von 5,5 g (10 mMol) eines Polyethylenglycolmonomethylethers mit einem durchschnittlichen Molekulargewicht von etwa 560 (Carbowax MPEG 550 der Firma Union Carbide, das im Durchschnitt 12 Einheiten der Formel $-CH_2CH_2-O-$ aufweist) in 50 ml Toluol wird mit 1,78 g (11 Mmol) 1,1'-Carbonyl-bis-1H-imidazol versetzt, bei 70°C 1 Stunde gerührt und abgekühlt. Die Aufarbeitung gemäss Beispiel 1 ergibt die Titelverbindung, welche mit derjenigen des Beispiels 1 übereinstimmt.

Beispiel 4: N-[ω-Methoxy-(dodecakis-ethylenoxy)-carbonylaminosulfonyl]-desferrioxamin B, worin Dodecakis-ethylenoxy einen Rest der Formel $-(CH_2-CH_2-O)_n-$ bedeutet, wobei n für einen Durchschnittswert von 12 steht.

In analoger Weise und mit denselben Substanzmengen wie in Beispiel 3 wird Desferrioxamin B-methansulfonat silyliert und anschliessend mit einem folgendermassen zubereiteten Acylierungsreagens umgesetzt: Eine Lösung von 5,5 g (10 mMol) eines Polyethylenglycolmonomethylethers mit einem durchschnittlichen Molekulargewicht von etwa 560 (Carbowax MPEG 550 der Firma Union Carbide, das im Durchschnitt 12 Einheiten der Formel $-CH_2CH_2-O-$ aufweist) in 50 ml Toluol wird mit 0,95 ml (11 mMol) Chlorsulfonylisocyanat versetzt, bei 70°C während 1 Stunde gerührt und abgekühlt. Nach Zugabe von 2000 ml Methanol wird das Reaktionsgemisch zur Trockne eingeengt. Der Rückstand, stark Pyridin-haltig, wird aus etwa 500 ml Methylenchlorid und 1000 ml Diethylether kristallisiert und während 16 Stunden im Hochvakuum getrocknet. Eine weitere Reinigung kann durch Chromatographie an einem hydroxypropylierten Dextrangelmaterial in Kügelchenform (Sephadex® LH-20) erfolgen.

Beispiel 5: Trockenampullen mit einem Gehalt von 0,25 g Wirkstoff zum Zubereiten von 10%igen bzw. 5%igen Gewicht/Volumen (G/V) wässrigen Injektionslösungen in sterilisiertem Wasser werden hergestellt, indem man 2,5 ml einer 10%igen (G/V) Lösung des Wirkstoffes in Ampullen zu 2,5 bzw. 5 ml abfüllt und in üblicher Weise lyophilisiert.

In analoger Weise werden Trockenampullen, die 0,5 g Wirkstoff enthalten, hergestellt, indem 5,0 ml einer 10%igen (G/V) wässrigen Lösung oder 2,5 ml einer 20%igen (G/V) wässrigen Lösung des Wirkstoffs in Ampullen für 5 ml bzw. 2,5 ml abgefüllt und lyophilisiert werden.

Die für das Lyophilisieren verwendeten Lösungen können zusätzlich z.B. 8 % (G/V) Mannit, entsprechend 0,2 g bzw. 0,4 g pro Ampulle, enthalten.

Als "Wirkstoff" verwendet man eine der in den vorstehenden und nachfolgenden Beispielen beschriebenen N-acylierten Desferrioxaminverbindungen.

Beispiel 6: N-(ω-Methoxy-(undecakis-ethylenoxy)-carbonyl]-desferrioxamin B, worin Undecakis-ethylenoxy einen Rest der Formel $-(CH_2-CH_2-O)_n-$bedeutet, wobei n für einen Durchschnittswert von 11 steht.

Aus einem Gemisch von 446,0 g Desferrioxamin B-methansulfonat in 4'000 ml Tetrahydrofuran und 1'000 ml destilliertem Wasser entsteht durch Erwärmen auf 35°-40°C eine leicht trübe Lösung, die mit 72,0 g Natriumcarbonat versetzt und während 10 Minuten gerührt wird.

Ein Gemisch von 448,0 g eines Polyethylenglycolmonomethyläthers mit einem durchschnittlichen Molekulargewicht von etwa 516 (Carbowax MPEG 550, der Firma Union Carbide, wobei die verwendete Charge von derjenigen des Beispiels 1 verschieden ist, und das Polyethylenglycol im Durchschnitt 11 Einheiten der Formel $-CH_2-CH_2-O-$ enthält) in 2'000 ml Tetrahydrofuran wird mit 142,4 g 1,1'-Carbonyl-bis-1H-imidazol versetzt, mit 200 ml Tetrahydrofuran verdünnt und während 16 Stunden gerührt. Die so erhaltene klare Lösung wird innerhalb von 30 Minuten zum Desferrioxamingemisch getropft und nach 2

Stunden mit 1'000 ml Wasser verdünnt, wobei eine klare Lösung entsteht, in der nach 5 Stunden mittels Hochdruckflüssigchromatographie (HPLC) kein Desferrioxamin mehr festzustellen ist. Man rührt während 16 Stunden bei 0° weiter, dampft das organische Lösungsmittel ab und lyophilisiert den wässrigen Anteil.

Das Lyophilisat wird bei 40° in 3'000 ml Methanol aufgenommen, das Gemisch wird während 10 Minuten gerührt, filtriert und mit Methanol gespült. Innerhalb von 75 Minuten und bei 20° wird das klare Filtrat mit 6'000 ml Di-isopropylether versetzt, der entstandene Niederschlag abfiltriert und der Filterrückstand in 2'200 ml eines 2:1-Gemisches von Di-isopropylether und Methanol aufgenommen und während 45 Minuten gerührt. Die so erhältliche Suspension kann nicht mehr filtriert werden; sie wird eingedampft, bei 50°-60°C in 2'800 ml Methanol aufgenommen, heiss filtriert und mit 6'000 ml Di-isopropylether versetzt. Nach dem Abkühlen auf 10° wird filtriert und der Filterrückstand mit 1'000 ml eines 2:1-Gemisches von Di-isopropylether und Methanol gewaschen, getrocknet und gemahlen, dann mittels Chromatographie an einem Adsorptionsharz des Polystyroltyps (Amberlite® XAD-1180) entsalzen, wobei das Produkt in Wasser aufgetragen und mit 4:1- bis 1:1-Gemischen von Wasser und Isopropanol eluiert wird. Es fällt nach dem Lyophilisieren in Form eines weissen, amorphen Pulvers an, das ein halbes Mol Wasser enthält und dessen Elementaranalyse der Summenformel $C_{49}H_{94}N_6O_{21}$ x 0,5 Mol $H_2O$ entspricht:

| | | | |
|---|---|---|---|
| Gefunden:<br>Berechnet: | C 52.87 %;<br>C 52.91 %; | H 8.55 %;<br>H 8.61 %; | N 7.79 %.<br>N 7.56 %. |

Das Produkt ist bis zu 25 % in Wasser, bis zu 40 % in Dimethylsulfoxid und bis zu 10 % in Methanol löslich.

Beispiel 7: N-[ω-Methoxy-(undecakis-ethylenoxy)-carbonyl]-desferrioxamin B, worin Undecakis-ethylenoxy einen Rest der Formel $-(CH_2-CH_2-O)_n-$ bedeutet, worin n für einen Durchschnittswert von 11 steht.

Ein Gemisch von 500,0 g Desferrioxamin B-methansulfonat in 2'500 ml destilliertem Wasser, das nach 10-minütigem Rühren in eine klare Lösung übergeht, die auf 10° abgekühlt und innerhalb von 5 Minuten mit 80 g Natriumcarbonat in 500 ml Wasser versetzt wird. Durch Animpfen mit freiem Desferrioxamin B entsteht eine dickflüssige, milchige Suspension, die filtriert wird. Der Rückstand wird mit 500 ml Wasser und mit 500 ml eines 1:1-Gemisches von Wasser und Aceton, dann mit Portionen von total 2'000 ml Aceton gewaschen, während 16 Stunden bei 40° unter einem Druck von etwa 20 mm/Hg und während weiteren 16 Stunden bei 40°C unter Hochvakuum getrocknet.

Ein Gemisch von 381,0 g des so erhältlichen Desferrioxamins B in 4'000 ml Tetrahydrofuran und 2'000 ml destilliertem Wasser wird innerhalb von 30 Minuten zum Acylierungsmittel gegeben, das wie folgt hergestellt wird: Ein Gemisch von 448,0 g eines Polyethylenglycolmonomethylethers mit einem durchschnittlichen Molekulargewicht von etwa 516 (Carbowax MPEG 550, der Firma Union Carbide, wobei die verwendete Charge von derjenigen des Beispiels 1 verschieden ist und das Polyethylenglycol im Durchschnitt 11 Einheiten der Formel $-CH_2-CH_2-O-$ enthält) wird in 2000 ml Tetrahydrofuran und mit 142,4 g 1,1'-Carbonyl-bis-1H-imidazol versetzt und mit 200 ml Tetrahydrofuran versetzt, während 16 Stunden gerührt und als klare Lösung verwendet.

Das Reaktionsgemisch wird während 3 Stunden bei 40°-45°C gerührt, die klare Lösung abgekühlt und in 4 Portionen eingedampft. Jede Portion wird zweimal mit je 1'000 ml n-Butanol versetzt und zur Trockne eingedampft. Die beiden ersten Portionen werden vereinigt, auf ein Gewicht von 905 g eingeengt und mit 345 g n-Butanol verdünnt. Die Suspension wird auf 10°C abgekühlt und mit 18 ml Wasser versetzt, während 16 Stunden gerührt und filtriert. Der Filterrückstand wird mit 1'000 ml eines 1:1-Gemisches von Di-isopropylether und n-Butanol gewaschen, in 1'000 ml Butanol aufgenommen, während 3 Stunden bei 40°-45°C, dann während 16 Stunden bei Raumtemperatur gerührt und auf 10°C abgekühlt. Man verdünnt mit 5'000 ml Di-isopropylether, versetzt innerhalb von 2 Stunden mit 20 ml Wasser und rührt während 16 Stunden bei 10°C und filtriert. Der Filterrückstand wird mit 1'000 ml eines 1:1-Gemisches von Di-isopropylether und n-Butanol gewaschen und bei 40°C unter Hochvakuum getrocknet. Die beiden anderen Portionen werden in gleicher Weise aufgearbeitet. Das Produkt enthält 1 Mol Wasser und die Elementaranalyse entspricht der Summenformel $C_{49}H_{94}N_6O_{21}$ x 1 Mol $H_2O$:

| | | | |
|---|---|---|---|
| Gefunden:<br>Berechnet: | C 52.50 %;<br>C 52.49 %; | H 8.50 %;<br>H 8.63 %; | N 7.50 %.<br>N 7.49 %. |

Beispiel 8: N-[ω-Methoxy-(dodecakis-ethylenoxy)-carbonyl]-O,O',O''-tri-(n-octanoyl)-desferrioxamin B, worin Dodecakis-ethylenoxy einen Rest der Formel $-(CH_2-CH_2-O)_n-$ bedeutet, wobei n für einen Durchschnittswert von 12 steht.

Eine Suspension von 11,47 g (10 mMol) N-[ω-Methoxy-(undecakis-ethylenoxy)-carbonyl]-desferrioxamin B, worin Undecakis-ethylenoxy einen Rest der Formel $-(CH_2-CH_2-O)_n-$ bedeutet, worin n für einen Durchschnittswert von 12 steht (Beispiel 1), in 100 ml Acetonitril und 100 ml Methylenchlorid wird mit 5,6 ml (40 mMol) Triethylamin und dann tropfenweise bei Raumtemperatur mit 11,99 ml (70 mMol) Caprylsäurechlorid versetzt. Das leicht erwärmte Reaktionsgemisch wird während 16 Stunden bei Raumtemperatur gerührt, wobei sich nach 1 Stunde eine klare Lösung bildet. Die Lösungsmittel werden dann verdampft, der Rückstand zwischen Methylenchlorid und Wasser verteilt, und die organische Phase über Natriumsulfat getrocknet und unter vermindertem Druck eingedampft. Das gelbe, ölige Produkt wird mittels Chromatographie an Silikagel gereinigt, wobei es in amorpher, öliger und farbloser Form erhalten wird. Das stark hygroskopische Produkt enthält ein halbes Mol Wasser und seine Elementaranalyse entspricht der Bruttoformel $C_{75}H_{140}N_6O_{25}$:

| | | | | |
|---|---|---|---|---|
| Gefunden: | C 58.74 %; | H 9.34 %; | N 5.43 %; | O 26.60 %. |
| Berechnet: | C 58.69 %; | H 9.26 %; | N 5.47 %; | O 26.58 %. |

Im Dünnschichtchromatogramm zeigt das Produkt Rf-Werte von 0,40 (9:1-Gemisch von Methylenchlorid und Isopropanol) und 0,80 (4:1-Gemisch von Methylenchlorid und Methanol).

Beispiel 9: N-[ω-Methoxy-(dodecakis-ethylenoxy)-carbonyl]-O,O',O''-tri-(ethoxycarbonyl)-desferrioxamin B, worin Dodecakis-ethylenoxy einen Rest der Formel $-(CH_2-CH_2-O)_n-$ bedeutet, wobei n für einen Durchschnittswert von 12 steht.

Eine Supension von 11,47 g (10 mMol) N-[ω-Methoxy-(undecakis-ethylenoxy)-carbonyl]-desferrioxamin B, worin Undecakis-ethylenoxy einen Rest der Formel $-(CH_2-CH_2-O)_n-$ bedeutet, worin n für einen Durchschnittswert von 12 steht (Beispiel 1), in 100 ml Acetonitril und 100 ml Methylenchlorid wird mit 5,6 ml (40 mMol) Triethylamin und bei Raumtemperatur tropfenweise mit 6,70 ml (70 mMol) Chlorameisensäureethylester versetzt. Das leicht warme Gemisch wird während 16 Stunden bei Raumtemperatur gerührt, wobei nach einer Stunde vollständige Lösung eintritt. Die Lösungsmittel werden unter vermindertem Druck entfernt, der Rückstand zwischen Methylenchlorid und Wasser verteilt, und die organische Phase über Natriumsulfat getrocknet und eingedampft. Total 11,4 g des öligen, gelben Rückstandes werden an 750 ml Silikagel chromatographiert (Auftrag in Methylenchlorid, Elution mit einem 95:5-Gemisch von Methylenchlorid/Isopropanol) und ergibt das amorphe, ölige und farblose Produkt, das stark hygroskopisch ist und ein halbes Mol Wasser enthält; seine Elementaranalyse entspricht der Bruttoformel:
$C_{60}H_{110}N_6O_{28}$ x 0,5 Mol $H_2O$:

| | | | | |
|---|---|---|---|---|
| Gefunden: | C 52.39 %; | H 8.28 %; | N 6.45 %; | O 33.44 %. |
| Berechnet: | C 52.51 %; | H 8.15 %; | N 6.12 %; | O 33.22 %. |

Im Dünnschichtchromatogramm zeigt das Produkt Rf-Werte von 0,20 (in einem 9:1-Gemisch von Methylenchlorid und Isopropanol) und 0,60 (in einem 4:1-Gemisch von Methylenchlorid und Methanol).

In analoger Weise kann man das N-[ω-Methoxy-(dodecakis-ethylenoxy)-carbonyl]-O,O',O''-tri-[ω-methoxy-(bis- oder tris-ethylenoxy)-carbonyl]-desferrioxamin B, worin Dodecakis-ethylenoxy einen Rest der Formel $-(CH_2-CH_2-O)_n-$ bedeutet, wobei n für einen Durchschnittswert von 12 steht, erhalten.

Beispiel 10: N-[ω-Methoxy-(dodecakis-ethylenoxy)-carbonyl]-O,O',O''-tri-(ethoxycarbonylmethylaminocarbonyl)-desferrioxamin B, worin Dodecakis-ethylenoxy einen Rest der Formel $-(CH_2-CH_2-O)_n-$ bedeutet, wobei n für einen Durchschnittswert von 12 steht.

Eine Supension von 11,47 g (10 mMol) N-[ω-Methoxy-(undecakis-ethylenoxy)-carbonyl]-desferrioxamin B, worin Undecakis-ethylenoxy einen Rest der Formel $-(CH_2-CH_2-O)_n-$ bedeutet, worin n für einen Durchschnittswert von 12 steht (Beispiel 1), in 100 ml Acetonitril und 100 ml Methylenchlorid wird mit 1,4 ml (10 mMol) Triethylamin und bei Raumtemperatur tropfenweise mit 5,15 ml (70 mMol) Isocyanatoessigsäureethylester versetzt. Das leicht warme Reaktionsgemisch wird während 4 Stunden bei Raumtemperatur gerührt, wobei nach einer Stunde vollständige Lösung eintritt. Die Lösungsmittel werden unter vermindertem Druck entfernt, der Rückstand wird zwischen Wasser und Methylenchlorid verteilt, und die organische Phase über Natriumsulfat getrocknet und eingedampft. Total 13,4 g des gelben, öligen Rückstandes werden an 750 ml Silikagel chromatographiert, wobei das Rohprodukt in Methylenchlorid aufgetragen und das gewünschte Material mit einem 95:5-Gemisch von Methylenchlorid und Isopropanol eluiert wird. Man erhält so das amorphe, ölige und farblose Produkt, das stark hygroskopisch ist und ein Mol Wasser enthält. Die Elementaranalyse entspricht der Bruttoforme` : $C_{66}H_{119}N_9O_{31}$ x 1 Mol $H_2O$:

| | | | | |
|---|---|---|---|---|
| Gefunden: | C 50.96 %; | H 7.77 %; | N 8.63 %; | O 32.87 %. |
| Berechnet: | C 51.05 %; | H 7.85 %; | N 8.12 %; | O 32.97 %. |

Im Dünnschichtchromatogramm (Silikagel) zeigt das Produkt Rf-Werte von 0,10 (in einem 9:1-Gemisch von Methylenchlorid und Isopropanol) und 0,75 (in einem 4:1-Gemisch von Methylenchlorid und Methanol).

Beispiel 11: Eisen-III-Komplex von N-[ω-Methoxy-(undecakis-ethylenoxy)-carbonyl]-desferrioxamin B, worin Undecakis-ethylenoxy einen Rest der Formel $-(CH_2-CH_2-O)_n-$ bedeutet, wobei n für einen Durchschnittswert von 11 steht.

Ein Gemisch von 300 g (300 mMol) N-[ω-Methoxy-(undecakis-ethylenoxy)-carbonyl]-desferrioxamin B, worin Undecakis-ethylenoxy einen Rest der Formel $-(CH_2-CH_2-O)_n-$ bedeutet, wobei n für einen Durchschnittswert von 11 steht (Beispiel 6), in 3' 500 ml Wasser bildet nach 10 Minuten eine Lösung, die unter starkem Rühren mit 115 g (325 mMol) Eisen-III-acetylacetonat in 2'000 ml Essigsäurethylester versetzt wird; das Reaktionsgemisch wird während 2 Stunden bei Raumtemperatur stark gerührt, dann mit total 15'000 ml Essigsäureethylester gewaschen. Die wässrige Phase wird lyophilisiert und ergibt den gewünschten Eisen-III-Komplex in Form eines tiefroten harzigen Produktes, das zu 25 % Dimethylsulfoxid, 40 % in Wasser (klare Lösung bei 30 %), 40 % in Methanol und 30 % in Methylenchlorid löslich ist, und dessen Elementaranalyse der Bruttoformel von $C_{49}H_{91}FeN_6O_{21}$ entspricht:

| | | | | |
|---|---|---|---|---|
| Gefunden: | C 50.21 %; | H 7.99 %; | N 7.24 %; | Fe 4.90 %. |
| Berechnet: | C 50.12 %; | H 7.98 %; | N 7.16 %; | Fe 4.76 %. |

Beispiel 12: Eisen-III-Komplex von N-[ω-Methoxy-(heptadecakis-ethylenoxy)-carbonyl)-desferrioxamin B, worin Heptadecakis-ethylenoxy einen Rest der Formel $-(CH_2-CH_2-O)_n-$ bedeutet, wobei n für einen Durchschnittswert von 17 steht.

Eine Lösung von 27,3 g N-[ω-Methoxy-(heptadecakis-ethylenoxy)-carbonyl]-desferrioxamin B, worin Heptadecakis-ethylenoxy einen Rest der Formel $-(CH_2-CH_2-O)_n-$ bedeutet, wobei n für einen Durchschnittswert von 17 steht (Beispiel 2), in 400 ml Wasser mit einer Lösung von 8,5 g Eisen-(III)-acetylacetonat in 400 ml Essigsäureethylester versetzt und das Reaktionsgemisch während 2 Stunden gut gerührt. Man extrahiert mit total 10'000 ml Essigsäureethylester und lyophilisiert die wässrige Phase. Der gewünschte Eisen-(III)-Komplex wird als tiefrotes, harziges Produkt erhalten und an einem hydroxypropylierten Dextrangelmaterial in Kügelchenform (Sephadex® LH 20) chromatographiert, wobei das Rohprodukt in Methanol aufgetragen und die Reinsubstanz mit Methanol eluiert wird. Es ist bis zu 1 % in Dimethylsulfoxid, bis zu 30 % in Wasser, bis zu 40 % in Methanol und bis zu 20 % in Methylenchlorid löslich und seine Elementaranalyse entspricht der Bruttoformel $C_{61}H_{115}FeN_6O_{27}$:

| | | | | |
|---|---|---|---|---|
| Gefunden: | C 50.95 %; | H 8.23 %: | N 5.87 %; | Fe 3.97 %. |
| Berechnet: | C 50.93 %; | H 8.20 %; | N 5.84 %; | Fe 3.88 %. |

Beispiel 13: Gallium-(III)-Komplex von N-[ω-Methoxy-(undecakis-ethylenoxy)-carbonyl]-desferrioxamin B, worin Undecakis-ethylenoxy einen Rest der Formel $-(CH_2-CH_2-O)_n-$ bedeutet, wobei n für einen Durchschnittswert von 11 steht.

Eine Lösung von 5,51 g N-[ω-Methoxy-(undecakis-ethylenoxy)-carbonyl]-desferrioxamin B, worin Undecakis-ethylenoxy einen Rest der Formel $-(CH_2-CH_2-O)_n-$ bedeutet, wobei n für einen Durchschnittswert von 11 steht (Beispiel 6), in 50 ml Wasser wird unter starkem Rühren mit einer Lösung von 2,20 g (6 mMol) Gallium-(III)-acetylacetonat in 50 ml Essigsäureethylester versetzt, und das Reaktionsgemisch während einer Stunde bei Raumtemperatur kräftig gerührt. Man extrahiert einige Male mit viel Essigsäureethylester und lyophilisiert die wässrige Phase. Man erhält so in Form eines farblosen, amorphen Harzes den gewünschten Gallium-(III)-Komplex, der zu 30 % in Wasser und 20 % in Dimethylsulfoxid löslich ist. Die Elementaranalyse entspricht der Durchschnittsbruttoformel $C_{49}H_{91}GaN_6O_{21}$:

| | | | |
|---|---|---|---|
| Gefunden: | C 50.12 %; | H 7.80 %; | N 7.20 %. |
| Berechnet: | C 50.30 %; | H 7.84 %; | N 7.18 %. |

Beispiel 14: Indium-(III)-Komplexvon N-[ω-Methoxy-(undecakis-ethylenoxy)-carbonyl]-desferrioxamin B, worin Undecakis-ethylenoxy einen Rest der Formel $-(CH_2-CH_2-O)_n-$ bedeutet, wobei n für einen Durchschnittswert von 11 steht.

Eine Lösung von 5,51 g (5 mMol) N-[ω-Methoxy-(undecakis-ethylenoxy)-carbonyl]-desferrioxamin B, worin Undecakis-ethylenoxy einen Rest der Formel $-(CH_2-CH_2-O)_n-$ bedeutet, wobei n für einen Durchschnittswert von 11 steht (Beispiel 6), in 50 ml Wasser wird unter starkem Rühren mit einer Lösung von 2,47 g (6 mMol) Indium-(III)-acetylacetonat in 50 ml Essigsäureethylester versetzt, und das Reaktionsgemisch während einer Stunde weitergerührt. Die wässrige Phase wird mit einer grossen Menge Essigsäureethylester gewaschen und dann lyophilisiert. Der gewünschte Indium-Komplex fällt als weisses amorphes Harz an, das in Wasser bis zu 30 % und in Dimethylsulfoxid bis zu 20 % löslich ist. Die Elementaranalyse entspricht der Durchschnittsbruttoformel $C_{49}H_{91}InN_6O_{21}$:

| | | | |
|---|---|---|---|
| Gefunden: | C 47.50 %; | H 7.50 %; | N 6.80 %. |
| Berechnet: | C 47.73 %; | H 7.60 %; | N 6.82 %. |

Beispiel 15: Mangan-III-Komplex von N-[ω-Methoxy-(undecakis-ethylenoxy)-carbonyl]-desferrioxamin B, worin Undecakis-ethylenoxy einen Rest der Formel $-(CH_2-CH_2-O)_n-$ bedeutet, wobei n für einen Durchschnittswert von 11 steht.

Ein Gemisch von 88,26 g (80 mMol) N-[ω-Methoxy-(undecakis-ethylenoxy)-carbonyl]-desferrioxamin B, worin Undecakis-ethylenoxy einen Rest der Formel $-(CH_2-CH_2-O)_n-$ bedeutet, wobei n für einen Durchschnittswert von 11 steht (Beispiel 6), und 700 ml Wasser wird unter kräftigem Rühren mit einer Lösung von 31,7 g (90 mMol) Mangan-(III)-acetylacetonat in 700 ml Essigsäureethylester versetzt und während einer Stunde bei Raumtemperatur gerührt. Die wässrige Phase wird mit viel Essigsäureethylester gewaschen und dann lyophilisiert. Man erhält den gewünschten Mangankomplex in Form eines tiefgrünen, harzigen Produktes, das bis zu 40 % in Wasser, bis zu 25 % in Dimethylsulfoxid, bis zu 40 % in Methanol und bis zu 30 % in Methylenchlorid löslich ist. Die Elementaranalyse entspricht der Durchschnittsbruttoformel $C_{49}H_{91}MnN_6O_{21}$:

| | | | | |
|---|---|---|---|---|
| Gefunden: | C 50.60 %; | H 7.90 %; | N 7.22 %; | Mn 5.05 %. |
| Berechnet: | C 50.55 %; | H 7.96 %; | N 7.22 %; | Mn 4.72 %. |

Beispiel 16: Eisen-III-Komplex von N-[ω-Methoxy-(undecakis-ethylenoxy)-carbonyl]-desferrioxamin B, worin Undecakis-ethylenoxy einen Rest der Formel $-(CH_2-CH_2-O)_n-$ bedeutet, wobei n für einen Durchschnittswert von 11 steht.

Eine Lösung von 11,03 g (10 mMol) N-[ω-Methoxy-(undecakis-ethylenoxy)-carbonyl]-desferrioxamin B, worin Undecakis-ethylenoxy einen Rest der Formel $-(CH_2-CH_2-O)_n-$ bedeutet, wobei n für einen Durchschnittswert von 11 steht (Beispiel 6), in 500 ml deionisiertem Wasser, die sich innerhalb von 10 Minuten

bildet, wird mit total 27,03 g (100 mMol) Eisen-III-chlorid versetzt, wobei man dieses in 10 Portionen zu je 2,70 g in 15-minütigen Abständen zugibt. Nach etwa 2-1/2 Stunden ist mittels Hochdruckflüssigchromatographie kein Ausgangsmaterial feststellbar. Das Reaktionsprodukt wird durch Zugabe von wässrigem Natriumhydroxid auf pH 7.5 gestellt, dann lyophilisiert. Das Lyophilisat wird in Methanol gelöst und mit Hilfe eines hydroxypropylierten Dextrangelmaterials in Kügelchenform (Sephadex® LH-20) gereinigt. Die mit Methanol ausgewaschenen dunkelroten Fraktionen enthaltenden gewünschten Eisen-III-komplex, dessen Elementaranalyse der Durchschnittsbruttoformel $C_{49}H_{91}FeN_6O_2$ x 1 Mol $H_2O$ entspricht:

| | | | | |
|---|---|---|---|---|
| Gefunden: | C 50.21 %; | H 7.99 %; | N 7.24 %; | Fe 4.90 %. |
| Berechnet: | C 50.12 %; | H 7.98 %; | N 7.16 %; | Fe 4.76 %. |

Das Produkt ist bis zu 25 % in Dimethylsulfoxid, bis zu 40 % in Wasser (viskose Lösung; klare Lösung bis zu 30 %), bis zu 40 % in Methanol und bis zu 30 % in Methylenchlorid löslich.

## Patentansprüche

## Patentansprüche für folgende Vertragsstaaten : AT, BE, CH, LI, DE, FR, GB, IT, LU, NL, SE

**1.** Verbindungen der Formel

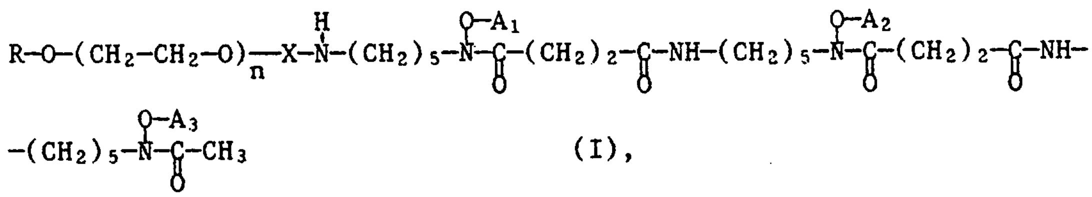

(I),

worin R für Alkyl mit bis zu 4 Kohlenstoffatomen steht, n einen Durchschnittswert von mindestens 9 darstellt, X einen Rest der Formel $-C(=O)-(NH-SO_2)_m-$ bedeutet, worin m für 0 oder 1 steht und, falls m für 1 steht, die Carbonylgruppe mit dem Sauerstoffatom oder dem Stickstoffatom verbunden sein kann, und jeder der Reste $A_1$, $A_2$ und $A_3$, unabhängig von den anderen, Wasserstoff oder einen Acylrest darstellt, und Salze von salzbildenden Verbindungen der Formel I, sowie Metallkomplexe von Verbindungen der Formel I, worin $A_1$, $A_2$ und $A_3$ für Wasserstoff stehen.

**2.** Verbindungen der Formel I gemäss Anspruch 1, worin R, X und m die im Anspruch 1 gegebenen Bedeutungen haben, und worin der Rest der Formel $-(CH_2-CH_2-O)_n-$ die Gruppe der Formel

$$-(CH_2-CH_2-O)_{n-1}-CH_2-CH_2-O-$$

bedeutet, wobei das Molekulargewicht des Restes $-(CH_2-CH_2-O)_{n-1}-$ zwischen etwa 500 und etwa 5000 liegt, und jeder der Reste $A_1$, $A_2$ und $A_3$, unabhängig von den anderen, für Wasserstoff oder einen von einer Carbonsäure abgeleiteten Acylrest steht, oder Salze solcher Verbindungen mit salzbildenden Eigenschaften.

**3.** Verbindungen der Formel I gemäss Anspruch 1, worin R und X die im Anspruch 1 gegebenen Bedeutungen haben, und $A_1$, $A_2$ und $A_3$, sowie der Rest der Formel $-(CH_2-CH_2-O)_n-$ die im Anspruch 2 gegebenen Bedeutungen haben, und worin m für 0 steht, sowie Salze von Verbindungen mit salzbildenden Eigenschaften.

**4.** Verbindungen der Formel I gemäss Anspruch 1, worin R, X und m die im Anspruch 1 und der Rest der Formel $-(CH_2-CH_2-O)_n-$ die im Anspruch 2 gegebenen Bedeutungen haben, und worin $A_1$, $A_2$ und $A_3$ für Wasserstoff stehen.

**5.** Verbindungen der Formel I gemäss Anspruch 1, worin R, X und m die im Anspruch 1 gegebenen Bedeutungen haben, n einen Durchschnittswert zwischen etwa 9 und etwa 115 darstellt, und jeder der Reste $A_1$, $A_2$ und $A_3$, unabhängig von den anderen, Wasserstoff oder den Rest der Formel Z-C(=O)-

(Ib) darstellt, worin Z für Wasserstoff steht oder einen Hydrocarbylrest $R^o$ darstellt, der zusammen mit der Carbonylgruppe den Acylrest einer gegebenenfalls substituierten acyclischen, carbocyclischen, carbocyclisch-acyclischen, heterocyclischen oder heterocyclischacyclischen Carbonsäure bildet, oder einen Hydrocarbyloxyrest der Formel $R^o$-O- darstellt, der zusammen mit der Carbonylgruppe den Acylrest einer monoveresterten Kohlensäure bildet, oder einen Hydrocarbylaminorest der Formel $R^o$-N-($R^1$)- bedeutet, worin $R^1$ für Wasserstoff steht oder die Bedeutung von $R^o$ hat, und der zusammen mit der Carbonylgruppe den Acylrest einer mono- oder di-substituierten Carbaminsäure bildet, oder Salze von solchen Verbindungen mit salzbildenden Eigenschaften, oder Komplexe von solchen Verbindungen, worin $A_1$, $A_2$ und $A_3$ für Wasserstoff stehen, mit dreiwertigen paramagnetischen Uebergangsmetallen, einschliesslich entsprechenden Lanthaniden, Metallen der dritten Hauptgruppe des Periodensystems und Radionucliden.

**6.** Verbindungen der Formel I gemäss Anspruch 1, worin R, X und m die im Anspruch 1 gegebenen Bedeutungen haben, n einen Durchschnittswert von etwa 10 bis etwa 17 darstellt, und jeder der Reste $A_1$, $A_2$ und $A_3$, unabhängig von den anderen, Wasserstoff, Alk(en)oyl mit bis und mit 20 Kohlenstoffatomen, Alkyloxycarbonyl mit bis und mit 20 Kohlenstoffatomen im Alkylteil, worin bis und mit 5 Methylengruppen durch Sauerstoffatome ersetzt sein können, wobei jeweils zwei Kohlenstoffatome die Sauerstoffatome voneinander trennen, oder Alkylaminocarbonyl mit bis und mit 20, vorzugsweise mit bis und mit 7 Kohlenstoffatomen, im Alkylteil, der gegebenenfalls durch Carboxy, Niederalkyloxycarbonyl mit bis und mit 4 Kohlenstoffatomen im Niederalkylteil, Carbamoyl und/oder durch Amino, Hydroxy, Mercapto, Niederalkylthio mit bis und mit 4 Kohlenstoffatomen, Phenyl oder Hydroxyphenyl substituiert sein kann, oder Salze von solchen Verbindungen mit salzbildenden Eigenschaften, und Komplexe von solchen Verbindungen, worin $A_1$, $A_2$ und $A_3$ für Wasserstoff stehen, mit dreiwertigen paramagnetischen Uebergangsmetallen, inklusive entsprechenden Lanthaniden, und geeigneten Metallen der dritten Hauptgruppe des Periodensystems, sowie mit geeigneten Radionucliden.

**7.** Verbindungen der Formel I gemäss Anspruch 1, worin R und X die im Anspruch 1 gegebenen Bedeutungen haben, m für 0 steht, n einen Durchschnittswert von etwa 10 bis etwa 17 darstellt, und jeder der Reste $A_1$, $A_2$ und $A_3$, unabhängig von den anderen, für Wasserstoff, Alkanoyl mit bis und mit 12 Kohlenstoffatomen, Alkyloxycarbonyl, worin Alkyl bis und mit 7 Kohlenstoffatome enthält, wobei eine oder zwei Methylengruppen durch Sauerstoff ersetzt sein können, und Sauerstoffatome jeweils durch zwei Kohlenstoffatome voneinander getrennt sind, oder Alkylaminocarbonyl steht, worin Alkyl bis und mit 7 Kohlenstoffatome enthält, das als Substituenten in 1- oder in 2-Stellung gegebenenfalls Alkyloxycarbonyl aufweist, worin Alkyl bis und mit 4 Kohlenstoffatome enthält, und Komplexe von solchen Verbindungen, worin $A_1$, $A_2$ und $A_3$ für Wasserstoff stehen, mit dreiwertigen paramagnetischen Uebergangsmetallen, einschliesslich entsprechenden Lanthaniden, und mit Metallen der dritten Hauptgruppe des Periodensystems, sowie mit geeigneten Radionucliden.

**8.** Verbindungen der Formel I gemäss Anspruch 1, worin R für Methyl steht, X die im Anspruch 1 gegebene Bedeutung hat und m für 0 steht, n einen Durchschnittswert von etwa 10 bis etwa 17 darstellt, und jeder der Reste $A_1$, $A_2$ und $A_3$ für Wasserstoff steht, und Komplexe von solchen Verbindungen mit dreiwertigen paramagnetischen Uebergangsmetallen, einschliesslich Lanthaniden, oder mit Metallen der dritten Hauptgruppe des Periodensystems, sowie mit geeigneten Radionucliden.

**9.** N-[ω-Methoxy-(dodecakis-ethylenoxy)-carbonyl]-desferrioxamin B, worin Dodecakis-ethylenoxy einen Rest der Formel $-(CH_2-CH_2-O)_n$-bedeutet, wobei n für einen Durchschnittswert von 12 steht, gemäss Anspruch 1.

**10.** N-[ω-Methoxy-(heptadecakis-ethylenoxy)-carbonyl]-desferrioxamin B, worin Heptadecakis-ethylenoxy einen Rest der Formel $-(CH_2-CH_2-O)_n-$ bedeutet, wobei n für einen Durchschnittswert von 17 steht, gemäss Anspruch 1.

**11.** N-[ω-Methoxy-(undecakis-ethylenoxy)-carbonyl]-desferrioxamin B, worin Undecakis-ethylenoxy einen Rest der Formel $-(CH_2-CH_2-O)_n$-bedeutet, wobei n für einen Durchschnittswert von 11 steht, gemäss Anspruch 1.

**12.** N-[ω-Methoxy-(dodecakis-ethylenoxy)-carbonyl]-O,O',O''-tri-(n-octanoyl)-desferrioxamin B, worin Dodecakis-ethylenoxy einen Rest der Formel $-(CH_2-CH_2-O)_n-$ bedeutet, wobei n für einen Durchschnittswert

von 12 steht, gemäss Anspruch 1.

**13.** N-[ω-Methoxy-(dodecakis-ethylenoxy)-carbonyl]-O,O',O''-tri-(ethoxycarbonyl)-desferrioxamin B, worin Dodecakis-ethylenoxy einen Rest der Formel $-(CH_2-CH_2-O)_n-$ bedeutet, wobei n für einen Durchschnittswert von 12 steht, gemäss Anspruch 1.

**14.** N-[ω-Methoxy-(dodecakis-ethylenoxy)-carbonyl]-O,O',O''-tri-(ethoxycarbonylmethylaminocarbonyl)-desferrioxamin B, worin Dodecakis-ethylenoxy einen Rest der Formel $-(CH_2-CH_2-O)_n-$ bedeutet, wobei n für einen Durchschnittswert von 12 steht, gemäss Anspruch 1.

**15.** Eisen-III-Komplex von N-[ω-Methoxy-(undecakis-ethylenoxy)-carbonyl]-desferrioxamin B, worin Undecakis-ethylenoxy einen Rest der Formel $-(CH_2-CH_2-O)_n-$ bedeutet, wobei n für einen Durchschnittswert von 11 steht, gemäss Anspruch 1.

**16.** Mangan-III-Komplex von N-[ω-Methoxy-(undecakis-ethylenoxy)-carbonyl]-desferrioxamin B, worin Undecakis-ethylenoxy einen Rest der Formel $-(CH_2-CH_2-O)_n-$ bedeutet, wobei n für einen Durchschnittswert von 11 steht, gemäss Anspruch 1.

**17.** Eine Verbindung gemäss einem der Ansprüche 2-4, 9 und 10 zur Anwendung in einem Verfahren zur therapeutischen bzw. diagnostischen Behandlung des menschlichen und tierischen Körpers.

**18.** Eine Verbindung gemäss einem der Ansprüche 5-8 und 11-16 zur Anwendung in einem Verfahren zur therapeutischen bzw. diagnostischen Behandlung des menschlichen und tierischen Körpers.

**19.** Pharmazeutische Präparate enthaltend eine Verbindung gemäss einem der Ansprüche 2-4, 9 und 10.

**20.** Pharmazeutische Präparate enthaltend eine Verbindung gemäss einem der Ansprüche 5-8 und 11-14.

**21.** Diagnostische Präparate enthaltend eine Verbindung gemäss einem der Ansprüche 1, 5-8, 15 und 16.

**22.** Verwendung von Verbindungen der Ansprüche 1, 5-8 und 11-16 zur Herstellung von pharmazeutischen oder diagnostischen Präparaten.

**23.** Verwendung von Verbindungen der Ansprüche 2-4, 9 und 10 zur Herstellung von pharmazeutischen Präparaten.

**24.** Verfahren zur Herstellung von Verbindungen der Formel I gemäss Anspruch 1, dadurch gekennzeichnet, dass man eine Verbindung der Formel

$R—O—(CH_2-CH_2-O)_n-Y_1$ (III)

mit einer Verbindung der Formel

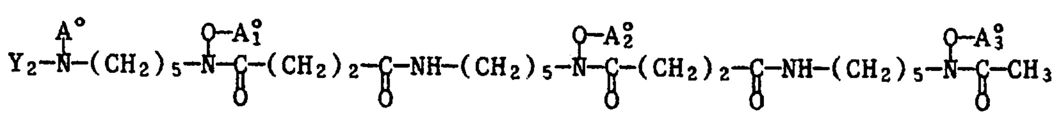

(IV)

oder ein Salz davon umsetzt, wobei (a) $Y_2$ für Wasserstoff steht, und $Y_1$ eine Gruppe der Formel $-X-Z_1$ (IIIa) bedeutet, in welcher $Z_1$ eine, zusammen mit dem Wasserstoff $Y_2$ unter Bildung der Bindung zwischen den Reaktionsteilnehmern abspaltbare Gruppe Z darstellt, oder (b) $Y_1$ für Wasserstoff steht, und $Y_2$ eine Gruppe der Formel $Z_2$-X- (IVa) bedeutet, worin $Z_2$ eine Gruppe Z darstellt, oder, falls m in einem Rest X für 0 steht, zusammen mit $A^\circ$ eine Bindung bildet, und worin $A^\circ$ Wasserstoff oder, falls $Y_2$ Wasserstoff bedeutet, eine Aminoschutzgruppe darstellt, und jeder der Reste $A_1^\circ$, $A_2^\circ$ und $A_3^\circ$, unabhängig voneinander, je für Wasserstoff, eine geeignete Schutzgruppe oder einen Acylrest steht, wobei in Acylresten $A_1^\circ$, $A_2^\circ$ und $A_3^\circ$ funktionelle Gruppen gegebenenfalls in geschützter Form vorliegen, und, wenn erwünscht oder notwendig, in einer erfindungsgemäss erhältlichen Verbindung vorhandene

Schutzgruppen abspaltet, und, wenn erwünscht, in einer erfindungsgemäss erhältlichen Verbindung der Formel I, in welcher mindestens eine der Gruppen $A_1$, $A_2$ und $A_3$ Wasserstoff bedeutet, diesen durch einen Acylrest ersetzt, und/oder, wenn- erwünscht, eine erfindungsgemäss erhältliche Verbindung der Formel I, worin $A_1$, $A_2$ und $A_3$ für Wasserstoff stehen, in einen Metallkomplex umwandelt, und/oder, wenn erwünscht, ein erfindungsgemäss erhältliches Salz einer salzbildenden Verbindung der Formel I in die freie Verbindung bzw. eine erfindungsgemäss erhältliche Verbindung mit salzbildenden Eigenschaften in ein Salz überführt.

**25.** Verfahren nach Anspruch 24 zur Herstellung von Verbindungen gemäss Anspruch 2, dadurch gekennzeichnet, dass Ausgangsstoffe der Formel III, worin n die im Anspruch 1 und der Rest der Formel $-(CH_2-CH_2-O)_n-$ die im Anspruch 2 gegebenen Bedeutungen haben, mit Ausgangsstoffen der Formel IV, worin jeder der Reste $A_1^{\circ}$, $A_2^{\circ}$ und $A_3^{\circ}$, unabhängig vom anderen, Wasserstoff, Acylreste von Carbonsäuren oder organische Silylgruppen darstellt, wobei in einem Acylrest eine gegebenenfalls vorhandene zusätzliche Aninogruppe in geschützter Form vorliegt, und $A^{\circ}$ Wasserstoff oder, falls $Y_2$ für Wasserstoff steht, eine organische Silylgruppe darstellt, umgesetzt werden, wobei (a) $Y_2$ für Wasserstoff und $Y_1$ für den Rest der Formel IIIa oder (b) $Y_1$ für Wasserstoff und $Y_2$ für den Rest der Formel IVa stehen, in welchen X die im Anspruch 1 gegebene Bedeutung hat und $Z_1$ und $Z_2$ Halogen mit einer Atomzahl von mindestens 19 oder 1-Imidazolyl darstellen, und gegebenenfalls vorhandene N- und/oder O-Silylgruppen und/oder Aninoschutzgruppen unter Freisetzung der entsprechenden Hydroxyl- bzw. Aninogruppen abspaltet, und, wenn erwünscht, eine erhaltene Verbindung der Formel I, worin mindestens eine der Gruppen $A_1$, $A_2$ und $A_3$ Wasserstoff bedeutet, diesen mittels Acylieren durch den Acylrest einer Carbonsäure ersetzt wird, und/oder, wenn erwünscht, eine erhaltene freie Verbindung der Formel I mit salzbildenden Eigenschaften in ein Salz umgewandelt wird und/oder eine Verbindung der Formel I aus einem solchen Salz freigesetzt wird.

**26.** Die nach dem Verfahren gemäss Anspruch 24 erhältlichen Verbindungen.

**27.** Die nach dem Verfahren gemäss Anspruch 25 erhältlichen Verbindungen.

**Patentansprüche für folgende Vertragsstaaten : ES, GR**

**1.** Verfahren zur Herstellung von

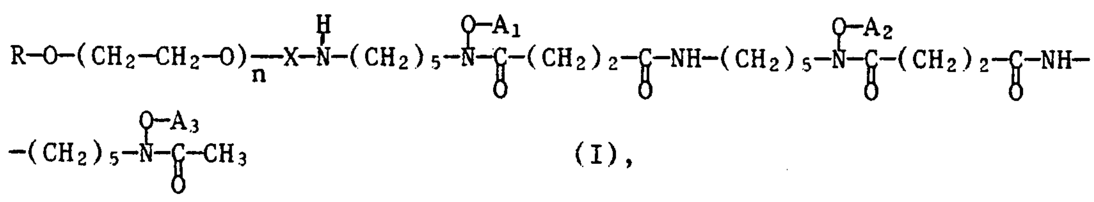

(I),

worin R für Alkyl mit bis zu 4 Kohlenstoffatomen steht, n einen Durchschnittswert von mindestens 9 darstellt, X einen Rest der Formel $-C(=O)-(NH-SO_2)_m-$ bedeutet, worin m für 0 oder 1 steht und, falls m für 1 steht, die Carbonylgruppe mit dem Sauerstoffatom oder dem Stickstoffatom verbunden sein kann, und-jeder der Reste $A_1$, $A_2$ und $A_3$, unabhängig von den anderen, Wasserstoff oder einen Acylrest darstellt, und Salzen von salzbildenden Verbindungen der Formel I, sowie Metallkomplexen von Verbindungen der Formel I, worin $A_1$, $A_2$ und $A_3$ für Wasserstoff stehen, dadurch gekennzeichnet, dass man eine Verbindung der Formel

$R—O—(CH_2-CH_2-O)_n-Y_1$ (III)

mit einer Verbindung der Formel

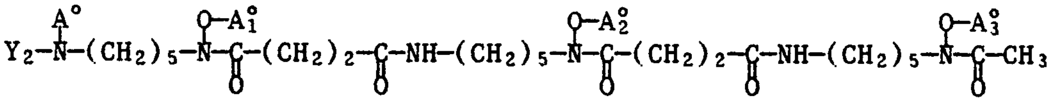

(IV)

oder ein Salz davon umsetzt, wobei (a) $Y_2$ für Wasserstoff steht, und $Y_1$ eine Gruppe der Formel $-X-Z_1$ (IIIa) bedeutet, in welcher $Z_1$ eine, zusammen mit dem Wasserstoff $Y_2$ unter Bildung der Bindung zwischen den Reaktionsteilnehmern abspaltbare Gruppe Z darstellt, oder (b) $Y_1$ für Wasserstoff steht, und $Y_2$ eine Gruppe der Formel $Z_2-X-$ (IVa) bedeutet, worin $Z_2$ eine Gruppe Z darstellt, oder, falls m in einem Rest X für 0 steht, zusammen mit A° eine Bindung bildet, und worin A° Wasserstoff oder, falls $Y_2$ Wasserstoff bedeutet, eine Aminoschutzgruppe darstellt, und jeder der Reste $A_1^\circ$, $A_2^\circ$ und $A_3^\circ$, unabhängig voneinander, je für Wasserstoff, eine geeignete Schutzgruppe oder einen Acylrest steht, wobei in Acylresten $A_1^\circ$, $A_2^\circ$ und $A_3^\circ$ funktionelle Gruppen gegebenenfalls in geschützter Form vorliegen, und, wenn erwünscht oder notwendig, in einer erfindungsgemäss erhältlichen Verbindung vorhandene Schutzgruppen abspaltet, und, wenn erwünscht, in einer erfindungsgemäss erhältlichen Verbindung der Formel I, in welcher mindestens eine der Gruppen $A_1$, $A_2$ und $A_3$ Wasserstoff bedeutet, diesen durch einen Acylrest ersetzt, und/oder, wenn erwünscht, eine erfindungsgemäss erhältliche Verbindung der Formel I, worin $A_1$, $A_2$ und $A_3$ für Wasserstoff stehen, in einen Metallkomplex umwandelt, und/oder, wenn erwünscht, ein erfindungsgemäss erhältliches Salz einer salzbildenden Verbindung der Formel I in die freie Verbindung bzw. eine erfindungsgemäss erhältliche Verbindung mit salzbildenden Eigenschaften in ein Salz überführt.

2. Verfahren nach Anspruch 1 zur Herstellung von Verbindungen der Formel I gemäss Anspruch 1, worin R, X und m die im Anspruch 1 gegebenen Bedeutungen haben, und worin der Rest der Formel $-(CH_2-CH_2-O)_n-$ die Gruppe der Formel

$$-(CH_2-CH_2-O)_{n-1}-CH_2-CH_2-O-$$

bedeutet, wobei das Molekulargewicht des Restes $-(CH_2-CH_2-O)_{n-1}-$ zwischen etwa 500 und etwa 5000 liegt, und jeder der Reste $A_1$, $A_2$ und $A_3$, unabhängig von den anderen, für Wasserstoff oder einen von einer Carbonsäure abgeleiteten Acylrest steht, oder Salzen von solcher Verbindungen mit salzbildenden Eigenschaften, dadurch gekennzeichnet, dass Ausgaßgsstoffe der Formel III, worin n die im Anspruch 1 und der Rest der Formel $-(CH_2-CH_2-O)_n-$ die im Anspruch 2 gegebenen Bedeutungen haben, mit Ausgangsstoffen der Formel IV, worin jeder der Reste $A_1^\circ$, $A_2^\circ$ und $A_3^\circ$, unabhängig vom anderen, Wasserstoff, Acylreste von Carbonsäuren oder organische Silylgruppen darstellt, wobei in einem Acylrest eine gegebenenfalls vorhandene zusätzliche Aminogruppe in geschützter Form vorliegt, und A° Wasserstoff oder, falls $Y_2$ für Wasserstoff steht, eine organische Silylgruppe darstellt, umgesetzt werden, wobei (a) $Y_2$ für Wasserstoff und $Y_1$ für den Rest der Formel IIIa oder (b) $Y_1$ für Wasserstoff und $Y_2$ für den Rest der Formel IVa stehen, in welchen X die im Anspruch 1 gegebene Bedeutung hat und $Z_1$ und $Z_2$ Halogen mit einer Atomzahl von mindestens 19 oder 1-Imidazolyl darstellen, und gegebenenfalls vorhandene N- und/oder O-Silylgruppen und/oder Aminoschutzgruppen unter Freisetzung der entsprechenden Hydroxyl- bzw. Aminogruppen abspaltet, und, wenn erwünscht, eine erhaltene Verbindung der Formel I, worin mindestens eine der Gruppensymbole $A_1$, $A_2$ und $A_3$ Wasserstoff bedeutet, diesen mittels Acylieren durch den Acylrest einer Carbonsäure ersetzt wird, und/oder, wenn erwünscht, eine erhaltene freie Verbindung der Formel I mit salzbildenden Eigenschaften in ein Salz umgewandelt wird und/oder eine Verbindung der Formel I aus einem solchen Salz freigesetzt wird.

3. Verfahren nach Anspruch 1, dadurch gekennzeichnet, dass Z für reaktionsfähiges verestertes Hydroxy, wie Halogen, mit einer Atomzahl von mindestens 19, z.B. Chlor, oder Azido, oder für eine geeignete, über ein Ringstickstoffatom gebundene monocyclische azacyclische Gruppe, z.B. 1-Imidazolyl steht.

4. Verfahren nach einem der Ansprüche 1 und 3, dadurch gekennzeichnet, dass Schutzgruppen $A_1^\circ$, $A_2^\circ$ und/oder $A_3^\circ$, sowie A° organische Silylgruppen, wie Trimethylsilyl, darstellen.

5. Verfahren nach einem der Ansprüche 1, 3 und 4, dadurch gekennzeichnet, dass man in einer erfindungsgemäss erhältlichen Verbindung der Formel I, worin mindestens ein Rest $A_1$, $A_2$ und/oder $A_3$ Wasserstoff bedeutet, diesen durch Behandeln mit einem, einen Acylrest einführenden Mittel, wie einem Anhydrid oder einem aktivierten Ester einer entsprechenden Säure, oder einer Säure in Gegenwart eines geeigneten Kondensationsmittels, durch einen Acylrest ersetzt.

6. Verfahren nach einem der Ansprüche 1, 3 und 4, dadurch gekennzeichnet, dass man eine erfindungsgemäss erhältliche Verbindung der Formel I, worin $A_1$, $A_2$ und $A_3$ für Wasserstoff stehen, durch Behandeln mit einem anorganischen oder organischen Salz oder Derivat eines Metalls, wie einem entsprechenden Komplex mit einer organischen Verbindung, deren Bindungsaffinität zu den Metallionen niedriger ist, als diejenige der Verbindungen der Formel I, worin $A_1$, $A_2$ und $A_3$ für Wasserstoff stehen, wie einer geeigneten $\beta$-Dicarbonylverbindung, z.B. Acetylaceton, in einen Metallkomplex umwandelt.

7. Verfahren nach Anspruch 6, dadurch gekennzeichnet, dass man ein Salz oder ein Derivat, z.B. einen Komplex von Eisen-III, Mangan-III, Chrom-III oder Gadolinium-III, z.B. das entsprechende Acetylacetonat, verwendet.

8. Verfahren nach einem der Ansprüche 1 und 3-7, dadurch gekennzeichnet, dass man Verbindungen der Formel I gemäss Anspruch 1, worin R, X und m die im Anspruch 1 gegebenen Bedeutungen haben, n einen Durchschnittswert zwischen etwa 9 und etwa 115 darstellt, und jeder der Reste $A_1$, $A_2$ und $A_3$, unabhängig von den anderen, Wasserstoff oder den Rest der Formel Z-C(=O)- (Ib) darstellt, worin Z für Wasserstoff steht oder einen Hydrocarbylrest $R^o$ darstellt, der zusammen mit der Carbonylgruppe den Acylrest einer gegebenenfalls substituierten acyclischen, carbocyclischen, carbocyclisch-acyclischen, heterocyclischen oder heterocyclischacyclischen Carbonsäure bildet, oder einen Hydrocarbyloxyrest der Formel $R^o$-O- darstellt, der zusammen mit der Carbonylgruppe den Acylrest einer monoveresterten Kohlensäure bildet, oder einen Hydrocarbylaminorest der Formel $R^o$-N($R^1$)- bedeutet, worin $R^1$ für Wasserstoff steht oder die Bedeutung von $R^o$ hat, und der zusammen mit der Carbonylgruppe den Acylrest einer mono- oder di-substituierten Carbaminsäure bildet, oder Salze von solchen Verbindungen mit salzbildenden Eigenschaften, oder Komplexe von solchen Verbindungen, worin $A_1$, $A_2$ und $A_3$ für Wasserstoff stehen, mit dreiwertigen paramagnetischen Uebergangsmetallen, einschliesslich entsprechenden Lanthaniden, Metallen der dritten Hauptgruppe des Periodensystems und Radionucliden herstellt.

9. Verfahren nach einem der Ansprüche 1 und 3-7, dadurch gekennzeichnet, dass man Verbindungen der Formel I gemäss Anspruch 1, worin R, X und m die im Anspruch 1 gegebenen Bedeutungen haben, n einen Durchschnittswert von etwa 10 bis etwa 17 darstellt, und jeder der Reste $A_1$, $A_2$ und $A_3$, unabhängig von den anderen, Wasserstoff, Alk(en)oyl mit bis und mit 20 Kohlenstoffatomen, Alkyloxycarbonyl mit bis und mit 20 Kohlenstoffatomen im Alkylteil, worin bis und mit 5 Methylengruppen durch Sauerstoffatome ersetzt sein können, wobei jeweils zwei Kohlenstoffatome die Sauerstoffatome voneinander trennen, oder Alkylaminocarbonyl mit bis und mit 20, vorzugsweise mit bis und mit 7 Kohlenstoffatomen, im Alkylteil, der gegebenenfalls durch Carboxy, Niederalkyloxycarbonyl mit bis und mit 4 Kohlenstoffatomen im Niederalkylteil, Carbamoyl und/oder durch Amino, Hydroxy, Mercapto, Niederalkylthio mit bis und mit 4 Kohlenstoffatomen, Phenyl oder Hydroxyphenyl substituiert sein kann, oder Salze von solchen Verbindungen mit salzbildenden Eigenschaften, und Komplexe von solchen Verbindungen, worin $A_1$, $A_2$ und $A_3$ für Wasserstoff stehen, mit dreiwertigen paramagnetischen Uebergangsmetallen, inklusive entsprechenden Lanthaniden, und geeigneten Metallen der dritten Hauptgruppe des Periodensystems, sowie mit geeigneten Radionucliden herstellt.

10. Verfahren nach einem der Ansprüche 1 und 3-7, dadurch gekennzeichnet, dass man Verbindungen der Formel I gemäss Anspruch 1, worin R und X die im Anspruch 1 gegebenen Bedeutungen haben, m für 0 steht, n einen Durchschnittswert von etwa 10 bis etwa 17 darstellt, und jeder der Reste $A_1$, $A_2$ und $A_3$, unabhängig von den anderen, für Wasserstoff, Alkanoyl mit bis und mit 12 Kohlenstoffatomen, Alkyloxycarbonyl, worin Alkyl bis und mit 7 Kohlenstoffatome enthält, wobei eine oder zwei Methylengruppen durch Sauerstoff ersetzt sein können, und Sauerstoffatome jeweils durch zwei Kohlenstoffatome voneinander getrennt sind, oder Alkylaminocarbonyl steht, worin Alkyl bis und mit 7 Kohlenstoffatome enthält, das als Substituenten in 1- oder in 2-Stellung gegebenenfalls Alkyloxycarbonyl aufweist, worin Alkyl bis und mit 4 Kohlenstoffatome enthält, und Komplexe von solchen Verbindungen, worin $A_1$, $A_2$ und $A_3$ für Wasserstoff stehen, mit dreiwertigen paramagnetischen Uebergangsmetallen, einschliesslich entsprechenden Lanthaniden, und mit Metallen der dritten Hauptgruppe des Periodensystems, sowie mit geeigneten Radionucliden, herstellt.

11. Verfahren nach einem der Ansprüche 1, 3, 4, 6 und 7, dadurch gekennzeichnet, dass man Verbindungen der Formel I gemäss Anspruch 1, worin R für Methyl steht, X die im Anspruch 1 gegebene Bedeutung hat und m für 0 steht, n einen Durchschnittswert von etwa 10 bis etwa 17 darstellt, und

jeder der Reste $A_1$, $A_2$ und $A_3$ für Wasserstoff steht, und Komplexe von solchen Verbindungen mit dreiwertigen paramagnetischen Uebergangsmetallen, einschliesslich Lanthaniden, oder mit Metallen der dritten Hauptgruppe des Periodensystems, sowie mit geeigneten Radionucliden, herstellt.

**12.** Verfahren nach einem der Ansprüche 1, 3 und 4, dadurch gekennzeichnet, dass man N-[ω-Methoxy-(dodecakis-ethylenoxy)-carbonyl]-desferrioxamin B, worin Dodecakis-ethylenoxy einen Rest der Formel $-(CH_2-CH_2-O)_n-$ bedeutet, wobei n für einen Durchschnittswert von 12 steht, herstellt.

**13.** Verfahren nach einem der Ansprüche 1, 3 und 4, dadurch gekennzeichnet, dass man N-[ω-Methoxy-(heptadecakis-ethylenoxy)-carbonyl]-desferrioxamin B, worin Heptadecakis-ethylenoxy einen Rest der Formel $-(CH_2-CH_2-O)_n-$ bedeutet, wobei n für einen Durchschnittswert von 17 steht, herstellt.

**14.** Verfahren nach einem der Ansprüche 1, 3 und 4, dadurch gekennzeichnet, dass man N-[ω-Methoxy-(undecakis-ethylenoxy)-carbonyl]-desferrioxamin B, worin Undecakis-ethylenoxy einen Rest der Formel $-(CH_2-CH_2-O)_n-$ bedeutet, wobei n für einen Durchschnittswert von 11 steht, herstellt.

**15.** Verfahren nach einem der Ansprüche 1 und 3-5, dadurch gekennzeichnet, dass man N-[ω-Methoxy-(dodecakis-ethylenoxy)-carbonyl]-O,O',O''-tri-(n-octanoyl)-desferrioxamin B, worin Dodecakis-ethylenoxy einen Rest der Formel $-(CH_2-CH_2-O)_n-$ bedeutet, wobei n für einen Durchschnittswert von 12 steht, herstellt.

**16.** Verfahren nach einem der Ansprüche 1 und 3-5, dadurch gekennzeichnet, dass man N-[ω-Methoxy-(dodecakis-ethylenoxy)-carbonyl]-O,O',O''-tri-(ethoxycarbonyl)-desferrioxamin B, worin Dodecakis-ethylenoxy einen Rest der Formel $-(CH_2-CH_2-O)_n-$ bedeutet, wobei n für einen Durchschnittswert von 12 steht, herstellt.

**17.** Verfahren nach einem der Ansprüche 1 und 3-5, dadurch gekennzeichnet, dass man N-[ω-Methoxy-(dodecakis-ethylenoxy)-carbonyl]-O,O',O''-tri-(ethoxycarbonylmethylaminocarbonyl)-desferrioxamin B, worin Dodecakis-ethylenoxy einen Rest der Formel $-(CH_2-CH_2-O)_n-$ bedeutet, wobei n für einen Durchschnittswert von 12 steht, herstellt.

**18.** Verfahren nach einem der Ansprüche 1, 3, 4, 6 und 7, dadurch gekennzeichnet, dass man den Eisen-III-Komplex von N-[ω-Methoxy-(undecakis-ethylenoxy)-carbonyl]-desferrioxamin B, worin Undecakis-ethylenoxy einen Rest der Formel $-(CH_2-CH_2-O)_n-$ bedeutet, wobei n für einen Durchschnittswert von 11 steht, herstellt.

**19.** Verfahren nach einem der Ansprüche 1, 3, 4, 6 und 7, dadurch gekennzeichnet, dass man den Mangan-III-Komplex von N-[ω-Methoxy-(undecakis-ethylenoxy)-carbonyl]-desferrioxamin B, worin Undecakis-ethylenoxy einen Rest der Formel $-(CH_2-CH_2-O)_n-$ bedeutet, wobei n für einen Durchschnittswert von 11 steht, herstellt.

**20.** Die nach dem Verfahren der Ansprüche 1 und 3-19 erhältlichen Verbindungen.

**21.** Die nach dem Verfahren des Anspruchs 2 erhältlichen Verbindungen.

## Claims

## Claims for the following Contracting States : AT, BE, CH, LI, DE, FR, GB, IT, LU, NL, SE

**1.** A compound of the formula

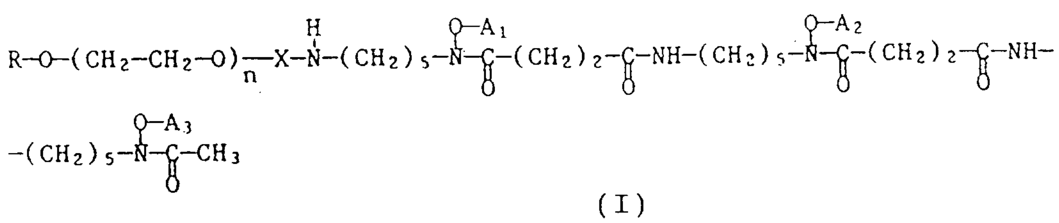

(I)

in which R is alkyl having up to 4 carbon atoms, n has an average value of at least 9, X is a radical of the formula -C(=O)-$(NH-SO_2)_m$- in which m is 0 or 1 and, if m is 1, the carbonyl group may be bonded to the oxygen atom or to the nitrogen atom, and each of the radicals $A_1$, $A_2$ and $A_3$, independently of the others, is hydrogen or an acyl radical, or a salt of a salt-forming compound of formula I, or a metal complex of a compound of formula I, in which $A_1$, $A_2$ and $A_3$ are hydrogen.

2. A compound of formula I according to claim 1 in which R, X and m have the meanings given in claim 1 and in which the radical of the formula $-(CH_2-CH_2-O)_n-$ is a group of the formula

$$-(CH_2-CH_2-O)_{n-1}CH_2-CH_2-O-$$

wherein the molecular weight of the $-(CH_2-CH_2-O)_{n-1}-$ radical is from approximately 500 to approximately 5000, and each of the radicals $A_1$, $A_2$ and $A_3$, independently of the others, is hydrogen or an acyl radical derived from a carboxylic acid, or a salt of such a compound having salt-forming properties.

3. A compound of formula I according to claim 1 in which R and X have the meanings given in claim 1 and $A_1$, $A_2$ and $A_3$ and the radical of the formula $-(CH_2-CH_2-O)_n-$ have the meanings given in claim 2, and in which m is 0, or a salt of a compound having salt-forming properties.

4. A compound of formula I according to claim 1 in which R, X and m have the meanings given in claim 1 and the radical of the formula $-(CH_2-CH_2-O)_n-$ has the meanings given in claim 2, and wherein $A_1$, $A_2$ and $A_3$ are hydrogen.

5. A compound of formula I according to claim 1 in which R, X and m have the meanings given in claim 1, n has an average value of from approximately 9 to approximately 115 and each of the radicals $A_1$, $A_2$ and $A_3$, independently of the others, is hydrogen or a radical of the formula Z-C(=O)- (Ib) in which Z is hydrogen or a hydrocarbyl radical $R^o$ that, together with the carbonyl group, forms the acyl radical of an unsubstituted or substituted acyclic, carbocyclic, carbocyclic-acyclic, heterocyclic or heterocyclic-acyclic carboxylic acid, or a hydrocarbyloxy radical of the formula $R^o$-O- that, together with the carbonyl group, forms the acyl radical of a monoesterified carbonic acid, or a hydrocarbylamino radical of the formula $R^o$-N($R^1$)- in which $R^1$ is hydrogen or has the meaning of $R^o$ and which, together with the carbonyl group, forms the acyl radical of a mono- or di- substituted carbamic acid, or a salt of such a compound having salt-forming properties, or a complex of such a compound, in which $A_1$, $A_2$ and $A_3$ are hydrogen, with a trivalent paramagnetic transition metal, including a corresponding lanthanide, a metal of main group 3 of the Periodic Table and a radionuclide.

6. A compound of formula I according to claim 1 in which R, X and m have the meanings given in claim 1, n has an average value of from approximately 10 to approximately 17, and each of the radicals $A_1$, $A_2$ and $A_3$, independently of the others, is hydrogen, alk(en)oyl having up to and including 20 carbon atoms, alkoxycarbonyl having up to and including 20 carbon atoms in the alkyl moiety, wherein up to and including 5 methylene groups may have been replaced by oxygen atoms and in each case two carbon atoms separate the oxygen atoms from one another, or alkylaminocarbonyl having up to and including 20, preferably up to and including 7, carbon atoms in the alkyl moiety, which may be unsubstituted or substituted by carboxy, lower alkoxycarbonyl having up to and including 4 carbon atoms in the lower alkyl moiety, carbamoyl and/or by amino, hydroxy, mercapto, lower alkylthio having up to and including 4 carbon atoms, phenyl or by hydroxyphenyl, or a salt of such a compound having salt-forming properties, or a complex of such a compound, in which $A_1$, $A_2$ and $A_3$ are hydrogen, with a trivalent paramagnetic transition metal, including a corresponding lanthanide, or with a suitable metal of main group 3 of the Periodic Table, or with a suitable radionuclide.

7. A compound of formula I according to claim 1 in which R and X have the meanings given in claim 1 and m is 0, n has an average value of from approximately 10 to approximately 17, and each of the radicals $A_1$, $A_2$ and $A_3$, independently of the others, is hydrogen, alkanoyl having up to and including 12 carbon atoms, alkoxycarbonyl in which alkyl has up to and including 7 carbon atoms, wherein one or two methylene groups may have been replaced by oxygen and oxygen atoms are in each case separated from each other by two carbon atoms, or alkylaminocarbonyl in which alkyl has up to and

including 7 carbon atoms and which may carry, as substituent in the 1- or 2-position, alkoxycarbonyl in which alkyl contains up to and including 4 carbon atoms, or a complex of such a compound, in which $A_1$, $A_2$ and $A_3$ are hydrogen, with a trivalent paramagnetic transition metal, including a corresponding lanthanide, or with a metal of main group 3 of the Periodic Table, or with a suitable radionuclide.

**8.** A compound of formula I according to claim 1 in which R is methyl, X has the meaning given in claim 1 and m is 0, n has an average value of from approximately 10 to approximately 17, and each of the radicals $A_1$, $A_2$ and $A_3$ is hydrogen, or a complex of such a compound with a trivalent paramagnetic transition metal, including a lanthanide, or with a metal of main group 3 of the Periodic Table, or with a suitable radionuclide.

**9.** N-[ω-methoxy-(dodecakis-ethyleneoxy)-carbonyl]-desferrioxamine B, in which dodecakis-ethyleneoxy denotes a radical of the formula $-(CH_2-CH_2-O)_n-$ in which n has an average value of 12, according to claim 1.

**10.** N-[ω-methoxy-(heptadecakis-ethyleneoxy)-carbonyl]-desferrioxamine B, in which heptadecakis-ethyleneoxy denotes a radical of the formula $-(CH_2-CH_2-O)_n-$ in which n has an average value of 17, according to claim 1.

**11.** N-[ω-methoxy-(undecakis-ethyleneoxy)-carbonyl]-desferrioxamine B, in which undecakis-ethyleneoxy denotes a radical of the formula $-(CH_2-CH_2-O)_n-$ in which n has an average value of 11, according to claim 1.

**12.** N-[ω-methoxy-(dodecakis-ethyleneoxy)-carbonyl]-O,O',O''-tri-(n-octanoyl)-desferrioxamine B, in which dodecakis-ethyleneoxy denotes a radical of the formula $-(CH_2-CH_2-O)_n-$ in which n has an average value of 12, according to claim 1.

**13.** N-[ω-methoxy-(dodecakis-ethyleneoxy)-carbonyl]-O,O',O''-tri-(ethoxycarbonyl)-desferrioxamine B, in which dodecakis-ethyleneoxy denotes a radical of the formula $-(CH_2-CH_2C)n-$ in which n has an average value of 12, according to claim 1.

**14.** N-[ω-methoxy-(dodecakis-ethyleneoxy)-carbonyl]-O,O',O''-tri-(ethoxycarbonylmethylaminocarbonyl)-desferrioxamine B, in which dodecakis-ethyleneoxy denotes a radical of the formula $-(CH_2-CH_2-O)_n-$ in which n has an average value of 12, according to claim 1.

**15.** Iron(III) complex of N-[ω-methoxy-(undecakis-ethyleneoxy)-carbonyl]-desferrioxamine B, in which undecakis-ethyleneoxy denotes a radical of the formula $-(CH_2-CH_2-O)_n-$ in which n has an average value of 11, according to claim 1.

**16.** Manganese(III) complex of N-[ω-methoxy-(undecakis-ethyleneoxy)-carbonyl]-desferrioxamine B, in which undecakis-ethyleneoxy denotes a radical of the formula $-(CH_2-CH_2-O)_n-$ in which n has an average value of 11, according to claim 1.

**17.** A compound according to any one of claims 2 to 4, 9 and 10 for use in a method for the therapeutic or diagnostic treatment of the human or animal body.

**18.** A compound according to any one of claims 5 to 8 and 11 to 16 for use in a method for the therapeutic or diagnostic treatment of the human or animal body.

**19.** A pharmaceutical preparation comprising a compound according to any one of claims 2 to 4, 9 and 10.

**20.** A pharmaceutical preparation comprising a compound according to any one of claims 5 to 8 and 11 to 14.

**21.** A diagnostic preparation comprising a compound according to any one of claims 1, 5 to 8, 15 and 16.

**22.** Use of a compound of claims 1, 5 to 8 and 11 to 16 for the manufacture of pharmaceutical or diagnostic preparations.

**23.** Use of a compound of claims 2 to 4, 9 and 10 for the manufacture of pharmaceutical preparations.

**24.** A process for the manufacture of a compound of formula I according to claim 1, which comprises reacting a compound of the formula

$R\text{-}O\text{-}(CH_2\text{-}CH_2\text{-}O)_n\text{-}Y_1$ (III)

with a compound of the formula

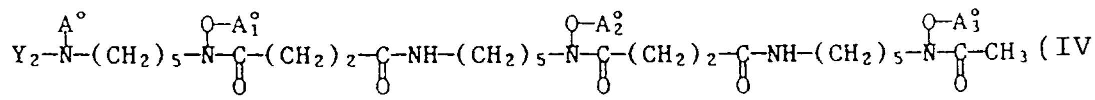 (IV)

or with a salt thereof, in which (a) $Y_2$ is hydrogen and $Y_1$ is a group of the formula $-X-Z_1$ (IIIa) in which $Z_1$ is a group Z that can be removed together with the hydrogen $Y_2$ to form the bond between the reactants, or (b) $Y_1$ is hydrogen and $Y_2$ is a group of the formula $Z_2-X-$ (IVa) in which $Z_2$ is a group Z or, if m in a radical X is 0, together with $A^o$ forms a bond, and in which $A^o$ is hydrogen or, if $Y_2$ is hydrogen, is an amino-protecting group, and each of the radicals $A_1^o$ , $A_2^o$ and $A_3^o$ , independently of the others, is hydrogen, a suitable protecting group or an acyl radical, functional groups in acyl radicals $A_1^o$ , $A_2^o$ and $A_3^o$ optionally being in protected form and, if desired or necessary, protecting groups present in a compound obtainable in accordance with the invention are removed and, if desired, in a compound of the formula I obtainable in accordance with the invention in which at least one of the groups $A_1$, $A_2$ and $A_3$ is hydrogen, this is replaced by an acyl radical and/or, if desired, a compound of the formula I obtainable in accordance with the invention in which $A_1$, $A_2$ and $A_3$ are hydrogen is converted into a metal complex and/or, if desired, a salt obtainable in accordance with the invention of a salt-forming compound of the formula I is converted into the free compound or a compound obtainable in accordance with the invention having salt-forming properties is converted into a salt.

**25.** A process according to claim 24 for the manufacture of a compound according to claim 2 which comprises reacting a starting material of formula III, in which n has the meanings given in claim 1 and the radical of the formula $-(CH_2\text{-}CH_2\text{-}O)_n-$ has the meanings given in claim 2, with a starting material of the formula IV in which each of the radicals $A_1^o$ , $A_2^o$ and $A_3^o$ , independently of the others, is hydrogen, an acyl radical of a carboxylic acid or an organic silyl group, an additional amino group optionally present in an acyl radical being in protected form, and $A^o$ is hydrogen or, if $Y_2$ is hydrogen, is an organic silyl group, wherein (a) $Y_2$ is hydrogen and $Y_1$ is a radical of the formula IIIa or (b) $Y_1$ is hydrogen and $Y_2$ is a radical of the formula IVa in which X has the meaning given in claim 1 and $Z_1$ and $Z_2$ are halogen having an atomic number of at least 19 or are 1-imidazolyl, and N- and/or O-silyl groups and/or amino-protecting groups that may be present are removed to free the corresponding hydroxy and amino groups, respectively, and, if desired, in a resulting compound of formula I in which at least one of the groups $A_1$, $A_2$ and $A_3$ is hydrogen, this is replaced by the acyl radical of a carboxylic acid by means of acylation and/or, if desired, a resulting free compound of the formula I having salt-forming properties is converted into a salt and/or a compound of formula I is freed from such a salt.

**26.** A compound obtainable in accordance with the process according to claim 24.

**27.** A compound obtainable in accordance with the process according to claim 25.

**Claims for the following Contracting States : ES, GR**

**1.** A process for the manufacture of

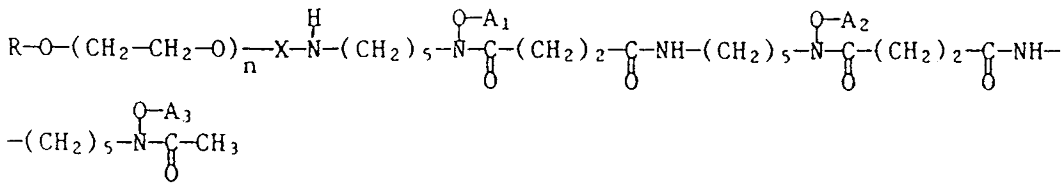

(I)

in which R is alkyl having up to 4 carbon atoms, n has an average value of at least 9, X is a radical of the formula $-C(=O)-(NH-SO_2)_m-$ in which m is 0 or 1 and, if m is 1, the carbonyl group may be bonded to the oxygen atom or to the nitrogen atom, and each of the radicals $A_1$, $A_2$ and $A_3$, independently of the others, is hydrogen or an acyl radical, or a salt of a salt-forming compound of formula I, or a metal complex of a compound of formula I, in which $A_1$, $A_2$ and $A_3$ are hydrogen, which process comprises reacting a compound of the formula

$R-O-(CH_2-CH_2-O)_n-Y_1$ (III)

with a compound of the formula

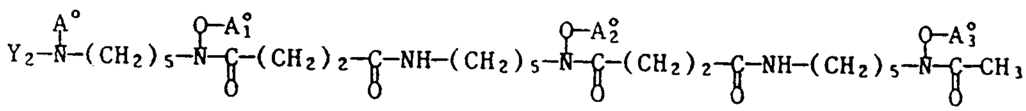

(IV)

or with a salt thereof, in which (a) $Y_2$ is hydrogen and $Y_1$ is a group of the formula $-X-Z_1$ (IIIa) in which $Z_1$ is a group Z that can be removed together with the hydrogen $Y_2$ to form the bond between the reactants, or (b) $Y_1$ is hydrogen and $Y_2$ is a group of the formula $Z_2-X-$ (IVa) in which $Z_2$ is a group Z or, if m in a radical X is 0, together with $A^o$ forms a bond, and in which $A^o$ is hydrogen or, if $Y_2$ is hydrogen, is an amino-protecting group, and each of the radicals $A^o_1$, $A^o_2$ and $A^o_3$, independently of the others, is hydrogen, a suitable protecting group or an acyl radical, functional groups in acyl radicals $A^o_1$, $A^o_2$ and $A^o_3$ optionally being in protected form and, if desired or necessary, protecting groups present in a compound obtainable in accordance with the invention are removed and, if desired, in a compound of the formula I obtainable in accordance with the invention in which at least one of the groups $A_1$, $A_2$ and $A_3$ is hydrogen, this is replaced by an acyl radical and/or, if desired, a compound of the formula I obtainable in accordance with the invention in which $A_1$, $A_2$ and $A_3$ are hydrogen is converted into a metal complex and/or, if desired, a salt obtainable in accordance with the invention of a salt-forming compound of the formula I is converted into the free compound or a compound obtainable in accordance with the invention having salt-forming properties is converted into a salt.

**2.** A process according to claim 1 for the manufacture of a compound of formula I according to claim 1, in which R, X and m have the meanings given in claim 1 and in which the radical of the formula $-(CH_2-CH_2-O)_n-$ is a group of the formula

$$-(CH_2-CH_2-O)_{n-1}CH_2-CH_2-O-$$

wherein the molecular weight of the $-(CH_2-CH_2-O)_{n-1}-$ radical is from approximately 500 to approximately 5000, and each of the radicals $A_1$, $A_2$ and $A_3$, independently of the others, is hydrogen or an acyl radical derived from a carboxylic acid, or a salt of such a compound having salt-forming properties, which process comprises reacting a starting material of formula III, in which n has the meanings given in claim 1 and the radical of the formula $-(CH_2-CH_2-O)_n-$ has the meanings given in

claim 2, with a starting material of the formula IV in which each of the radicals $A_1^o$, $A_2^o$ and $A_3^o$, independently of the others, is hydrogen, an acyl radical of a carboxylic acid or an organic silyl group, an additional amino group optionally present in an acyl radical being in protected form, and $A^o$ is hydrogen or, if $Y_2$ is hydrogen, is an organic silyl group, wherein (a) $Y_2$ is hydrogen and $Y_1$ is a radical of the formula IIIa or (b) $Y_1$ is hydrogen and $Y_2$ is a radical of the formula IVa in which X has the meaning given in claim 1 and $Z_1$ and $Z_2$ are halogen having an atomic number of at least 19 or are 1-imidazolyl, and N-and/or O-silyl groups and/or amino-protecting groups that may be present are removed to free the corresponding hydroxy and amino groups, respectively, and, if desired, in a resulting compound of formula I in which at least one of the group symbols $A_1$, $A_2$ and $A_3$ is hydrogen, this is replaced by the acyl radical of a carboxylic acid by means of acylation and/or, if desired, a resulting free compound of the formula I having salt-forming properties is converted into a salt and/or a compound of formula I is freed from such a salt.

**3.** A process according to claim 1, wherein Z is reactive esterified hydroxy, such as halogen, having an atomic number of at least 19, for example chlorine, or azido, or a suitable monocyclic azacyclic group bonded by way of a ring nitrogen atom, for example 1-imidazolyl.

**4.** A process according to either claim 1 or claim 3 wherein the protecting groups $A_1^o$, $A_2^o$ and/or $A_3^o$, and also $A^o$ are organic silyl groups, such as trimethylsilyl.

**5.** A process according to any one of claims 1, 3 and 4 wherein in a compound of formula I obtainable in accordance with the invention in which at least one of the radicals $A_1$, $A_2$ and/or $A_3$ is hydrogen, this is replaced by an acyl radical by treatment with an agent that introduces an acyl radical, such as an anhydride or an activated ester of a corresponding acid, or of an acid in the presence of a suitable condensing agent.

**6.** A process according to any one of claims 1, 3 and 4 which comprises converting a compound of formula I obtainable in accordance with the invention in which $A_1$, $A_2$ and $A_3$ are hydrogen into a metal complex by treatment with an inorganic or organic salt or derivative of a metal, such as with a corresponding complex with an organic compound of which the binding affinity to the metal ions is lower than that of compounds of formula I in which $A_1$, $A_2$ and $A_3$ are hydrogen, such as a suitable $\beta$-dicarbonyl compound, for example acetylacetone.

**7.** A process according to claim 6 in which a salt or a derivative is used, for example a complex of iron-(III), manganese(III), chromium(III) or gadolinium(III), for example the corresponding acetylacetonate.

**8.** A process according to any one of claims 1 and 3 to 7 wherein a compound of formula I according to claim 1 in which R, X and m have the meanings given in claim 1, n has an average value of from approximately 9 to approximately 115 and each of the radicals $A_1$, $A_2$ and $A_3$, independently of the others, is hydrogen or a radical of the formula Z-C(=O)- (Ib) in which Z is hydrogen or a hydrocarbyl radical $R^o$ that, together with the carbonyl group, forms the acyl radical of an unsubstituted or substituted acyclic, carbocyclic, carbocyclic-acyclic, heterocyclic or heterocyclic-acyclic carboxylic acid, or a hydrocarbyloxy radical of the formula $R^o$-O- that, together with the carbonyl group, forms the acyl radical of a monoesterified carbonic acid, or a hydrocarbylamino radical of the formula $R^o$-N($R^1$)- in which $R^1$ is hydrogen or has the meaning of $R^o$ and which, together with the carbonyl group, forms the acyl radical of a mono- or di- substituted carbamic acid, or a salt of such a compound having salt-forming properties, or a complex of such a compound, in which $A_1$, $A_2$ and $A_3$ are hydrogen, with a trivalent paramagnetic transition metal, including a corresponding lanthanide, a metal of main group 3 of the Periodic Table or a radionuclide, is manufactured.

**9.** A process according to any one of claims 1 and 3 to 7 wherein a compound of formula I according to claim 1 in which R, X and m have the meanings given in claim 1, n has an average value of from approximately 10 to approximately 17, and each of the radicals $A_1$, $A_2$ and $A_3$, independently of the others, is hydrogen, alk(en)oyl having up to and including 20 carbon atoms, alkoxycarbonyl having up to and including 20 carbon atoms in the alkyl moiety, wherein up to and including 5 methylene groups may have been replaced by oxygen atoms and in each case two carbon atoms separate the oxygen atoms from one another, or alkylaminocarbonyl having up to and including 20, preferably up to and including 7, carbon atoms in the alkyl moiety, which may be unsubstituted or substituted by carboxy,

lower alkoxycarbonyl having up to and including 4 carbon atoms in the lower alkyl moiety, carbamoyl and/or by amino, hydroxy, mercapto, lower alkylthio having up to and including 4 carbon atoms, phenyl or by hydroxyphenyl, or a salt of such a compound having salt-forming properties, or a complex of such a compound, in which $A_1$, $A_2$ and $A_3$ are hydrogen, with a trivalent paramagnetic transition metal, including a corresponding lanthanide, or with a suitable metal of main group 3 of the Periodic Table, or with a suitable radionuclide, is manufactured.

**10.** A process according to any one of claims 1 and 3 to 7 wherein a compound of formula I according to claim 1 in which R and X have the meanings given in claim 1 and m is 0, n has an average value of from approximately 10 to approximately 17, and each of the radicals $A_1$, $A_2$ and $A_3$, independently of the others, is hydrogen, alkanoyl having up to and including 12 carbon atoms, alkoxycarbonyl in which alkyl has up to and including 7 carbon atoms, wherein one or two methylene groups may have been replaced by oxygen and oxygen atoms are in each case separated from each other by two carbon atoms, or alkylaminocarbonyl in which alkyl has up to and including 7 carbon atoms and which may carry, as substituent in the 1-or 2-position, alkoxycarbonyl in which alkyl contains up to and including 4 carbon atoms, or a complex of such a compound, in which $A_1$, $A_2$ and $A_3$ are hydrogen, with a trivalent paramagnetic transition metal, including a corresponding lanthanide, or with a metal of main group 3 of the Periodic Table, or with a suitable radionuclide, is manufactured.

**11.** A process according to any one of claims 1, 3, 4, 6 and 7 wherein a compound of formula I according to claim 1 in which R is methyl, X has the meaning given in claim 1 and m is 0, n has an average value of from approximately 10 to approximately 17, and each of the radicals $A_1$, $A_2$ and $A_3$ is hydrogen, or a complex of such a compound with a trivalent paramagnetic transition metal, including a lanthanide, or with a metal of main group 3 of the Periodic Table, or with a suitable radionuclide, is manufactured.

**12.** A process according to any one of claims 1, 3 and 4 wherein N-[ω-methoxy-(dodecakis-ethyleneoxy)-carbonyl]-desferrioxamine B, in which dodecakis-ethyleneoxy denotes a radical of the formula $-(CH_2-CH_2-O)_n-$ in which n has an average value of 12, is manufactured.

**13.** A process according to any one of claims 1, 3 and 4 wherein N-[ω-methoxy-(heptadecakis-ethyleneoxy)-carbonyl]-desferrioxamine B, in which heptadecakis-ethyleneoxy denotes a radical of the formula $-(CH_2-CH_2-O)_n-$ in which n has an average value of 17, is manufactured.

**14.** A process according to any one of claims 1, 3 and 4 wherein N-[ω-methoxy-(undecakis-ethyleneoxy)-carbonyl]-desferrioxamine B, in which undecakis-ethyleneoxy denotes a radical of the formula $-(CH_2-CH_2-O)_n-$ in which n has an average value of 11, is manufactured.

**15.** A process according to any one of claims 1 and 3 to 5 wherein N-[ω-methoxy-(dodecakis-ethyleneoxy)-carbonyl]-O,O',O''-tri-(n-octanoyl)-desferrioxamine B, in which dodecakis-ethyleneoxy denotes a radical of the formula $-(CH_2-CH_2-O)_n-$ in which n has an average value of 12, is manufactured.

**16.** A process according to any one of claims 1 and 3 to 5 wherein N-[ω-methoxy-(dodecakis-ethyleneoxy)-carbonyl]-O,O',O''-tri-(ethoxycarbonyl)-desferrioxamine B, in which dodecakis-ethyleneoxy denotes a radical of the formula $-(CH_2-CH_2-O)_n-$ in which n has an average value of 12, is manufactured.

**17.** A process according to any one of claims 1 and 3 to 5 wherein N-[ω-methoxy-(dodecakis-ethyleneoxy)-carbonyl]-O,O',O''-tri-(ethoxycarbonylmethylaminocarbonyl)-desferrioxamine B, in which dodecakis-ethyleneoxy denotes a radical of the formula $-(CH_2-CH_2-O)_n-$ in which n has an average value of 12, is manufactured.

**18.** A process according to any one of claims 1, 3, 4, 6 and 7, wherein the iron(III) complex of N-[ω-methoxy-(undecakis-ethyleneoxy)-carbonyl]-desferrioxamine B, in which undecakis-ethyleneoxy denotes a radical of the formula $-(CH_2-CH_2-O)_n-$ in which n has an average value of 11, is manufactured.

**19.** A process according to any one of claims 1, 3, 4, 6 and 7, wherein the manganese(III) complex of N-[ω-methoxy-(undecakis-ethyleneoxy)-carbonyl]-desferrioxamine B, in which undecakis-ethyleneoxy denotes a radical of the formula $-(CH_2-CH_2-O)_n-$ in which n has an average value of 11, is manufactured.

**20.** A compound obtainable in accordance with the process of claims 1 and 3 to 19.

**21.** A compound obtainable in accordance with the process of claim 2.

## Revendications

**Revendications pour les Etats contractants suivants : AT, BE, CH, LI, DE, FR, GB, IT, LU, NL, SE**

**1.** Composés de formule

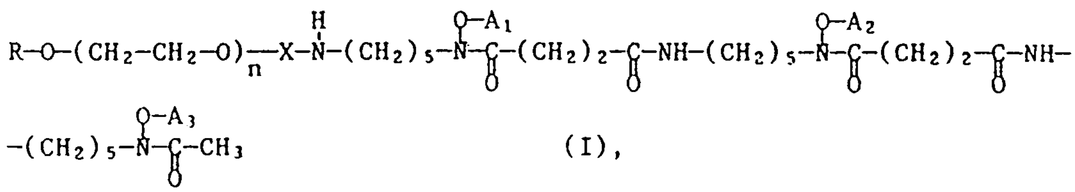

(I),

dans laquelle R représente un groupe alkyle ayant jusqu'à 4 atomes de carbone, n a une valeur moyenne d'au moins 9, X représente un radical de formule $-C(=O)-(NH-SO_2)_m-$, étant 0 ou 1, et, lorsque m est 1, le groupe carbonyle pouvant être lié à l'atome d'oxygène ou à l'atome d'azote, et chacun des radicaux $A_1$, $A_2$ et $A_3$, indépendamment des autres, représente un atome d'hydrogène ou un radical acyle, et sels des composés de formule I salifiables, ainsi que complexes métalliques des composés de formule I dans lesquels $A_1$, $A_2$ et $A_3$ représentent des atomes d'hydrogène.

**2.** Composés de formule I selon la revendication 1, dans lesquels R, X et m ont les significations données dans la revendication 1, et dans lesquels le radical de formule $-(CH_2-CH_2-O)_n-$ représente le groupe de formule $-(CH_2-CH_2-O)_{n-1}-CH_2-CH_2-O-$, la masse moléculaire du radical $-(CH_2-CH_2-O)_{n-1}-$ étant comprise entre environ 500 et environ 5 000, et chacun des radicaux $A_1$, $A_2$ et $A_3$, indépendamment des autres, représente un atome d'hydrogène ou un reste acyle dérivé d'un acide carboxylique, ou sels de tels composés à propriétés salifiables.

**3.** Composés de formule I selon la revendication 1, dans lesquels R et X ont les significations données dans la revendication 1, et $A_1$, $A_2$ et $A_3$, ainsi que le radical de formule $-(CH_2-CH_2-O)_n-$ ont les significations données dans la revendication 2, et m représente 0, et sels des composés à propriétés salifiables.

**4.** Composés de formule I selon la revendication 1, dans lesquels R, X et m ont les significations données dans la revendication 1 et le radical de formule $-(CH_2-CH_2-O)_n-$ a les significations données dans la revendication 2, et dans lesquels $A_1$, $A_2$ et $A_3$ représentent des atomes d'hydrogène.

**5.** Composés de formule I selon la revendication 1, dans lesquels R, X et m ont les significations données dans la revendication 1, n a une valeur moyenne comprise entre environ 9 et environ 115, et chacun des radicaux $A_1$, $A_2$ et $A_3$, indépendamment des autres, représente un atome d'hydrogène ou un radical de formule $Z-C(=O)-$ (Ib), dans laquelle Z représente un atome d'hydrogène ou un radical hydrocarboné $R^o$ qui forme, conjointement avec le groupe carbonyle, le reste acyle d'un acide carboxylique acyclique, carbocyclique, carbocyclique-acyclique, hétérocyclique ou hétérocyclique-acyclique, éventuellement substitué, ou représente un radical hydrocarboné-oxy de formule $R^o-O-$ qui forme, conjointement avec le groupe carbonyle, le reste acyle d'un acide carbonique monoestérifié, ou représente un radical hydrocarboné-amino de formule $R^o-N(R^1)-$, dans laquelle $R^1$ représente un atome d'hydrogène ou a la signification de $R^o$,ou qui forme, conjointement avec le groupe carbonyle, le reste acyle d'un acide carbamique mono- ou disubstitué, ou sels de tels composés à propriétés salifiables, ou complexes de tels composés, dans lesquels $A_1$, $A_2$ et $A_3$ représentent des atomes d'hydrogène, avec des métaux de transition paramagnétiques trivalents, y compris les lanthanides correspondants, des métaux du groupe IIIA du système périodique et des radionucléides.

**6.** Composés de formule I selon la revendication 1, dans lesquels R, X et m ont les significations données dans la revendication 1, n a une valeur moyenne d'environ 10 à environ 17, et chacun des radicaux $A_1$, $A_2$ et $A_3$, indépendamment des autres, représente un atome d'hydrogène ou un groupe alkyle ou

alcényle ayant jusqu'à et y compris 20 atomes de carbone, alkyloxycarbonyle ayant jusqu'à et y compris 20 atomes de carbone dans le fragment alkyle, dans lequel jusqu'à et y compris 5 groupes méthylène peuvent être remplacés par des atomes d'oxygène, dans chaque cas 2 atomes de carbone séparant l'un de l'autre les atomes d'oxygène, ou un groupe alkylaminocarbonyle ayant jusqu'à et y compris 20, de préférence jusqu'à et y compris 7 atomes de carbone dans le fragment alkyle, qui peut éventuellement être substitué par des substituants carboxy, (alkyl inférieur)-oxycarbonyle ayant jusqu'à et y compris 4 atomes de carbone dans le fragment alkyle inférieur, carbamoyle et/ou par des substituants amino, hydroxy, mercapto, (alkyl inférieur)-thio ayant jusqu'à et y compris 4 atomes de carbone, phényle ou hydroxyphényle, ou sels de tels composés à propriétés salifiables, et complexes de tels composés, dans lesquels $A_1$, $A_2$ et $A_3$ représentent des atomes d'hydrogène, avec des métaux de transition paramagnétiques trivalents, y compris les lanthanides correspondants, et des métaux appropriés du groupe IIIA du système périodique, ainsi qu'avec des radionucléides appropriés.

**7.** Composés de formule I selon la revendication 1, dans lesquels R et X ont les significations données dans la revendication 1, m est 0, n a une valeur moyenne d'environ 10 à environ 17, et chacun des radicaux $A_1$, $A_2$ et $A_3$, indépendamment des autres, représente un atome d'hydrogène ou un groupe alcanoyle ayant jusqu'à et y compris 12 atomes de carbone, un radical alkyloxycarbonyle dans lequel le fragment alkyle contient jusqu'à et y compris 7 atomes de carbone, 1 ou 2 groupes méthylène pouvant être remplacés par un atome d'oxygène, et les atomes d'oxygène étant séparés l'un de l'autre dans chaque cas par 2 atomes de carbone, ou un radical alkylaminocarbonyle dans lequel le fragment alkyle contient jusqu'à et y compris 7 atomes de carbone, qui comporte éventuellement en tant que substituant en position 1 ou 2 un groupe alkyloxycarbonyle, dans lequel le fragment alkyle contient jusqu'à et y compris 4 atomes de carbone, et complexes de tels composés dans lesquels $A_1$, $A_2$ et $A_3$ représentent des atomes d'hydrogène, avec des métaux de transition paramagnétiques trivalents, y compris des lanthanides appropriés, et avec des métaux du groupe IIIA du système périodique, ainsi qu'avec des radionucléides appropriés.

**8.** Composés de formule I selon la revendication 1, dans lesquels R représente le groupe méthyle, X a la signification donnée dans la revendication 1 et m est 0, n a une valeur moyenne d'environ 10 à environ 17, et chacun des radicaux $A_1$, $A_2$ et $A_3$ représente un atome d'hydrogène, et complexes de tels composés avec des métaux de transition paramagnétiques trivalents, y compris des lanthanides, ou avec des métaux du groupe IIIA du système périodique, ainsi qu'avec des radionucléides appropriés.

**9.** N-[ω-méthoxy-(dodécakis-éthylène-oxy)-carbonyl]-desferrioxamine B, dans laquelle le radical dodécakis-éthylène-oxy représente un radical de formule $-(CH_2-CH_2-O)_n-$, n ayant une valeur moyenne de 12, selon la revendication 1.

**10.** N-[ω-méthoxy-(heptadécakis-éthylène-oxy)-carbonyl]-desferrioxamine B, dans laquelle le radical heptadécakis-éthylène-oxy représente un radical de formule $-(CH_2-CH_2-O)_n-$, n ayant une valeur moyenne de 17, selon la revendication 1.

**11.** N-[ω-méthoxy-(undécakis-éthylène-oxy)-carbonyl]-desferrioxamine B, dans laquelle le radical undécakis-éthylène-oxy représente un radical de formule $-(CH_2-CH_2-O)_n-$, n ayant une valeur moyenne de 11, selon la revendication 1.

**12.** N-[ω-méthoxy-(dodécakis-éthylène-oxy)-carbonyl]-O,O',O''-tri-(n-octanoyl)-desferrioxamine B, dans laquelle le radical dodécakis-éthylène-oxy représente un radical de formule $-(CH_2-CH_2-O)_n-$, n ayant une valeur moyenne de 12, selon la revendication 1.

**13.** N-[ω-méthoxy-(dodécakis-éthylène-oxy)-carbonyl]-O,O,'O''-tri-(éthoxycarbonyl)-desferrioxamine B, dans laquelle le radical dodécakis-éthylène-oxy représente un radical de formule $-(CH_2-CH_2-O)_n-$, n ayant une valeur moyenne de 12, selon la revendication 1.

**14.** N-[ω-méthoxy-(dodécakis-éthylène-oxy)-carbonyl]-O,O,'O''-tri-(éthoxycarbonylméthylaminocarbonyl)-desferrioxamine B, dans laquelle le radical dodécakis-éthylène-oxy représente un radical de formule $-(CH_2-CH_2-O)_n-$, n ayant une valeur moyenne de 12, selon la revendication 1.

**15.** Complexe ferrique de N-[ω-méthoxy-(undécakis-éthylène-oxy)-carbonyl]-desferrioxamine B, dans laquelle le radical undécakis-éthylène-oxy représente un radical de formule $-(CH_2-CH_2-O)_n-$, n ayant une valeur moyenne de 11, selon la revendication 1.

**16.** Complexe manganique de N-[ω-méthoxy-(undécakis-éthylène-oxy)-carbonyl]-desferrioxamine B, dans laquelle le radical undécakis-éthylène-oxy représente un radical de formule $-(CH_2-CH_2-O)_n-$, n ayant une valeur moyenne de 11, selon la revendication 1.

**17.** Composé selon l'une des revendications 2-4, 9 et 10, pour utilisation dans un procédé pour le traitement thérapeutique ou diagnostique de l'organisme humain et de l'organisme animal.

**18.** Composé selon l'une des revendications 5-8, et 11-16, pour utilisation dans un procédé pour le traitement thérapeutique ou diagnostique de l'organisme humain et de l'organisme animal.

**19.** Compositions pharmaceutiques contenant un composé selon l'une des revendications 2-4, 9 et 10.

**20.** Compositions pharmaceutiques contenant un composé selon l'une des revendications 5-8 et 11-14.

**21.** Compositions diagnostiques contenant un composé selon l'une des revendications 1, 5-8, 15 et 16.

**22.** Utilisation des composés des revendications 1, 5-8 et 11-16, pour la préparation de compositions pharmaceutiques ou diagnostiques.

**23.** Utilisation des composés des revendications 2-4, 9 et 10 pour la préparation de compositions pharmaceutiques.

**24.** Procédé pour la préparation des composés de formule I selon la revendication 1, caractérisé en ce que l'on fait réagir un composé de formule

$R-O-(CH_2-CH_2-O)_n-Y_1$ (III)

avec un composé de formule

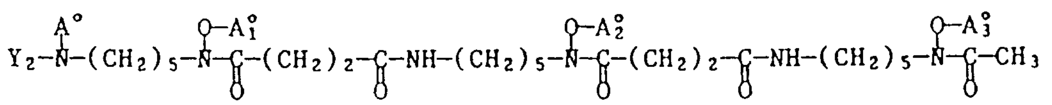

(IV)

ou un sel de celui-ci, (a) $Y_2$ représentant un atome d'hydrogène et $Y_1$ représentant un groupe de formule $-X-Z_1$ (IIIa),dans laquelle $Z_1$ représente un groupe Z séparable conjointement avec l'atome d'hydrogène $Y_2$, avec formation de la liaison entre les partenaires réactionnels, ou (b) $Y_1$ représentant un atome d'hydrogène et $Y_2$ représentant un groupe de formule $Z_2-X-$ (IVa), dans laquelle $Z_2$ représente un groupe Z, ou, lorsque m dans dans un radical X représente 0, forme conjointement avec $A^o$ une liaison, et dans laquelle $A^o$ représente un atome d'hydrogène ou, lorsque $Y_2$ représente un atome d'hydrogène, un groupe protecteur de fonction amino, et chacun des radicaux $A_1^o$ , $A_2^o$ et $A_3^o$ représente, indépendamment les uns des autres, un atome d'hydrogène, un groupe protecteur approprié ou un radical acyle, des groupes fonctionnels dans les radicaux acyle $A_1^o$ , $A_2^o$ et $A_3^o$ se trouvant éventuellement sous forme protégée, et, si nécessaire ou si on le désire, on élimine les groupes protecteurs présents dans un composé préparable selon l'invention, et, si on le désire, dans un composé de formule I préparable selon l'invention, dans lequel au moins l'un des groupes $A_1$, $A_2$ et $A_3$ représente un atome d'hydrogène, on remplace celui-ci par un radical acyle, et/ou, si on le désire, on convertit en un complexe métallique un composé de formule I préparable selon l'invention, dans lequel $A_1$, $A_2$ et $A_3$ représentent des atomes d'hydrogène, et/ou, si on le désire, on convertit un sel, préparable selon l'invention, d'un composé salifiable de formule I, en le composé libre, ou un composé, préparable selon l'invention, à propriétés salifiables, en un sel.

**25.** Procédé selon la revendication 24, pour la préparation de composés selon la revendication 2, caractérisé en ce que l'on fait réagir des produits de départ de formule III, dans lesquels n a la signification donnée dans la revendication 1 et le radical de formule $-(CH_2-CH_2-O)_n-$ a la signification donnée dans la revendication 2, avec des produits de départ de formule IV dans lesquels chacun des radicaux $A_1^o$ , $A_2^o$ et $A_3^o$ , indépendamment des autres, représente un atome d'hydrogène ou des restes acyle d'acides carboxyliques ou des groupes silyle organiques, dans un reste acyle un groupe amino supplémentaire éventuellement présent se trouvant sous forme protégée, et $A^o$ représente un atome d'hydrogène ou, lorsque $Y_2$ représente un atome d'hydrogène, un groupe silyle organique, (a) $Y_2$ représentant un atome d'hydrogène et $Y_1$ représentant le radical de formule IIIa ou (b) $Y_1$ représentant un atome d'hydrogène et $Y_2$ représentant le radical de formule IVa dans lequel X a la signification donnée dans la revendication 1, et $Z_1$ et $Z_2$ représentent un atome d'halogène ayant un numéro atomique d'au moins 19, ou le radical 1-imidazolyle, et on élimine des groupes protecteurs de fonction amino et/ou des groupes N- et/ou O-silyle éventuellement présents, avec libération des groupes amino ou, respectivement, hydroxy correspondants, et, si on le désire, dans un composé de formule I obtenu, dans lequel au moins l'un des groupes $A_1$, $A_2$ et $A_3$ représente un atome d'hydrogène, on remplace celui-ci, par acylation, par le reste acyle d'un acide carboxylique, et/ou, si on le désire, on convertit en un sel un composé libre de formule I obtenu, à propriétés salifiables, et/ou on libère un composé de formule I à partir d'un tel sel.

**26.** Composés préparables par le procédé selon la revendication 24.

**27.** Composés préparables par le procédé selon la revendication 25.

**Revendications pour les Etats contractants suivants : ES, GR**

**1.** Procédé pour la préparation de composés de formule

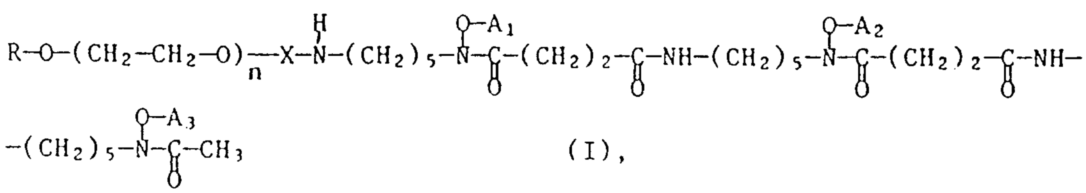

(I),

dans laquelle R représente un groupe alkyle ayant jusqu'à 4 atomes de carbone, n a une valeur moyenne d'au moins 9, X représente un radical de formule $-C(=O)-(NH-SO_2)_m-$, m étant 0 ou 1, et, lorsque m est 1, le groupe carbonyle pouvant être lié à l'atome d'oxygène ou à l'atome d'azote, et chacun des radicaux $A_1$, $A_2$ et $A_3$, indépendamment des autres, représente un atome d'hydrogène ou un radical acyle, et de sels des composés de formule I salifiables, ainsi que de complexes métalliques des composés de formule I dans lesquels $A_1$, $A_2$ et $A_3$ représentent des atomes d'hydrogène, caractérisé en ce que l'on fait réagir un composé de formule

$R-O-(CH_2-CH_2-O)_n-Y_1$ (III)

avec un composé de formule

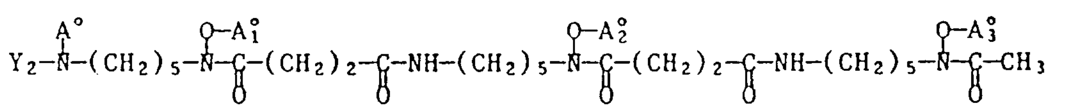

(IV)

ou un sel de celui-ci, (a) $Y_2$ représentant un atome d'hydrogène et $Y_1$ représentant un groupe de formule $-X-Z_1$ (IIIa),dans laquelle $Z_1$ représente un groupe Z séparable conjointement avec l'atome d'hydrogène $Y_2$, avec formation de la liaison entre les partenaires réactionnels, ou (b) $Y_1$ représentant un atome d'hydrogène et $Y_2$ représentant un groupe de formule $Z_2-X-$ (IVa), dans laquelle $Z_2$ représente un groupe Z, ou, lorsque m dans dans un radical X représente 0, forme conjointement avec $A^o$ une liaison, et dans laquelle $A^o$ représente un atome d'hydrogène ou, lorsque $Y_2$ représente un

atome d'hydrogène, un groupe protecteur de fonction amino, et chacun des radicaux $A_1^o$ , $A_2^o$ et $A_3^o$ représente, indépendamment les uns des autres, un atome d'hydrogène, un groupe protecteur approprié ou un radical acyle, des groupes fonctionnels dans les radicaux acyle $A_1^o$ , $A_2^o$ et $A_3^o$ se trouvant éventuellement sous forme protégée, et, si nécessaire ou si on le désire, on élimine les groupes protecteurs présents dans un composé préparable selon l'invention, et, si on le désire, dans un composé de formule I préparable selon l'invention, dans lequel au moins l'un des groupes $A_1$, $A_2$ et $A_3$ représente un atome d'hydrogène, on remplace celui-ci par un radical acyle, et/ou, si on le désire, on convertit en un complexe métallique un composé de formule I préparable selon l'invention, dans lequel $A_1$, $A_2$ et $A_3$ représentent des atomes d'hydrogène, et/ou, si on le désire, on convertit un sel, préparable selon l'invention, d'un composé salifiable de formule I, en le composé libre, ou un composé, préparable selon l'invention, à propriétés salifiables, en un sel.

2. Procédé selon la revendication 1, pour la préparation de composés de formule I selon la revendication 1, dans lesquels R, X et m ont les significations données dans la revendication 1, et dans lesquels le radical de formule $-(CH_2-CH_2-O)_n-$ représente le groupe de formule $-(CH_2-CH_2-O)_{n-1}-CH_2-CH_2-O-$, la masse moléculaire du radical $-(CH_2-CH_2-O)_{n-1}-$ étant comprise entre environ 500 et environ 5 000, et chacun des radicaux $A_1$, $A_2$ et $A_3$, indépendamment des autres, représente un atome d'hydrogène ou un reste acyle dérivé d'un acide carboxylique, ou de sels de tels composés à propriétés salifiables, caractérisé en ce que l'on fait réagir des produits de départ de formule III, dans lesquels n a la signification donnée dans la revendication 1 et le radical de formule $-(CH_2-CH_2-O)_n-$ a la signification donnée dans la revendication 2, avec des produits de départ de formule IV dans lesquels chacun des radicaux $A_1^o$ , $A_2^o$ et $A_3^o$ , indépendamment des autres, représente un atome d'hydrogène ou des restes acyle d'acides carboxyliques ou des groupes silyle organiques, dans un reste acyle un groupe amino supplémentaire éventuellement présent se trouvant sous forme protégée, et $A^o$ représente un atome d'hydrogène ou, lorsque $Y_2$ représente un atome d'hydrogène, un groupe silyle organique, (a) $Y_2$ représentant un atome d'hydrogène et $Y_1$ représentant le radical de formule IIIa ou (b) $Y_1$ représentant un atome d'hydrogène et $Y_2$ représentant le radical de formule IVa dans lequel X a la signification donnée dans la revendication 1, et $Z_1$ et $Z_2$ représentent un atome d'halogène ayant un numéro atomique d'au moins 19, ou le radical 1-imidazolyle, et on élimine des groupes protecteurs de fonction amino et/ou des groupes N- et/ou O-silyle éventuellement présents, avec libération des groupes amino ou, respectivement, hydroxy correspondants, et, si on le désire, dans un composé de formule I obtenu, dans lequel au moins l'un des groupes $A_1$, $A_2$ et $A_3$ représente un atome d'hydrogène, on remplace celui-ci, par acylation, par le reste acyle d'un acide carboxylique, et/ou, si on le désire, on convertit en un sel un composé libre de formule I obtenu, à propriétés salifiables, et/ou on libère un composé de formule I à partir d'un tel sel.

3. Procédé selon la revendication 1, caractérisé en ce que Z représente un groupe hydroxy estérifié réactif, tel qu'un halogène ayant un numéro atomique d'au moins 19, par exemple le chlore, ou un groupe azido, ou un groupe azacyclique ou monocyclique approprié, lié par l'intermédiaire d'un atome d'azote formant le cycle, par exemple le groupe 1-imidazolyle.

4. Procédé selon la revendication 1 ou 3, caractérisé en ce que les groupes protecteurs $A_1^o$ , $A_2^o$ et/ou $A_3^o$ ainsi que $A^o$ représentent des groupes silyle organiques, tels que le groupe triméthylsilyle.

5. Procédé selon l'une des revendications 1, 3 et 4, caractérisé en ce que, dans un composé de formule I préparable selon l'invention, dans lequel au moins un radical $A_1$, $A_2$ et/ou $A_3$ représente un atome d'hydrogène, on remplace ce dernier par un radical acyle, par traitement par un agent introduisant un radical acyle, tel qu'un anhydride ou un ester activé d'un acide correspondant, ou un acide en présence d'un agent de condensation approprié.

6. Procédé selon l'une des revendications 1, 3 et 4, caractérisé en ce que l'on convertit en un complexe métallique un composé de formule I, préparable selon l'invention, dans lequel $A_1$, $A_2$ et $A_3$ représentent des atomes d'hydrogène, par traitement par un sel organique ou minéral ou un dérivé d'un métal, tel qu'un complexe approprié avec un composé organique, dont l'affinité de liaison aux ions métalliques est plus faible que celle des composés de formule I dans lesquels $A_1$, $A_2$ et $A_3$ représentent des atomes d'hydrogène, tels qu'un composé $\beta$-dicarbonyle approprié, par exemple l'acétylacétone.

7. Procédé selon la revendication 6, caractérisé en ce que l'on utilise un sel ou un dérivé, par exemple un complexe de fer- (III), manganèse-(III), chrome-(III) ou gadolinium-(III), par exemple l'acétylacétonate correspondant.

8. Procédé selon l'une des revendications 1 et 3-7, caractérisé en ce que l'on prépare des composés de formule I selon la revendication 1 dans lesquels R, X et m ont les significations données dans la revendication 1, n a une valeur moyenne comprise entre environ 9 et environ 115, et chacun des radicaux $A_1$, $A_2$ et $A_3$, indépendamment des autres, représente un atome d'hydrogène ou un radical de formule Z-C(=O)- (Ib), dans laquelle Z représente un atome d'hydrogène ou un radical hydrocarboné $R^o$ qui forme, conjointement avec le groupe carbonyle, le reste acyle d'un acide carboxylique acyclique, carbocyclique, carbocyclique-acyclique, hétérocyclique ou hétérocyclique-acyclique, éventuellement substitué, ou représente un radical hydrocarbonéoxy de formule $R^o$-O- qui forme, conjointement avec le groupe carbonyle, le reste acyle d'un acide carbonique mono-estérifié, ou représente un radical hydrocarboné-amino de formule $R^o$-N($R^1$)-, dans laquelle $R^1$ représente un atome d'hydrogène ou a la signification de $R^o$, ou qui forme, conjointement avec le groupe carbonyle, le reste acyle d'un acide carbamique mono- ou disubstitué, ou des sels de tels composés à propriétés salifiables, ou des complexes de tels composés, dans lesquels $A_1$, $A_2$ et $A_3$ représentent des atomes d'hydrogène, avec des métaux de transition paramagnétiques trivalents, y compris les lanthanides correspondants, des métaux du groupe IIIA du système périodique et des radionucléides.

9. Procédé selon l'une des revendications 1 et 3-7, caractérisé en ce que l'on prépare des composés de formule I selon la revendication 1, dans lesquels R, X et m ont les significations données dans la revendication 1, n a une valeur moyenne d'environ 10 à environ 17, et chacun des radicaux $A_1$, $A_2$ et $A_3$, indépendamment des autres, représente un atome d'hydrogène ou un groupe alkyle ou alcényle ayant jusqu'à et y compris 20 atomes de carbone, alkyloxycarbonyle ayant jusqu'à et y compris 20 atomes de carbone dans le fragment alkyle, dans lequel jusqu'à et y compris 5 groupes méthylène peuvent être remplacés par des atomes d'oxygène, dans chaque cas 2 atomes de carbone séparant l'un de l'autre les atomes d'oxygène, ou un groupe alkylaminocarbonyle ayant jusqu'à et y compris 20, de préférence jusqu'à et y compris 7 atomes de carbone dans le fragment alkyle, qui peut éventuellement être substitué par des substituants carboxy, (alkyl inférieur)-oxycarbonyle ayant jusqu'à et y compris 4 atomes de carbone dans le fragment alkyle inférieur, carbamoyle et/ou par des substituants amino, hydroxy, mercapto, (alkyl inférieur)-thio ayant jusqu'à et y compris 4 atomes de carbone, phényle ou hydroxyphényle, ou des sels de tels composés à propriétés salifiables, et des complexes de tels composés, dans lesquels $A_1$, $A_2$ et $A_3$ représentent des atomes d'hydrogène, avec des métaux de transition paramagnétiques trivalents, y compris les lanthanides correspondants, et des métaux appropriés du groupe IIIA du système périodique, ainsi qu'avec des radionucléides appropriés.

10. Procédé selon l'une des revendications 1 et 3-7, caractérisé en ce que l'on prépare des composés de formule I selon la revendication 1, dans lesquels R et X ont les significations données dans la revendication 1, m est 0, n a une valeur moyenne d'environ 10 à environ 17, et chacun des radicaux $A_1$, $A_2$ et $A_3$, indépendamment des autres, représente un atome d'hydrogène ou un groupe alcanoyle ayant jusqu'à et y compris 12 atomes de carbone, un radical alkyloxycarbonyle dans lequel le fragment alkyle contient jusqu'à et y compris 7 atomes de carbone, 1 ou 2 groupes méthylène pouvant être remplacés par un atome d'oxygène, et les atomes d'oxygène étant séparés l'un de l'autre dans chaque cas par 2 atomes de carbone, ou un radical alkylaminocarbonyle dans lequel le fragment alkyle contient jusqu'à et y compris 7 atomes de carbone, qui comporte éventuellement en tant que substituant en position 1 ou 2 un groupe alkyloxycarbonyle, dans lequel le fragment alkyle contient jusqu'à et y compris 4 atomes de carbone, et des complexes de tels composés dans lesquels $A_1$, $A_2$ et $A_3$ représentent des atomes d'hydrogène, avec des métaux de transition paramagnétiques trivalents, y compris des lanthanides appropriés, et avec des métaux du groupe IIIA du système périodique, ainsi qu'avec des radionucléides appropriés.

11. Procédé selon l'une des revendications 1, 3, 4, 6 et 7, caractérisé en ce que l'on prépare des composés de formule I selon la revendication 1, dans lesquels R représente le groupe méthyle, X a la signification donnée dans la revendication 1 et m est 0, n a une valeur moyenne d'environ 10 à environ 17, et chacun des radicaux $A_1$, $A_2$ et $A_3$ représente un atome d'hydrogène, et des complexes de tels composés avec des métaux de transition paramagnétiques trivalents, y compris des lanthanides, ou avec des métaux du groupe IIIA du système périodique, ainsi qu'avec des radionucléides appropriés.

**12.** Procédé selon l'une des revendications 1, 3 et 4, caractérisé en ce que l'on prépare la N-[ω-méthoxy-(dodécakis-éthylène-oxy)-carbonyl]-desferrioxamine B, dans laquelle le radical dodécakis-éthylène-oxy représente un radical de formule $-(CH_2-CH_2-O)_n-$, n ayant une valeur moyenne de 12.

**13.** Procédé selon l'une des revendications 1, 3 et 4, caractérisé en ce que l'on prépare la N-[ω-méthoxy-(heptadécakis-éthylène-oxy)-carbonyl]-desferrioxamine B, dans laquelle le radical heptadécakis-éthylène-oxy représente un radical de formule $-(CH_2-CH_2-O)_n-$, n ayant une valeur moyenne de 17.

**14.** Procédé selon l'une des revendications 1, 3 et 4, caractérisé en ce que l'on prépare la N-[ω-méthoxy-(undécakis-éthylène-oxy)-carbonyl]-desferrioxamine B, dans laquelle le radical undécakis-éthylène-oxy représente un radical de formule $-(CH_2-CH_2-O)_n-$, n ayant une valeur moyenne de 11.

**15.** Procédé selon l'une des revendications 1 et 3-5, caractérisé en ce que l'on prépare la N-[ω-méthoxy-(dodécakis-éthylène-oxy)-carbonyl]-O,O',O''-tri-(n-octanoyl)-desferrioxamine B, dans laquelle le radical dodécakis-éthylène-oxy représente un radical de formule $-(CH_2-CH_2-O)_n-$, n ayant une valeur moyenne de 12.

**16.** Procédé selon l'une des revendications 1 et 3-5, caractérisé en ce que l'on prépare la N-[ω-méthoxy-(dodécakis-éthylène-oxy)-carbonyl]-O,O',O''-tri-(éthoxycarbonyl)-desferrioxamine B, dans laquelle le radical dodécakis-éthylène-oxy représente un radical de formule $-(CH_2-CH_2-O)_n-$, n ayant une valeur moyenne de 12.

**17.** Procédé selon l'une des revendications 1 et 3-5, caractérisé en ce que l'on prépare la N-[ω-méthoxy-(dodécakis-éthylène-oxy)-carbonyl]-O,O',O''-tri-(éthoxy-carbonylméthylaminocarbonyl)-desferrioxamine B, dans laquelle le radical dodécakis-éthylène-oxy représente un radical de formule $-(CH_2-CH_2-O)_n-$, n ayant une valeur moyenne de 12.

**18.** Procédé selon l'une des revendications 1, 3, 4, 6 et 7, caractérisé en ce que l'on prépare le complexe ferrique de N-[ω-méthoxy-(undécakis-éthylène-oxy)-carbonyl]-desferrioxamine B, dans laquelle le radical undécakis-éthylène-oxy représente un radical de formule $-(CH_2-CH_2-O)_n-$, n ayant une valeur moyenne de 11.

**19.** Procédé selon l'une des revendications 1, 3, 4, 6 et 7, caractérisé en ce que l'on prépare le complexe manganique de N-[ω-méthoxy-(undécakis-éthylène-oxy)-carbonyl]-desferrioxamine B, dans laquelle le radical undécakis-éthylène-oxy représente un radical de formule $-(CH_2-CH_2-O)_n-$, n ayant une valeur moyenne de 11.

**20.** Composés préparables selon le procédé des revendications 1 et 3-19.

**21.** Composés préparables selon le procédé de la revendication 2.